# EUROPEAN PATENT APPLICATION

(11) **EP 3 587 595 A1**
(43) Date of publication of application: **01.01.2020**
(21) Application number: 18305788.4
(22) Date of filing: 21.06.2018
(51) Int. Cl.: C12Q 1/6886

(54) **METHODS AND KITS FOR DETECTING EGFR MUTATIONS**

(71) Applicant: Diadx, 34270 Les Matelles (FR); INSERM (Institut National de la Santé et de la Recherche Médicale), 75013 Paris (FR)
(72) Inventor: THIERRY, Alain, 34270 Saint Mathieu de Tréviers (FR); OTTOLINI, Barbara, 34080 Montpellier (FR)
(74) Representative: Cabinet Becker et Associés

(57) **Abstract**

The present invention relates to methods, materials and kits for detecting mutations in the EGFR gene. The invention also relates to methods for detecting or diagnosing cancer in a subject, as well as for determining the clinical stage, and clinical progression of a cancer.

## Description

The present invention relates to methods, materials and kits for detecting mutations in the EGFR gene. The invention also relates to methods for detecting or diagnosing cancer in a subject, as well as for determining the clinical stage, and clinical progression of a cancer.

### Background

The epidermal growth factor receptor (EGFR) is a trans-membrane tyrosine-kinase protein whose deregulated activation can lead to cellular proliferation, inhibition of apoptosis, angiogenesis and to phenotypic changes towards cell adhesion, migration and invasion. Activating EGFR mutations are clustered within exons 18-21 which encode for a portion of the EGFR kinase domain. EGFR mutations are very frequent in lung adenocarcinoma and are estimated to affect approximately 15% of US patients and more than 30% patients of Asian descent. In this respect, small-molecule EGFR tyrosine kinase inhibitors (TKIs) have been designed and developed, which launched the era of targeted, personalised and precise medicine for lung cancer. EGFR tyrosine kinase inhibitors are associated with high response rate and progression-free survival for patients with non-small cell lung cancer with EGFR-mutant genotype. Iressa® (gefitinib) and Tarceva® (erlotinib) constitute the first generation of EGFR tyrosine kinase inhibitors. However, most patients develop acquired drug resistance mostly driven by the T790M mutation occurring within the exon 20 of the EGFR gene. Table 1 below presents a list of EGFR mutations conferring either sensitivity or resistance to the first-generation TKIs.

**Table 1**

| | | **global frequency *EGFR* mutant: 29%** *(COSMIC 2015)* | | | |
|---|---|---|---|---|---|
| | | EXON | Mutation | frequency of each mutation in *EGFR* mutant tumors | R/S TKI EGFR |
| | | 18 | G719A | 0,60% | **S** |
| | | | G719C | 0,30% | **S** |
| | | | G719S | 0,50% | **S** |
| ***EGFR*** | ***NSCLC*** | 19 | exon 19 DEL | 48% | **S** |
| | | 20 | 5768I | <1% | **S** |
| | | 20 | exon 20 INS | 4-9,2% | **R** |
| | | 20 | T790M | < 5% untreated tumors >50% of acquired resistance to TKIs | **R** |
| | | 21 | L861Q | 2% | **S** |
| | | 21 | L858R | 43% | **S** |

Although the second-generation EGFR TKIs, such as Giotrif® (afatinib), dacomitinib and neratinib, demonstrated promising activity against T790M in preclinical models, they have failed to overcome resistance in patients due to dose-limiting toxicity.

Recently, the third-generation EGFR TKIs Tagrisso® (osimertinib) have shown to be effective against cell lines and murine models harbouring T790M mutations whilst sparing EGFR wild-type cells, which represented a promising breakthrough approach in overcoming T790M-mediated resistance in NSCLC patients.

On March 30, 2017, following the positive results obtained by the AURA study (ClinicalTrials.gov identifier: NCT01802632) the U.S. Food and Drug Administration granted regular approval to Tagrisso® for the treatment of patients with metastatic EGFR T790M mutation-positive NSCLC, whose disease has progressed on or after EGFR TKI therapy. Positive EGFR T790M mutation status needs to be determined by an FDA-approved test, either performed on tissue samples or from circulating tumour DNA obtained from plasma samples.

Accurate and sensitive detection systems for prompt genotyping of sensitising mutations to EGFR TKIs treatments and for the identification of pre-existing or acquired resistance to first-generation TKIs drugs are hence pivotal for providing lung cancer patients with the best and most effective personalised treatment, based on their genetic profiles.

### Summary of the invention

The invention provides novel methods, kits and materials allowing fast and reliable detection of mutations in the EGFR gene. The invention more specifically provides methods and kits for optimized detection of mutations in EGFR sequence from cell-free nucleic acids ("cfNA"). The invention is suitable to detect any type of mutation, such as nucleotide substitution(s), deletion(s) and/or insertion(s) in the EGFR gene sequence.

In a first aspect, the invention more particularly relates to a method for determining the presence of a mutation in the EGFR gene in a sample from a subject, the method comprising amplifying, in a sample of a biological fluid from the subject containing cell free nucleic acids, at least a first target sequence from the EGFR gene, the first target sequence containing a site of a mutation on said gene, wherein the first amplified target sequence is 50-95nt in length and amplification of the first target sequence is conducted with a first set of reagents comprising (i) a mutation-specific primer of 15-25nt in length, (ii) a paired primer of 15-25nt in length and (iii) an oligoblocker, wherein the oligoblocker hybridizes specifically to the first target sequence in non-mutated form and blocks polymerase elongation.

In a preferred embodiment, the method further comprises amplifying a second non-mutated target sequence on the EGFR gene, said second target sequence being located between 100 and 400bp from the first target sequence on said gene, the first and second amplified sequences each being 50-95 nt in length and of a similar length.

In a further aspect, the invention relates to a kit for determining the presence of a mutation in the EGFR gene in a sample of a biological fluid containing cell free nucleic acids from a subject, the kit comprising at least a first set of reagents, wherein the first set of reagents amplifies a first target sequence from the EGFR gene, said first target sequence containing a site of a mutation in the EGFR gene, said first set comprising (i) a mutation-specific primer of 15-25nt in length, (ii) a paired primer of 15-25nt in length positioned such that the amplified first target sequence is 50-95nt in length, and (iii) an oligoblocker, wherein the oligoblocker hybridizes specifically to the first target sequence in non-mutated form and blocks polymerase elongation.

Preferably, the kit comprises a second set of reagents (b) which amplifies a second non-mutated target sequence from the EGFR gene, said second target sequence being located between 100 and 400bp from the first target sequence on said gene, and wherein said amplified second target sequence is 50-95nt in length and of a length similar to that of the first amplified target sequence.

The invention may be used preferably to detect a mutation selected from G719A, G719C, G719S, S768I, T790M, L858R, L861Q, exon19DEL and exon20INS, or a combination thereof.

A further object of the invention relates to a nucleic acid molecule consisting of a nucleotide sequence selected from anyone of SEQ ID NOs: 1 to 55.

The invention also relates to the use of a nucleic acid molecule as defined above for in vitro amplification of a target sequence in the EGFR gene.

The invention also relates to a method for diagnosing a patient, comprising detecting a mutation in the EGFR gene in a sample from the patient using a method or kit or nucleic acid as defined above.

The invention also relates to a method for treating a patient, comprising detecting a mutation in the EGFR gene in a sample from the patient using a method or kit or nucleic acid as defined above, and treating subjects having cancer.

### Legend to the figures

**Figure 1****:** Scheme of the method of the invention in conventional (Panel A) and inverse (Panel B) configuration.
**Figure 2****:** Position of the proposed T790M wild-type primer set in relation with the T790M allele-specific primer set.
**Figure 3****:** DNA region to target in conventional sense to produce suitable T790M wild-type primer sets.
**Figure 4****:** Melting curves of commercial DNA mutant for *EGFR* T790M using the oligonucleotide combination T790M 16 + T790 P19 + T790 OB19.
**Figure 5****:** Melting curves of genomic DNA WT for *EGFR* T790M from the Difi cell line compared to melting curves of commercial DNA mutant for *EGFR* T790M using the oligonucleotide combination T790M 16 + T790 P19 + T790 OB19.
**Figure 6****:** Efficiency comparison between the proposed T790M system and a comparative detection system by Zhao et al. on lung cancer patient number 8. This sample was determined as mutant by our *EGFR* T790M assay with a mutant allele frequency of 11% and mutant cfDNA concentrations varying from 0.18 to 10 pg/µL in the extract. The compared system did not detect any mutation in this sample.
**Figure 7****:** Comparison of the ARMS-PCR based system by Zhao et al with out proposed system. Despite the low cfDNA concentration, this sample was determined as mutant by our *EGFR* T790M assay, with mutant allele frequency of 0.32% and mutant cfDNA concentration of 0.3pg/µL. The compared system did not detect any mutation in this same sample.
**Figure 8****:** DNA region to target in conventional sense to produce suitable L858R wild-type primer sets.
**Figure 9****:** Melting curves of commercial DNA mutant for *EGFR* L858R using the oligonucleotide combination L858R 19+ L858 P20 + L858 OB19/3.
**Figure 10****:** Melting curves of genomic DNA WT *for EGFR* L858R from the Difi cell line compared to melting curves of commercial DNA mutant for *EGFR* L858R using the oligonucleotide combination L858R 19+ L858 P20 + L858 OB19/3.
**Figure 11****:** DNA region to target in inverse sense to produce suitable L861Q wild-type primer sets.
**Figure 12****:** Melting curves of commercial DNA mutant for *EGFR* L861Q using the oligonucleotide combination L861Q i18 + L861 iP20 + L861 iOB20/2.
**Figure 13****:** Melting curves of genomic DNA WT *for EGFR* L861Q from the Difi cell line compared to melting curves of commercial DNA mutant for *EGFR* L861Q using the oligonucleotide combination L861Q i18 + L861 iP20 + L861 iOB20/2.
**Figure 14****:** DNA region to target in inverse sense to produce suitable S768 wild-type primer sets.
**Figure 15****:** Melting curves of commercial DNA mutant for *EGFR* S768I using the oligonucleotide combination S768I i16 + S768 iP18 +S768 iOB19.
**Figure 16****:** Melting curves of genomic DNA WT for *EGFR* S768I from the Difi cell line compared to melting curves of commercial DNA mutant for *EGFR* S768I using the oligonucleotide combination S768I i16 + S768 iP18 +S768 iOB19.
**Figure 17****:** Melting curves of commercial DNA mutant for *EGFR* S768I using the oligonucleotide combination S768I i17 + S768 iP18 +S768 iOB19.
**Figure 18****:** Melting curves of genomic DNA WT for *EGFR* S768I from the Difi cell line compared to melting curves of commercial DNA mutant for *EGFR* S768I using the oligonucleotide combination S768I i17 + S768 iP18 +S768 iOB19.
**Figure 19****:** DNA region to target in conventional sense to produce suitable G719wild-type primer sets. The potential WT primer candidates produce a PCR product of 64bp.
**Figure 20****:** Melting curves of commercial DNA mutant for *EGFR* G719S using the oligonucleotide combination G719S 20+G719 P19+ G719 OB19.
**Figure 21****:** Melting curves of genomic DNA WT *for EGFR* G719S from the Difi cell line compared to melting curves of commercial DNA mutant for *EGFR* G719S using the oligonucleotide combination G719S 20+G719 P19+ G719 OB19.
**Figure 22****:** Melting curves of commercial DNA mutant for *EGFR* G719A using the oligonucleotide combination G719A 18 + G719 P19 + G719 OB18/2.
**Figure 23****:** Melting curves of commercial DNA positive for *EGFR* G719S and WT for *EGFR* G719A compared to melting curves of commercial DNA mutant *for EGFR* G719A using the oligonucleotide combination G719A 18 + G719 P19 + G719 OB18/2.

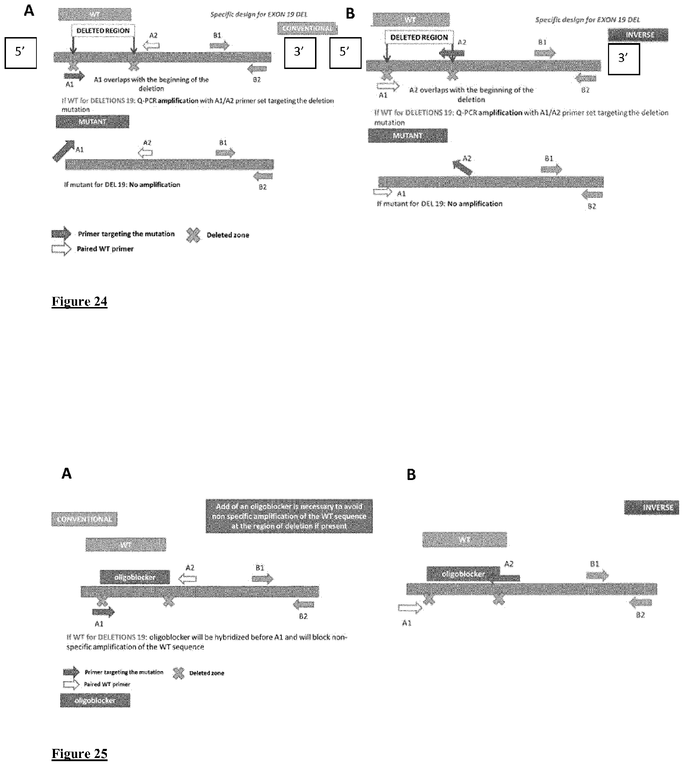

**Figure 24****:** Strategy for qualitative detection of the deletion. A. Strategy in conventional configuration: A1 is the primer targeting the beginning of the deletion. When DNA is WT for the deletion, the primer set A1-A2 amplifies the WT region. When DNA is mutant for the deletion, A1 is not hybridized with the DNA sequence and there is no amplification. **B.** Strategy in inverse configuration: A2 is the primer targeting the beginning of the deletion. When DNA is WT for the deletion, the primer set A1-A2 amplifies the WT region. When DNA is mutant for the deletion, A2 is not hybridized with the DNA sequence and there is no amplification.
**Figure 25****:** Strategy to improve specificity and sensitivity of deletions by IntPlex system in conventional configuration (A) and inverse configuration (B). The oligoblocker corresponds to the WT DNA sequence. As this oligoblocker is designed to have a higher affinity for the WT DNA sequence than the primer targeting the mutation, the oligoblocker will be hybridized first and will avoid non-specific WT DNA sequence amplification if present at the region of the mutation.
**Figure 26****.** Strategy to detect qualitatively and quantitatively deletion in *EGFR* exon 19 in conventional configuration (A) and inverse configuration (B).
**Figure 27****:** DNA region to target in conventional sense to produce suitable DEL19 wild-type primer sets.
**Figure 28****:** Melting curves of commercial DNA mutant for *EGFR* Δ(E746-A750) using the oligonucleotide combination DEL19 17N + DEL19 P19 + DEL19 OB21/3.
**Figure 29****:** DNA region to target in inverse sense to produce suitable Ins20 wild-type primer sets.

### Detailed description

The present invention relates to improved methods and kits for detecting mutations in EGFR gene sequence, as well as the uses thereof. EGFR mutations are correlated to cancers, particularly lung cancer, and the invention may be used e.g., in the diagnosis, drug development, or clinical monitoring of subjects with cancer, as well as to adjust/define optimized treatment.

The invention is suitable for detecting any mutation in EGFR. The term "mutation" designates any alteration in the nucleotide sequence encoding EGFR, such as nucleotide substitution(s), deletion(s), or insertion(s). In a particular embodiment, the mutation is a single nucleotide substitution in a coding (exon) region of the gene. In another particular embodiment, the mutation is a deletion or insertion of one or several (typically from 1 to 15) contiguous nucleotides, typically in an exon of the gene sequence.

The invention utilizes particular sets of allele-specific primers and oligoblockers allowing specific and sensitive detection of mutations in the gene sequence from circulating cell-free nucleic acids. The inventors have selected optimized target sequences within the EGFR gene and optimized primer design, which allow controlled and specific amplification from cell free nucleic acids. The invention can be implemented from any fluid sample containing cfNAs, e.g., cell free DNA (cfDNA), cell free RNA, cell free siRNA and/or cell free miRNA. Cell free nucleic acids are typically circulating nucleic acids contained in biological fluids, so that the method is advantageous as compared to an analysis from tissue biopsy. The invention may be implemented with samples from any subject, including any human patient having or suspected to have cancer.

The method of the invention typically comprises amplifying, in a sample of a biological fluid from the subject containing cell free nucleic acids, at least a first target sequence from the EGFR gene, the first target sequence containing a site of a mutation on said gene, wherein the first amplified target sequence is 50-95nt in length and amplification of the first target sequence is conducted with a first set of reagents comprising (i) a mutation-specific primer of 15-25nt in length, (ii) a paired primer of 15-25nt in length and (iii) an oligoblocker which hybridizes specifically to the first target sequence in non-mutated form resulting in a blockade of polymerase elongation.

The invention may be conducted with any fluid sample containing cfNA, such as blood, plasma, serum, pleural fluid, saliva, urine, or the like. The sample may be pretreated prior to being used in the invention (e.g., diluted, concentrated, separated, partially purified, frozen, etc.). The method is typically performed on a sample of, or derived from, blood, serum or plasma, such as a pretreated blood sample.

The invention uses a first set of primers which is designed to amplify a target sequence of 50-95nt in length within the EGFR gene, said sequence carrying a site of a targeted mutation in the gene.

The term "primer" designates, within the context of the present invention, nucleic acid molecules that can hybridize specifically to a target genetic sequence and serve to initiate amplification. Primers of the invention are typically single-stranded nucleic acids, with a length advantageously comprised between 10 and 30 bases, preferably between 15 and 30 bases, even more preferably between 15 and 25 bases. Primers should perfectly match with the targeted sequence in the EGFR gene, allowing specific hybridization thereto and essentially no hybridization to another region.

Preferred primers of the invention shall have a sequence with a GC content between 40 and 60%. The GC content is the number of G's and C's in the primer as a percentage of the total bases. Such a level of GC content provides the optimized combination of specific amplification and efficacy. Also, in a set of reagents of the invention, all of the primers shall have a similar Tm. More particularly, the Tm difference between the mutation-specific primer and its paired WT primer should preferably not be more than 5°C. Indeed, with such low difference, the difference of hybridization of each partner primer is low and does not affect specific mutant amplification. More preferably, the Tm of the primers should be between 40-60°C and should not differ between mutation-specific and paired by more than 5°C. Furthermore, in a preferred embodiment, the primers of the invention should not be self-annealing. Indeed, self-annealing may decrease the quantity of primer available for the amplification and reduce efficacy. Moreover, it contributes to non-specific amplification. The risk of self-annealing and self-hairpin loops of primers may be determined *in silico* using available software such as the software Oligoanalyzer®, which analyzes the change in Gibbs free energy required for the breaking of secondary structures (ΔG). Typically, if this energy is negative, it should not be below -4 kcal/mol, otherwise risk of self-annealing or self-hairpin loops could occur. In a particular embodiment, primers of the invention shall thus have a ΔG superior or equal to -4 kcal/mol as determined with Oligoanalyzer®. When using online tools as IDT OligoAnalyzer, which rely on different algorithms than Oligoanalyzer® for analyzing the change in Gibbs free energy required for the breaking of secondary structures, the ΔG should not be below -7kcal/mol otherwise risk of self-annealing or self-hairpin loops could occur. Accordingly, in another particular embodiment, primers of the invention shall have a ΔG superior or equal to -7 kcal/mol as determined with IDT OligoAnalyzer.

As indicated, the invention uses a first set of reagents comprising (i) a mutation-specific primer, (ii) a paired primer and (iii) an oligoblocker.

The mutation-specific primer is designed to hybridize to the mutated sequence, if and when present. In this regard, when the mutation is a nucleotide substitution, the mutation-specific primer preferably contains the mutated position at one terminal nucleotide thereof. Indeed, the invention shows such conformation allows increased specificity of the method. In a preferred embodiment, the mutated position is at the 3'-terminal nucleotide of the mutation-specific primer, or at the 5'-terminal nucleotide of the mutation-specific primer.

In another embodiment, when the mutation is a deletion, the mutation-specific primer overlaps with the junction region in the gene created by said deletion. The primer sequence is preferably centered on the targeted junction.

In still another embodiment, when the mutation is an insertion, the mutation-specific primer overlaps with at least one junction region in the gene created by said insertion. The primer sequence is preferably centered on the targeted junction.

The second primer, the "paired" primer, hybridizes to a sequence in the EGFR gene which is non-mutated and amplifies, with its paired mutation-specific primer, a target sequence of 50-95nt in length.

The first set of reagent contains an oligoblocker, which increases specificity of the method. The oligoblocker is an oligonucleotide which hybridizes specifically to the first target sequence in non-mutated form, such hybridization resulting in a blockade of amplification. When the targeted mutation is present, the oligoblocker does not hybridize and amplification of the mutated sequence occurs. In contrast, when the mutation is not present, the oligoblocker hybridizes to the target sequence and prevents non-specific amplification. The oligoblocker hybridizes "specifically" to the first target sequence in non-mutated form, which means that its sequence is exactly complementary to the non-mutated sequence. As a result, the oligoblocker essentially cannot hybridizes to the gene when the mutation is present. The oligoblocker preferably has a length similar to that of the two primers. It is thus preferably between 15-25 nucleotides in length. Furthermore, in order to increase specificity, in case of a single nucleotide mutation, the site of the non-mutated nucleotide targeted by the oligoblocker shall be preferably centered on the oligoblocker sequence, more preferably located within 80% central nucleotides, even more preferably within 60% central nucleotides, even further preferably within 40% central nucleotides.

Furthermore, in all embodiments, the oligoblocker is modified, preferably terminally, to prevent amplification upon hybridization, e.g. to prevent polymerase elongation. As a result, when the mutation is not present, the oligoblocker hybridizes to the target sequence and prevents any non-specific amplification. In a preferred embodiment, the oligoblocker is phosphorylated in 3', resulting in a blockade of polymerase elongation.

Amplification using the above set of reagents from a sample containing cfNA allows specific production of a 50-95nt long amplicon sequence containing a mutation when the targeted mutation is present, and no production of such an amplicon when the mutation is not present. The results contained in the present application show that such design provides high specificity and high sensitivity with any sample containing cell-free nucleic acids. Detection of an amplification product thus indicates the presence of the mutation in the sample.

Amplification may be performed using any technique known per se in the art such as with a polymerase, e.g., by PCR, Q-PCR, droplet-digital PCR and crystal-digital PCR.

In a preferred embodiment, the method uses two sets of reagents to amplify two distinct target sequences in the EGFR gene: The first set, as described above, targets a sequence containing a (site of a) mutation; and the second set targets a wild-type ("WT") sequence. By combining the two sets of reagents, the method of the invention allows measuring not only the presence or amount of a mutation, but also the level of total cfNA, thereby providing with high reliability a mutant allele frequency in circulating nucleic acids. The second targeted sequence should be located at a distance of about 100-400 bp of the mutant sequence. Furthermore, the second amplified sequence should have a length comprised between 50-95nt and similar to that of the first amplified sequence.

In a preferred embodiment, the method thus comprises a step of amplifying a second non-mutated target sequence on the EGFR gene, said second target sequence being located at a distance of 100 to 400bp from the first target sequence on said gene, the first and second amplified sequences each being 50-95 nt in length and of a similar length.

The term "similar length" indicates that the two amplified sequences should not differ in length by more than 15%, even more preferably by not more than 10%, further preferably by not more than 5%. In other words, if one set amplifies a sequence of 80nt, the other shall amplify a sequence of 68-92nt, preferably of 72-88nt, more preferably of 76-84nt.

The distance between the two targeted sequence shall be comprised between 100-400pb. Such a distance designates typically the number of nucleotides contained between the first amplified nucleotide of the first target sequence and the first amplified nucleotide of the second target sequence. Preferably, the distance is comprised between 200-400, more preferably between 250 and 350. Further preferably, at a distance of 300 nucleotides ± 10%.

The second set of reagents contains a pair of amplification primers, but no oligoblocker. Indeed, the second amplified sequence is non-mutated. The primers in the second set shall have a length between 15-25nt, and shall preferably have the characteristics as defined above for the first set in terms of Tm, GC content and self-annealing.

Both amplifications are preferably conducted in parallel, by separately incubating aliquots of a test sample with the two sets of reagents and suitable enzymes and nucleotides to perform amplification. The reactions thus produce:
- one mutation-specific amplicon of 50-95nt produced by the first set of reagents (if the mutation is present); and
- one WT amplicon of 50-95nt, produced by the second set of reagents.

Detection of the mutation-specific amplicon indicates the presence of the mutation in the sample. Detection of the other amplicon allows determination of a frequency of mutation in circulating NAs of the subject, e.g., by determining a ratio mt/WT. As illustrated in the experimental section, the method is highly selective and very sensitive.

Alternatively, the two amplifications may be conducted simultaneously, by incubating an aliquot of a test sample with the two sets of reagents and suitable enzymes and nucleotides to perform amplification. Such a reaction produces the following amplicons:
- one mutation-specific amplicon of 50-95nt produced by the first set of reagents (if the mutation is present);
- one WT amplicon of 50-95nt, produced by the second set of reagents; and
- one amplicon of 200-400nt, produced by the combination of primers of the two sets of reagents.

Furthermore, the method may be used to detect several different mutations in the EGFR gene from a subject sample, either sequentially, or in parallel. In such a case, the method uses one set of mutation-specific reagents as defined above for each targeted mutation. Typically, the method also uses at least one set of reagents that amplify a WT target sequence located 100-400 bp from one of the targeted mutated sequence.

Most preferred mutations that are detected with the present invention are described in the following table:

| **Gene** | **Disease** | **EXON** | **Mutation** | **Frequency** | **R/STKIEGFR** |
|---|---|---|---|---|---|
| ***EGFR*** | **NSCLC** | **global frequency** EGFR mutant: 29% (COSMIC 2015) | | | |
| | | frequency of each mutation in ECFR mutant tumors | | | |
| | | 18 | G719A | 0,60% | **S** |
| | | | G719C | 0,30% | S |
| | | | G7195 | 0,50% | **S** |
| | | 19 | exon 19 DEL | 48% | S |
| | | 20 | 5768I | <1% | S |
| | | 20 | exon 20 INS | 4-9,2% | **R** |
| | | 20 | T790M | < 5% untreated tumors | **R** |
| | | | | >50% of acquired resistance to TKIs | |
| | | 21 | L861Q | 2% | S |
| | | 21 | L858R | 43% | S |

These mutations are correlated to cancer, particularly lung cancer, such as NSCLC. The detection of these mutations, alone or in combinations, allows reliable diagnosis or prognosis of cancer in a subject, and also allows staging of the cancer.

In a particular embodiment, the invention is for detecting T790M mutation. *EGFR* T790M is a substitution mutation with a high frequency rate, especially in patients having acquired resistance to cancer treatment with tyrosine kinase inhibitors. Detecting the presence of this mutation allows an adaptation of the treatment. The T790M mutation results in an amino acid substitution at position 790, from a threonine (T) to a methionine (M). This mutation occurs within exon 20, which encodes part of the kinase domain. Nomenclature of EGFR T790M point mutation is c: 2369 C>T. It means that the 2369^{th} nucleotide on the cDNA reference sequence is a Cytosine that is substituted by a Thymine.

In a preferred embodiment, the mutation is EGFR T790M and the first set of reagents comprises a mutation-specific primer selected from SEQ ID NO: 1 or 2 (T790M 16; T790M 17), a paired primer selected from SEQ ID NO: 3 or 4 (T790 P19; T790 P20), and an oligoblocker selected from SEQ ID NO: 5 (T790 OB19). Most preferably, the mutation-specific primer is SEQ ID NO: 1 (T790M 16), the paired primer is SEQ ID NO: 3 (T790 P19) and the oligoblocker is SEQ ID NO: 5 (T790 OB19). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO: 6 (T790 WT L19) and a second primer SEQ ID NO: 7 (T790 WT R19).

In another embodiment, the mutation is *EGFR* L858R and the first set of reagents for detecting the *EGFR* L858R mutation comprises a mutation-specific primer selected from anyone of SEQ ID NOs: 8-11 (L858R 16, L858R 17, L858R 18, L858R 19), a paired primer selected from SEQ ID NO: 12 (L858 P20), and an oligoblocker selected from anyone of SEQ ID NOs: 13-14 (L858 OB19/3, L858 OB20/3). Most preferably, the mutation-specific primer is SEQ ID NO: 11 (L858R 19), the paired primer is SEQ ID NO: 12 (L858 P20) and the oligoblocker is SEQ ID NO: 13 (L858 OB19/3). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO: 15 (L858 WT L) and a second primer SEQ ID NO: 16 (L858 WT R).

In another embodiment, the mutation is *EGFR* L861Q and the first set of reagents for detecting the *EGFR* L861Q mutation comprises a mutation-specific primer selected from anyone of SEQ ID NOs: 17-18 (L861Q i17, L861Q i18), a paired primer selected from SEQ ID NO: 19 (L861 iP20), and an oligoblocker selected from anyone of SEQ ID NOs: 20-21 (L861 iOB19/2, L861 iOB20/2). Most preferably, the mutation-specific primer is SEQ ID NO: 18 (L861Q i18), the paired primer is SEQ ID NO: 19 (L861 iP20), and the oligoblocker is SEQ ID NO: 21 (L861 iOB20/2). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO: 22 (L861 WT L) and a second primer SEQ ID NO: 23 (L861 WT R).

In another embodiment, the mutation is *EGFR* G719S and the first set of reagents for detecting *EGFR* G719S mutation comprises a mutation-specific primer selected from anyone of SEQ ID NOs: 24-25 (G719S 19, G719S 20), a paired primer selected from SEQ ID NO: 26 (G719 P19), and an oligoblocker selected from anyone of SEQ ID NOs: 27-28 (G719 OB18/2, G719 OB19/2). Most preferably, the mutation-specific primer is SEQ ID NO: 25 (G719S 20), the paired primer is SEQ ID NO:26 (G719 P19), and the oligoblocker is SEQ ID NO: 28 (G719 OB19/2). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO:29 (G719 WT L) and a second primer SEQ ID NO: 30 (G719 WT R).

In another embodiment, the mutation is *EGFR* G719A and the first set of reagents for detecting the *EGFR* G719A mutation comprises a mutation-specific primer selected from anyone of SEQ ID NOs: 31-33 (G719A 18, G719A 19, G719A 20), a paired primer selected from SEQ ID NO: 26 (G719 P19), and an oligoblocker selected from anyone of SEQ ID NOs: 27-28 (G719 OB18/2, G719 OB19/2). Most preferably, the mutation-specific primer is SEQ ID NO: 31 (G719A 18), the paired primer is SEQ ID NO: 26 (G719 P19) and the oligoblocker is SEQ ID NO: 27 (G719 OB18/2). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO: 29 (G719 WT L) and a second primer SEQ ID NO: 30 (G719 WT R).

In another embodiment, the mutation is *EGFR* S768I and the first set of reagents for detecting the *EGFR* S768I mutation comprises a mutation-specific primer selected from anyone of SEQ ID NOs: 34-35 (S768I i16, S768I il7), a paired primer selected from SEQ ID NO: 36 (S768 iP18), and an oligoblocker selected from SEQ ID NO: 37 (S768 iOB19). Most preferably, the mutation-specific primer is SEQ ID NO: 35 (S768I il7), the paired primer is SEQ ID NO: 36 (S768 iP18) and the oligoblocker is SEQ ID NO: 37 (S768 iOB19). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO:38 (S768 WT L) and a second primer SEQ ID NO: 39 (S768 WT R).

In another embodiment, the mutation is EGFR exon19 Δ(E746-A750) and wherein the mutation-specific primer is SEQ ID NO: 40 (DEL19 17N), the paired primer is SEQ ID NO: 41 (DEL19 P19), and the oligoblocker is SEQ ID NO: 42 (DEL19 OB21/3). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO: 43 (DEL19 WT L) and a second primer SEQ ID NO: 44 (DEL19 WT R).

In another embodiment, the mutation is *EGFR* V769-D770 insASV (Ins20) and the first set of reagents for detecting the *EGFR* Ins20 mutation comprises a mutation-specific primer selected from anyone of SEQ ID NOs: 45-49 (INS20 AVS i16, INS20 AVS i16/2, INS20 AVS i16/3, INS20 AVS i16/4, INS20 AVS i16/5), a paired primer selected from anyone of SEQ ID NOs: 50-52 (Ins20 iP18, Ins20 iP17, Ins20 iP17/2), and an oligoblocker selected from SEQ ID NO: 53 (Ins20AVS iOB18). Most preferably, the mutation-specific primer is SEQ ID NO: 45 (INS20 AVS i16), the paired primer is SEQ ID NO: 51 (Ins20 iP17) and the oligoblocker is SEQ ID NO: 53 (Ins20AVS iOB18). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO:54 (Ins20 WT L) and a second primer SEQ ID NO: 55 (Ins20 WT R).

In another particular embodiment, the invention is for detecting a combination of two or more mutations in the EGFR gene.

In this regard, in a particular embodiment, the method is for detecting at least two nucleotide substitutions, preferably at least T790M and L858R.

In another embodiment, the method is for detecting at least one nucleotide substitution and one deletion.

In a particular embodiment, the method is for detecting T790M, L858R and DelExon19 (such as exon19 Δ(E746-A750)).

Such combination of mutations is particularly indicative of a subject condition. In particular, the detection of L858R and of deletions of Exon 19 covers approximately 91% of EGFR positive cases where targeted therapy with first-generation TKIs is predicted to work effectively. Moreover, the addition of T790M enables to identify the 5% of treatment-naive patients T790M-positive that would be resistant to first-generation TKIs, for whom third-generation TKIs should be considered as favored therapeutic option.

### Kits

In a further aspect, the invention relates to kits suitable for detecting EGFR mutations in a sample of a biological fluid containing cell free nucleic acids from a subject. Kits generally comprise reagents, containers and, optionally, instructions to perform the method. The reagents may be in a same container, in discrete parts thereof, of in separate containers. The user may then use any portion of the reagents to perform the reaction, typically following the instructions.

In a more particular embodiment, the kits of the invention comprise at least a first set of reagents (a), wherein the first set of reagents amplifies a first target sequence from the EGFR gene, said first target sequence containing a site of a mutation in the EGFR gene, said first set of reagents comprising (i) a mutation-specific primer of 15-25nt in length, (ii) a paired primer of 15-25nt in length positioned such that the amplified first target sequence is 50-95nt in length, and (iii) an oligoblocker, wherein the oligoblocker hybridizes specifically with the first target sequence in non-mutated form and blocks amplification.

In a further particular embodiment, the kits of the invention further comprise a second set of reagents (b) which amplifies a second non-mutated target sequence from the EGFR gene, said second target sequence being located between 100 and 400bp from the first target sequence on said gene, and wherein said amplified second target sequence is 50-95nt in length and of a length similar to that of the first amplified target sequence.

In a further particular embodiment, the kits of the invention comprise at least a third set of reagents (c), wherein the third set of reagents amplifies a third target sequence from the EGFR gene, said third target sequence containing a site of a mutation in the EGFR gene distinct from the first mutation targeted by set of reagent (a), said third set of reagents comprising (i) a mutation-specific primer of 15-25nt in length, (ii) a paired primer of 15-25nt in length positioned such that the amplified third target sequence is 50-95nt in length, and (iii) an oligoblocker, wherein the oligoblocker hybridizes specifically with the third target sequence in non-mutated form and blocks amplification.

Sets of reagents (a), (b) and (c) are preferably in separate containers of the kit, allowing amplification in parallel on different aliquots of a test sample.

In a particular embodiment, the kit further comprises other reagents to perform an amplification reaction, such as an enzyme (e.g., a polymerase such as Taq), nucleotides, and/or a buffer. Such reagents may be in separate containers, to be mixed by the user.

The kit is preferably suitable to detect a mutation in EGFR gene selected from G719A, G719C, G719S, S768I, T790M, L858R, L861Q, exon19DEL and exon20INS.

In a preferred embodiment, the kit of the invention is suitable for detecting the mutation EGFR T790M and the first set of reagents comprises a mutation-specific primer selected from anyone of SEQ ID NOs: 1-2 (T790M 16; T790M 17), a paired primer selected from SEQ ID NO: 3 or 4 (T790 P19; T790 P20), and an oligoblocker selected from SEQ ID NO:5 (T790 OB19). Most preferably, the mutation-specific primer is SEQ ID NO: 1 (T790M 16), the paired primer is SEQ ID NO: 3 (T790 P19) and the oligoblocker is SEQ ID NO: 5 (T790 OB19). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO: 6 (T790 WT L19) and a second primer SEQ ID NO: 7 (T790 WT R19).

In another embodiment, the kit of the invention is suitable for detecting the mutation *EGFR* L858R and the first set of reagents for detecting the *EGFR* L858R mutation comprises a mutation-specific primer selected from anyone of SEQ ID NOs: 8-11 (L858R 16, L858R 17, L858R 18, L858R 19), a paired primer selected from SEQ ID NO: 12 (L858 P20), and an oligoblocker selected from anyone of SEQ ID NOs: 13-14 (L858 OB19/3, L858 OB20/3). Most preferably, the mutation-specific primer is SEQ ID NO: 11 (L858R 19), the paired primer is SEQ ID NO: 12 (L858 P20) and the oligoblocker is SEQ ID NO: 13 (L858 OB19/3). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO: 15 (L858 WT L) and a second primer SEQ ID NO: 16 (L858 WT R).

In another embodiment, the kit of the invention is suitable for detecting the mutation *EGFR* L861Q and the first set of reagents for detecting the *EGFR* L861Q mutation comprises a mutation-specific primer selected from anyone of SEQ ID NOs: 17-18 (L861Q i17, L861Q i18), a paired primer selected from SEQ ID NO: 19 (L861 iP20), and an oligoblocker selected from anyone of SEQ ID NOs: 20-21 (L861 iOB19/2, L861 iOB20/2). Most preferably, the mutation-specific primer is SEQ ID NO: 18 (L861Q i18), the paired primer is SEQ ID NO: 19 (L861 iP20), and the oligoblocker is SEQ ID NO: 21 (L861 iOB20/2). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO: 22 (L861 WT L) and a second primer SEQ ID NO: 23 (L861 WT R).

In another embodiment, the kit of the invention is suitable for detecting the mutation *EGFR* G719S and the first set of reagents for detecting *EGFR* G719S mutation comprises a mutation-specific primer selected from anyone of SEQ ID NOs: 24-25 (G719S 19, G719S 20), a paired primer from SEQ ID NO: 26 (G719 P19), and an oligoblocker selected from anyone of SEQ ID NOs: 27-28 (G719 OB18/2, G719 OB19/2). Most preferably, the mutation-specific primer is SEQ ID NO: 25 (G719S 20), the paired primer is SEQ ID NO: 26 (G719 P19), and the oligoblocker is SEQ ID NO: 28 (G719 OB19/2). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO: 29 (G719 WT L) and a second primer SEQ ID NO: 30 (G719 WT R).

In another embodiment, the kit of the invention is suitable for detecting the mutation *EGFR* G719A and the first set of reagents for detecting the *EGFR* G719A mutation comprises a mutation-specific primer selected from anyone of SEQ ID NOs: 31-33 (G719A 18, G719A 19, G719A 20), a paired primer from SEQ ID NO: 26 (G719 P19), and an oligoblocker selected from anyone of SEQ ID NOs: 27-28 (G719 OB18/2, G719 OB19/2). Most preferably, the mutation-specific primer is SEQ ID NO: 31 (G719A 18), the paired primer is SEQ ID NO: 26 (G719 P19) and the oligoblocker is SEQ ID NO: 27 (G719 OB18/2). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO: 29 (G719 WT L) and a second primer SEQ ID NO: 30 (G719 WT R).

In another embodiment, the kit of the invention is suitable for detecting the mutation *EGFR* S768I and the first set of reagents for detecting the *EGFR* S768I mutation comprises a mutation-specific primer selected from anyone of SEQ ID NOs: 34-35 (S768I i16, S768I il7), a paired primer from SEQ ID NO: 36 (S768 iP18), and an oligoblocker from SEQ ID NO: 37 (S768 iOB19). Most preferably, the mutation-specific primer is SEQ ID NO: 35 (S768I il7), the paired primer is SEQ ID NO: 36 (S768 iP18) and the oligoblocker is SEQ ID NO: 37 (S768 iOB19). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO: 38 (S768 WT L) and a second primer SEQ ID NO: 39 (S768 WT R).

In another embodiment, the kit of the invention is suitable for detecting the mutation EGFR exon19 Δ(E746-A750) and wherein the mutation-specific primer is SEQ ID NO: 40 (DEL19 17N), the paired primer is SEQ ID NO: 41 (DEL19 P19), and the oligoblocker is SEQ ID NO: 42 (DEL19 OB21/3). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO: 43 (DEL19 WT L) and a second primer SEQ ID NO: 44 (DEL19 WT R).

In another embodiment, the kit of the invention is suitable for detecting the mutation *EGFR* V769-D770 insASV (Ins20) and the first set of reagents comprises a mutation-specific primer selected from anyone of SEQ ID NOs: 45-49 (INS20 AVS i16, INS20 AVS i16/2, INS20 AVS i16/3, INS20 AVS i16/4, INS20 AVS i16/5), a paired primer selected from anyone of SEQ ID NO:50-52 (Ins20 iP18, Ins20 iP17, Ins20 iP17/2), and an oligoblocker selected from SEQ ID NO: 53 (Ins20AVS iOB18). Most preferably, the mutation-specific primer is SEQ ID NO: 45 (INS20 AVS i16), the paired primer is SEQ ID NO: 51 (Ins20 iP17) and the oligoblocker is SEQ ID NO: 53 (Ins20AVS iOB18). In a preferred embodiment, the method amplifies a second WT target sequence, located preferably 200-400 bp from the first mutated target sequence, using a second set of reagents. In a more preferred embodiment, the second set of reagents comprises a first primer SEQ ID NO: 54 (Ins20 WT L) and a second primer SEQ ID NO: 55 (Ins20 WT R).

The kits of the invention may comprise reagents for detecting one mutation in the EGFR gene.

Alternatively, kits of the invention may comprise reagents suitable for detecting several distinct mutations in the EGFR gene. In this regard, particular preferred kits of the invention comprise reagents as described above for detecting at least T790M and L858R. A particular kit of the invention comprises reagents as described above for detecting T790M and L858R and Exon19del (in particular exon19 Δ(E746-A750)). Such reagents are preferably in separate containers of the kit, allowing amplification in parallel on different aliquots of a test sample.

In this regard, a particular kit of the invention comprises:
- a first set of reagents (a) comprising a mutation-specific primer selected from anyone of SEQ ID NOs: 1-2 (T790M 16; T790M 17), a paired primer selected from anyone of SEQ ID NOs: 3 or 4 (T790 P19; T790 P20), and an oligoblocker selected from SEQ ID NO:5 (T790 OB19). Most preferably, the mutation-specific primer is SEQ ID NO: 1 (T790M 16), the paired primer is SEQ ID NO: 3 (T790 P19) and the oligoblocker is SEQ ID NO: 5 (T790 OB19);
- a second set of reagents (b) for detecting a WT target sequence, located preferably 200-400 bp from the mutated sequence detected by the first set of reagents. In a more preferred embodiment, the second set of reagents comprises a first primer SEQ ID NO: 6 (T790 WT L19) and a second primer SEQ ID NO: 7(T790 WT R19);
- a third set of reagents (c) for detecting the *EGFR* L858R mutation comprising a mutation-specific primer selected from anyone of SEQ ID NOs: 8-11 (L858R 16, L858R 17, L858R 18, L858R 19), a paired primer from SEQ ID NO: 12 (L858 P20), and an oligoblocker selected from anyone of SEQ ID NOs: 13-14 (L858 OB19/3, L858 OB20/3). Most preferably, the mutation-specific primer is SEQ ID NO: 11 (L858R 19), the paired primer is SEQ ID NO: 12 (L858 P20) and the oligoblocker is SEQ ID NO: 13 (L858 OB19/3).

In a particular embodiment, the kit further comprises a fourth set of reagents (d) for detecting the mutation EGFR exon19 Δ(E746-A750) and wherein the mutation-specific primer is SEQ ID NO: 40 (DEL19 17N), the paired primer is SEQ ID NO: 41 (DEL19 P19), and the oligoblocker is SEQ ID NO: 42 (DEL19 OB21/3).

The invention also relates to the use of kits as described above for detecting EGFR mutations in a sample from a subject, preferably a human subject

The invention also relates to a nucleic acid molecule consisting of a nucleotide sequence selected from anyone one SEQ ID NOs: 1 to 55. Preferred nucleic acid molecules are selected from 1-5, 8-14, 17-21, 24-28, 31-33, 34-37, 40-42 and 45-53. Particular nucleic acid molecules of the invention are mutation-specific primers consisting of a nucleotide sequence selected from anyone of SEQ ID Nos: 1,2,8,11,17,18,24,25,31-33,34,35,40 and 45-49.

The invention also relates to the use of a nucleic acid molecule as defined above for in vitro amplification of a target sequence in an EGFR gene.

The invention also relates to a method for diagnosing a patient, comprising detecting a mutation in the EGFR gene in a sample from the patient using a method or kit or nucleic acid as defined above.

The invention also relates to a method for treating a patient, comprising detecting a mutation in the EGFR gene in a sample from the patient using a method or kit or nucleic acid as defined above, and treated subjects having cancer.

Further aspects and advantages of the invention will be disclosed in the following experimental section.

### Examples

### I. Methods

This section presents a summary of optimal criteria followed to ensure optimal efficiency and sensitivity for all mutation detection systems herein described, in combination with absence of any non-specificity.

### I1) Design of the allele-specific primer targeting the mutation of interest

To optimise specificity and sensitivity of the allele-specific primer targeting the single nucleotide mutation of interest, the mutant base was generally positioned as 3' end last nucleotide. The 3' end of an allele-specific primer is mismatched for one allele (the WT allele) and is a perfect match for the other allele (the mutated allele). The 3' positioning of the mismatch has the effect of partially blocking the amplification of the WT allele. Indeed, the *Taq* polymerase enzyme typically used for DNA amplification preferably extends DNA in presence of a perfect match at the 3' primer end with a free hydroxyl group present. Furthermore, any residual non-specific amplification from the WT allele is further avoided using an oligoblocker, as discussed in section I3.

The positioning of the targeted mutation as the 3' end of the allele-specific primers generates two possible options for assay design: one in "conventional configuration", with the primer sequence matching the reference sequence on the positive strand (hence with the primer annealing to the negative strand) with the mutation in 3'; one in "inverse configuration", with the primer sequence matching the negative strand (hence with the primer annealing to the positive strand) with the mutation in 3'.
Both options were considered in the experimental section, and the thermodynamic characteristics of both options were generally compared, to ensure optimal Tm and GC% with absence of primer-dimers, blocker-primer dimers, self-dimerisation, and hairpins. In addition, different combinations of primers at different lengths were tested, to provide optimal results.

In the selection of the primers targeting the mutation of interest the following recommendations were followed to prevent the risk of non-specific amplification and to improve the method's sensitivity:
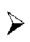 Low melting temperature (Low Tm): Ideally, the Tm of the primer should be 10°C below the annealing temperature of the PCR system (60°C), with accepted range between 45-55°C. As the variant is targeted at the 3'end last base of the primer, Tm changes towards the optimal temperature are obtained by varying the primer length at the 5' end.
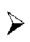 Length of the allele-specific primer: The primer targeting the mutation should have low Tm (45-55°C), suggesting a short length but it should be long enough to be highly specific and provide efficient amplification. Length was found optimal between 16 and 22 bp.
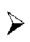 GC%: The GC content (percentage of guanines and cytosines out of the total number of bases) of the primer should ideally fall between 40 and 60%. Lower the GC%, lower the risk of non-specific amplification but it is associated with lower amplification efficiency. Higher the GC%, higher the risk of non-specific amplification but the amplification efficiency is increased.
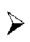 3'-tail GC%: Higher values (>25%) allow more specific annealing of the primer to its target sequence.
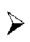 Secondary structures: the formation of primer self-dimers or hairpin loops is detrimental to the PCR efficiency as it considerably decreases the concentration of primer molecules available for the PCR reaction. Also, secondary structures can generate strong background signals that can negatively impact on the quantification accuracy of the desired PCR product. The software Oligoanalyzer® was used to analyse the change in Gibbs free energy required for breaking down the predicted secondary structures (ΔG). ΔG below -4 kcal/mol are indicative of primers at a very strong risk of self-annealing that should hence be modified to reach ΔG values closer to zero or discarded. Absence of any hairpin is ideal. In presence of hairpins with ΔG below -3 kcal/mol the primer should be modified to reach ΔG values closer to zero or discarded.

### 12) Paired Wild-Type primer to the allele-specific primer

In the selection of the paired wild-type primer the following criteria were followed to prevent the risk of non-specific amplification and to improve the method's sensitivity:
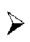 Amplicon size: the paired WT primer was positioned at 60-100 bp from the primer targeting the mutation in order to produce an amplicon of 60-100 bp. Such size was indeed found optimal by the inventors for detecting tumour-derived circulating DNA.
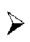 DNA strand to target:
   ∘ In the conventional configuration (allele-specific primer designed upon the reference sequence, annealing to the negative strand), the paired wild-type primer sequence is the complement-reverse of the reference sequence, in order to anneal to the positive strand.
   ∘ In the inverse configuration (allele-specific primer designed as complement-reverse of the reference sequence to maintain the mutation in 3', annealing to the positive strand), the paired wild type sequence is designed upon the reference sequence, in order to anneal to the negative strand.
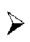 Melting temperature: Ideally, the Tm difference between the Tm of the primer targeting the mutation and its paired WT primer should not exceed 5°C. As a consequence, the Tm of the paired WT primer should preferably be 50-60°C.
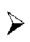 GC%, 3' tail GC% and secondary structures: the same parameters described in section I1. were followed.
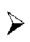 Off-target amplification: Co-amplification of any secondary product other than the target sequence should be avoided to ensure specificity. Presence of off-target amplification would generate false negative results and compromise the validity of the proposed method. To prevent this eventuality, the selected WT paired primers, together with their matched allele-specific primers, were analysed using several methods to confirm specificity for optimal primer combinations.
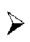 Unwanted homology regions: The amplicons are typically analysed using the "Blat" online tool on the UCSC Genome Browser website to determine the presence of potential secondary regions of homology that might generate non-specific products or alter the PCR efficiency.

### 13) Oligoblocker design

The oligoblocker is an oligonucleotide complementary to the WT allele with respect to the mutation of interest. It is modified (e.g., phosphorylated in 3') to block the polymerase elongation. The role of the oligoblocker is to avoid non-specific amplification of the WT sequence at the mutation locus. When the oligoblocker is hybridised to the WT sequence it avoids non-specific hybridisation of the primer targeting the mutation to the WT sequence, as it partially overlaps the primer target sequence.

The criteria for an optimal oligoblocker design are herein described, to prevent the risk of non-specific amplification and to improve the detection of low-frequency mutations.
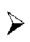 Melting temperature: The oligoblocker Tm should preferably be close to 60°C (the hybridisation/extension temperature of the polymerase enzyme required for the PCR amplification reaction) and preferably at least 4°C above the Tm of the primer targeting the mutation. This preferred strategy ensures that the oligoblocker hybridises to its target sequence before any potential non-specific hybridisation to the WT locus by the allele-specific primer. Ideally, the oligoblocker Tm should hence be 55-60°C. 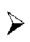 Position: The oligoblocker should be designed preferably so as to have the variant nt position towards the middle of its sequence. With this strategy, the oligoblocker occupies a larger portion of the allele-specific primer target region, aiding to avoid non-specific hybridisation.
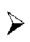 GC% and 3'GC%: the same parameters described in section I.1. should prefrerably be followed.
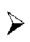 Secondary structures: The same parameters described in section I.1 should preferably be followed.
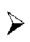 Length: The ideal length of an oligoblocker should be comprised between 19-23 bp.

### 14) Combinatorial hetero-dimer analysis

The absence of hetero-annealing among the oligonucleotides (allele-specific primer, paired WT primer and oligoblocker) should preferably be verified. This strategy prevents the risk of forming primer-dimers and non-specific amplification, improving the efficiency of the method. The software Oligoanalyzer® can be used for this purpose and, based on its algorithms for the calculations of Gibbs free energy, just values above -4 Kcal/mol can be accepted. Oligonucleotide combinations with ΔG <-4 kcal/mol need to be discarded and re-designed.

### 15) Design of the wild-type primer pair

This primer set should target a nucleic acid sequence located 100-350 bp, typically 300±10 bases pair, from the nucleic acid sequence targeted by the allele-specific primer set. Moreover, it should preferably target a nucleic acid sequence of the same size than the one targeted by the allele-specific primer set ± 10%. Preferred characteristics of the primers that ensure optimal performance are listed below:
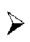 Melting temperature: The melting temperature should be comprised between 55 and 60°C, with less than 5°C difference between the Tm of the two primers.
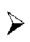 All other thermodynamic parameters to consider in the design of the wild-type primer pair (GC%, 3'GC%, amplicon size, secondary structures, off-target amplification and unwanted homology regions) are the same as described in section 1.2.

### 16) In Vitro validation for mutated region

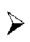 Specificity test: This phase is generally performed for verifying that the mutation-specific primer set under evaluation amplifies only its target sequence. It is typically realised on genomic DNA from cell lines carrying the mutation of interest, from commercial DNA carrying the mutation of interest or from synthetic double-strand DNA fragments carrying the mutation of interest diluted in wild-type genomic DNA background.
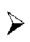 Non-specificity test: The non-specificity test can be performed to ensure that the primer set targeting the mutation of interest does not amplify any other region and that no primers-dimers or self-dimers are formed during the PCR reaction. Criteria to validate the non-specificity are typically: concentration values below 0.5 pg/µl and a different Tm from the expected Tm of the mutation, as determined by the specificity test.
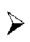 Efficiency test: The efficiency of mutation-specific primer set is generally evaluated by running the assay on positive controls of known mutational load.
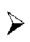 Sensitivity: To assess the sensitivity of the system in analysis, an assay is generally run on serial dilutions of DNA mutant for the variant in analysis.

### II) T790M mutation

*EGFR* T790M is a substitution mutation. The T790M mutation results in an amino acid substitution at position 790, from a threonine (T) to a methionine (M). This mutation occurs within *EGFR* exon 20, which encodes part of the kinase domain. Nomenclature of *EGFR* T790M point mutation is c: 2369 C>T. It means that the 2369^{th} nucleotide on the cDNA reference sequence is a Cytosine that is substituted by a Thymine.
The inventors have designed specific sets of primers allowing improved detection of the presence (or absence, or frequency) of such a mutation from a sample containing cfNA.

### II1) Design of the allele-specific T790M primer

Various allele-specific primers were considered in the design of this invention and their thermodynamic characteristics were analysed, as shown in Table 2:

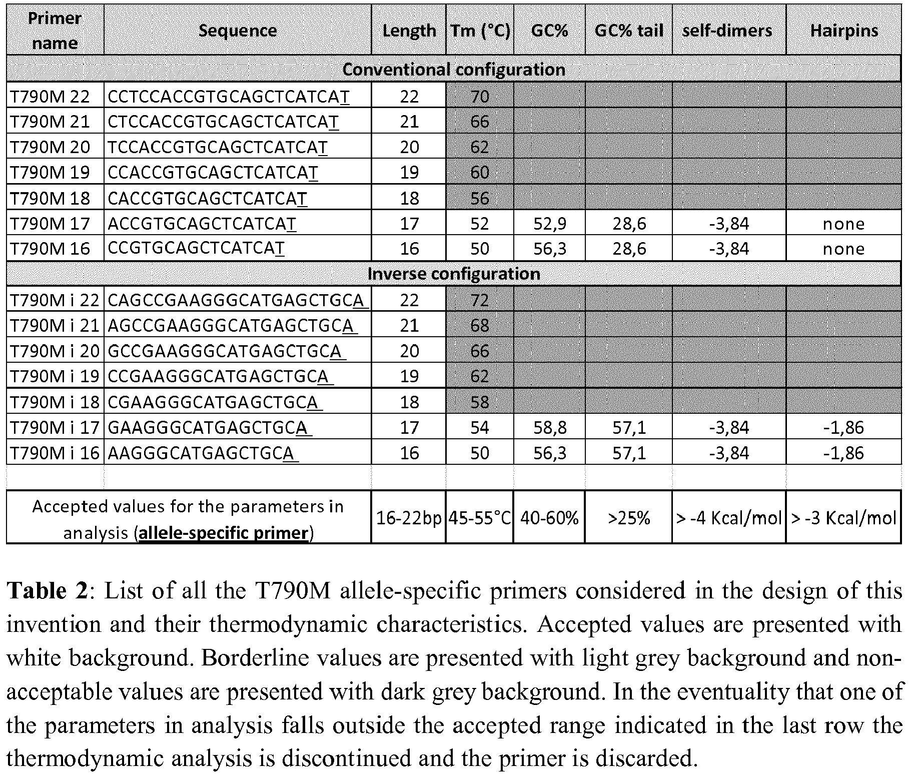

### II2) Paired Wild-Type primer

### Results obtained with Primer 3

All allele-specific primers that met our criteria (see Table 2) were used on Primer3 to find a compatible paired primer. Results are summarised in Table 3.

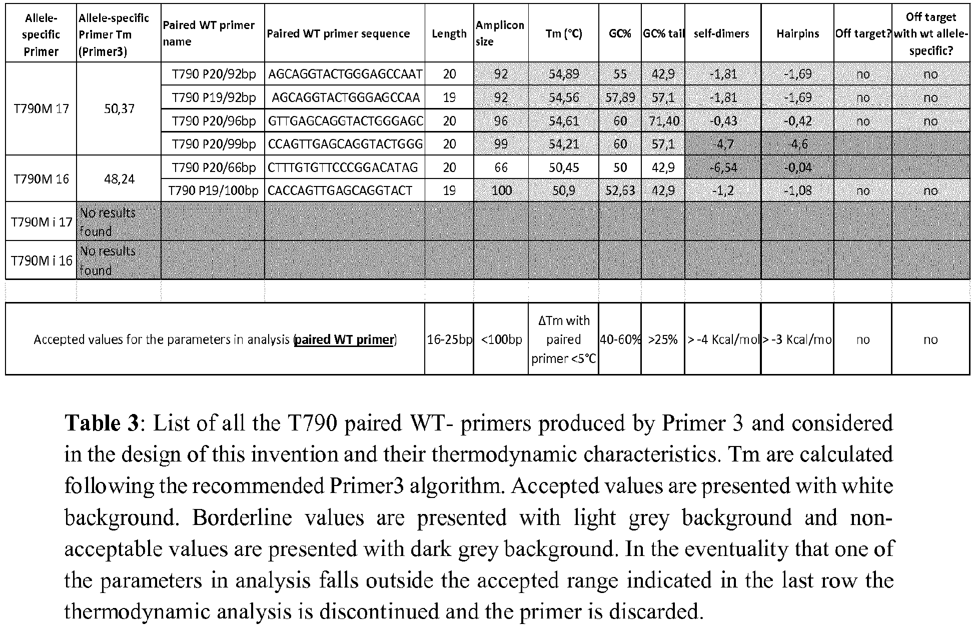

From the results presented in Table 2 it was not possible to select a definite candidate as paired WT primer for the T790M allele-specific primer. The candidates with the best thermodynamic characteristics produced amplicons considered borderline for low frequency detection of mutations from tumour-derived cfDNA fragments. On the other hand, the primer T790 P20/66bp in combination with the allele-specific primer T790M 16 produced an amplicon of suitable size but contains a region causing high self-dymerisation.

### Manually generated primer pair candidates in conventional configuration

In view of the unsuccessful generation of a paired primer using conventional tools, we designed such primers manually. All primers are listed in the following table. Their characteristics were analyzed.

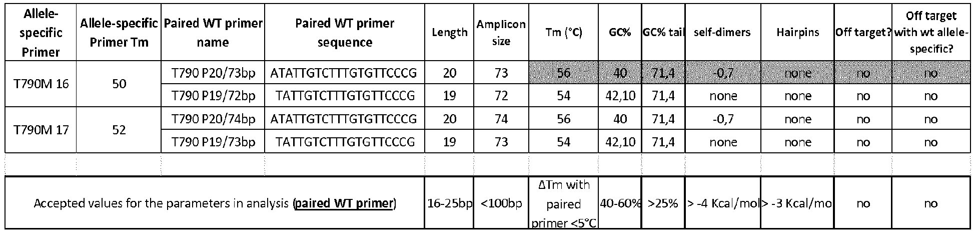

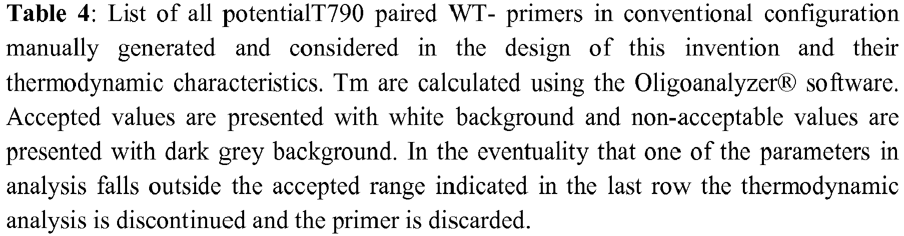

The following combinations were thus found, that meet the thermodynamic criteria required:
- T790M 16 + T790 P19/72bp
- T790M 17 + T790 P19/73bp
- T790M 17 + T790 P20/74bp

### Blat alignment of the validated primer pairs in conventional configuration

Blat alignments (Human GRCh38/hg38) for all the PCR amplicons generated by the combinations of primers meeting the thermodynamic criteria were performed. All primer pairs produced just one single amplicon at the expected chromosomal position. No other homologies were reported.

### Manually generated primer pair candidates in inverse configuration

We further designed paired primers in inverse configuration, as listed in the following table.

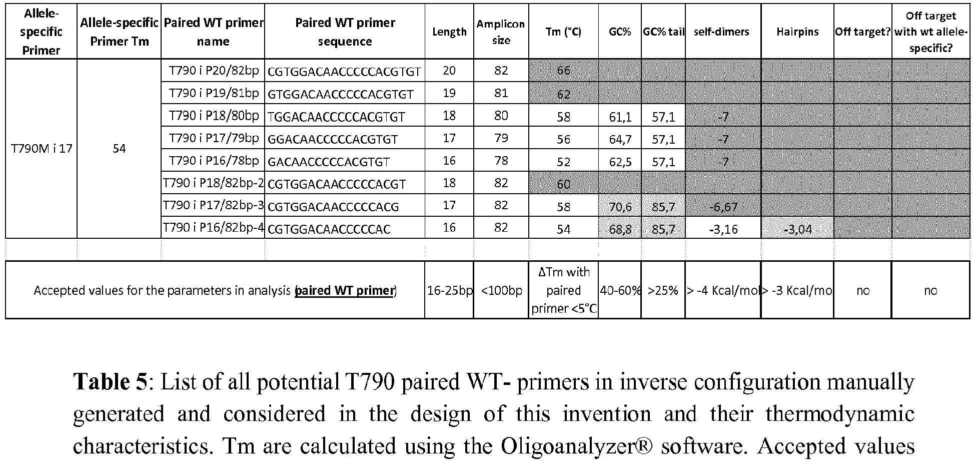

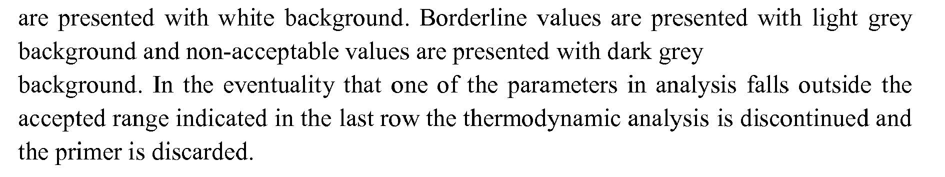

As can be seen from Table5, no suitable combinations could be identified by designing the T790M assay in inverse configuration.

### II3) Oligoblocker for the allele-specific T790M assay

We generated several oligoblocker that target the wild-type sequence for T790, and tested their characteritics in combination with the primers.

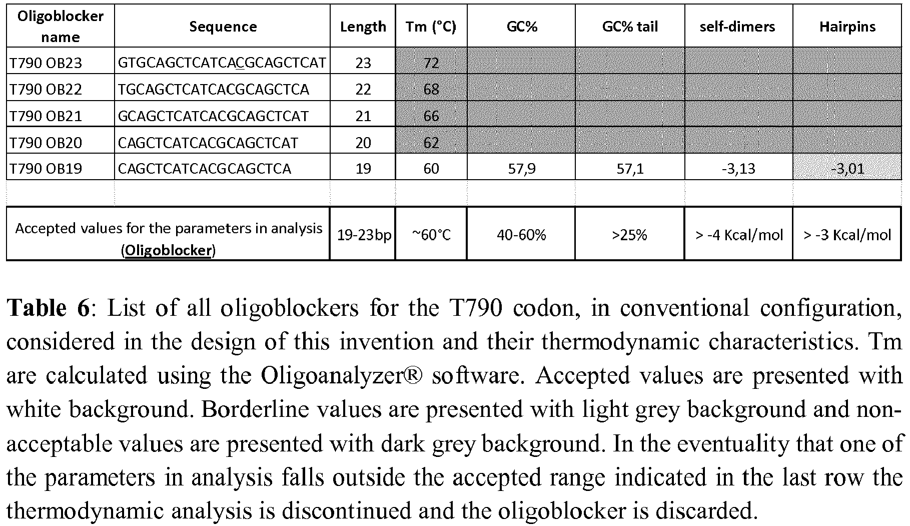

The best candidate was T790 OB19, despite the possibility of hairpins with borderline values (-3.01Kcal/mol). The hairpin was predicted to open at a temperature of 43.7°C. Given that the oligoblocker annealing takes place at a temperature of 60°C, the hairpin should not cause any efficiency problem. All further thermodynamic analyses were thus conducted considering T790 OB 19 as best oligoblocker candidate.

### II4) Thermodynamic analysis on all candidate primers and oligoblockers

The following heterodimers combinations were considered for all the primers/oligoblocker candidates that met the thermodynamic criteria described in the previous sections:
∘ Allele-specific primer + paired WT primer
∘ Allele-specific primer + oligoblocker
∘ Paired WT primer + oligoblocker

The results are presented in the following table.

**Table 7: Evaluation of heterodimers presence for all oligonucleotide candidates evaluated for the allele-specific system.**

| **Oligonucleotide combination** | **ΔG (Kcal/mol)** | **Validated? Y/N** |
|---|---|---|
| T790M 16 + T790 P19/72bp | none | Y |
| T790M 17 + T790 P19/72bp | none | Y |
| T790M 17 + T790 P20/74bp | none | Y |
| T790M 16 + T790 OB19 | -3,7 | Y |
| T790M 17 +T790 OB19 | -3,7 | Y |
| T790 P19/72bp + T790 OB19 | -0,08 | Y |
| T790 P20/74bp + T790 OB19 | -0,08 | Y |
| | | |
| Accepted values for the parameters in analysis | >-4 Kcal/mol | Y |

The results show all oligonucleotide candidates meet the thermodynamic criteria required to ensure absence of non-specific amplification and to improve the amplification efficiency.

### II5) Design of the T790M wild-type primer set

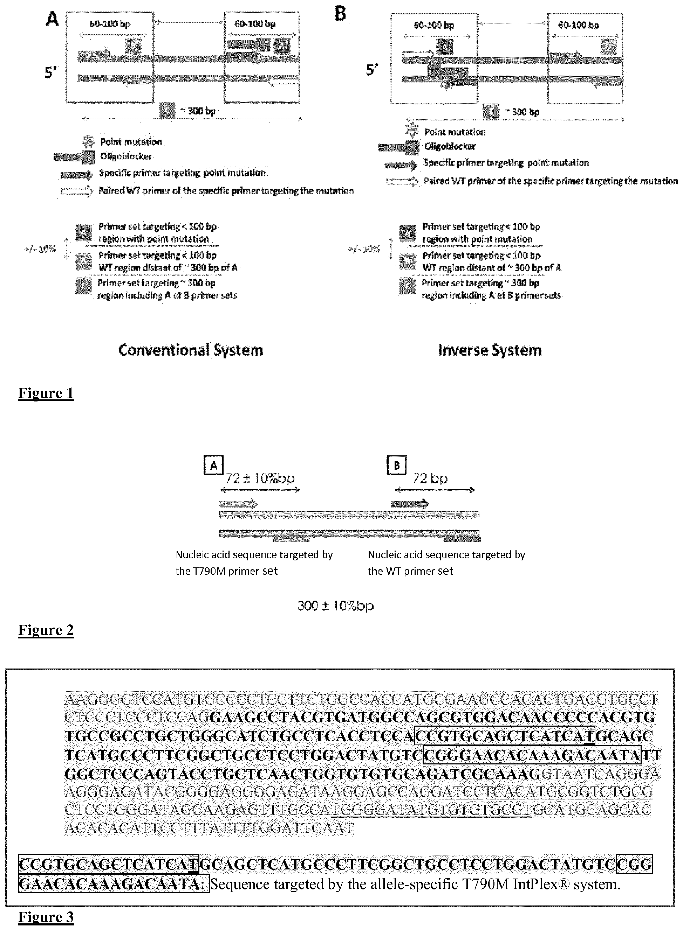

To produce optimal T790M Wild-Type primer sets, we chose to target the DNA region shown in **Fig.3****.**

### T790M wild-type set candidates

The tested primer candidates are disclosed in the following table.

**Table 8: Evaluation of all thermodynamic parameters considered to select appropriate oligonucleotides for the T790M wild-type set. Accepted values are presented with white background.**

| **Primer name** | **Sequence** | **Length** | **Tm (°C)** | **GC%** | **GC% tail** | **self-dimers** | **Hairpins** | **Heterodimers** | **Validated (Y/N)** |
|---|---|---|---|---|---|---|---|---|---|
| T790 WT L | CCTGTGCTAGGTCTTTTGC | 19 | 58 | 52,6 | 42,9 | -1,46 | -1,34 | None | Y |
| T790 WT R | CATGAATGCGATCTTGAGT | 19 | 54 | 42,1 | 42,9 | -2,17 | none | | Y |
| | | | | | | | | | |
| Accepted values for the parameters in analysis **(wild-type primer set)** | | 16-22bp | 55-60°C ΔTm with paired primer <5°C | 40-60% | >25% | > -4 Kcal/mol | > -3 Kcal/mol | > -4 Kcal/mol | Y |

Our results confirm the candidate primers exhibit the required characteristics. The pair was thus validated for further analysis.

### Blat alignment of the validated primer pairs in conventional sense

The Blat local alignment highlighted the presence of a secondary homology region. Nevertheless, by analysing the structure and extension of the homology region reported on chr14, we concluded that it would at most produce just a partial annealing region for the T790 WT L primer but, as no homology is present in the target region of the T790 WT R primer, no secondary product would be expected to be produced. This primer combination could thus be validated.

The oligonucleotide candidates of the T790M WT set met the thermodynamic criteria required to ensure absence of non-specific amplification and to improve the amplification efficiency.

**II6) In vitro validation of the T790M primer pairs**

**T790M 18** + **T790 P20** + **T790 OB19**

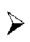 **Specificity**

The specificity of the combination was tested. They showed one peak only, indicating the specificity criteria are met.

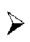 **Non-specificity**

A substantial non-specificity was observed when using this oligonucleotide combination. This combination is thus not optimal.

**T790M 18** + **T790 P19** + **T790 OB19**

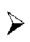 **Specificity**

The specificity of the combination was tested. The results show one peak only, indicating the specificity criteria are met

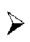 **Non-specificity**

Melting curves of genomic DNA WT for *EGFR* T790M from the Difi cell line was compared to the melting curves of commercial DNA mutant for *EGFR* T790M using this combination of oligonucleotides. A substantial non-specificity was observed when using this oligonucleotide combination. This combination is thus not optimal.

**T790M 16** + **T790 P19** + **T790 OB19**

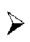 **Specificity**

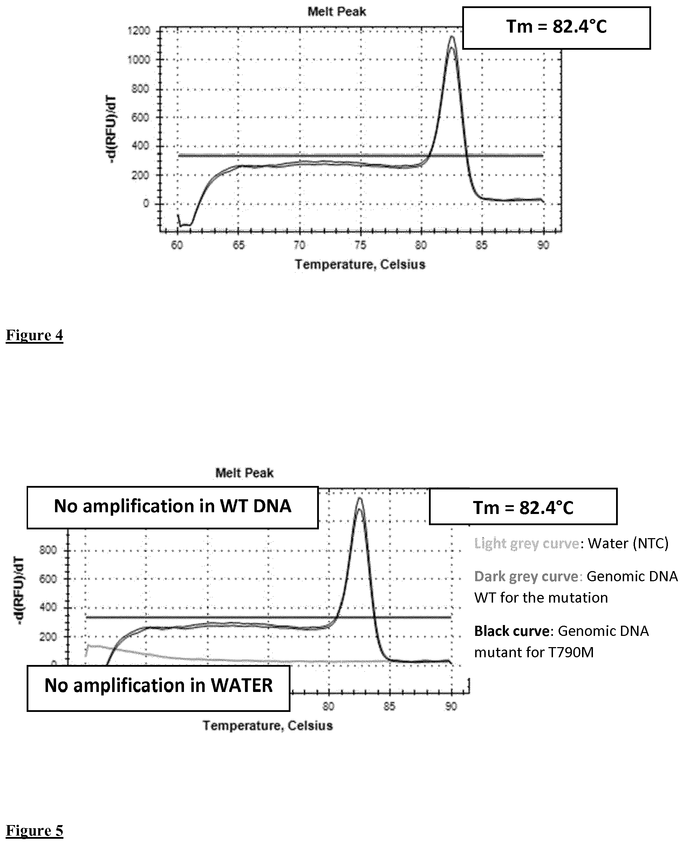

The specificity of the combination was tested. Melting curves of a commercial DNA mutant for *EGFR* T790M was determined using this oligonucleotide system. Just one peak was observed, as presented in **Fig 4****.** indicating the specificity criteria are met.

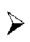 **Non-specificity**

Melting curves of genomic DNA WT for T790M from the Difi cell line was compared to melting curves of commercial DNA mutant for *EGFR* T790M using this combination of oligonucleotides (See **Fig. 5**). There is not any non-specificity observed. This oligonucleotide combination was thus further evaluated for efficacy.

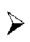 **Efficiency**

The efficiency analysis for the mutation-specific set comprised of T790M 16 + T790 P19 + T790 OB19 was performed in conjunction with the proposed combination for the T790 WT primer set. The results are presented in the following table.

**Table 9: Demonstration of the efficiency of the oligonucleotide system for the detection of EGFR T790M. The target T790M corresponds to the combination T790M 16 + T790 P19 + T790 OB19. The target T790 WT corresponds to the combination T790 WL L + T790 WT R. The target KRAS corresponds to an internal reference primer pair previously validated with efficiency close to 100%.**

| **Target** | **Content** | **Sample** | **Cq** | **[DNA] ng/µl** | **mean** | **mA%** |
|---|---|---|---|---|---|---|
| T790M | Positive control | T790M 50% Horizon standard | 26,59 | 0,38 | 0,39 | 62,94 |
| | | | 26,53 | 0,39 | | |
| T790 WT | | | 25,96 | 0,61 | 0,61 | |
| KRAS (internal reference) | | | 26,05 | 0,74 | 0,74 | 52,70% |

The observed mutant allele frequency of the T790M mutation-specific system on the T790 WT system is 62%, close to expected 50% reported by the supplier of the positive control used for the experiment.

The efficiency is thus appropriate and this oligonucleotide combination was further evaluated for sensitivity.

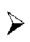 **Sensitivity**

The results are presented in the following table.

**Table 10: Demonstration of the high sensitivity of the method for the detection of the EGFR T790M point mutation. Serial dilutions of a commercial DNA control positive for the T790M mutation at expected mutational load of 0.25ng/µl were performed, up to 1/10000.**

| Sample | Cq | mutant [DNA] (ng/µl) | Specific Tm? | mean | Theoretical concentration |
|---|---|---|---|---|---|
| Ctrl+ T790M | 26,54 | 0,3342 | Y | 0,33705 | 0,25 |
| | 26,51 | 0,3399 | Y | | |
| Ctrl+ T790M 1/10 | 29,35 | 0,0455 | Y | 0,04433 | 0,025 |
| | 29,42 | 0,0431 | Y | | |
| Ctrl+ T790M 1/100 | 31,59 | 0,0092 | Y | 0,0045 | 0,0025 |
| | N/A | N/A | Y | | |
| | 31,56 | 0,0095 | Y | | |
| | N/A | N/A | N/A | | |
| | 31,76 | 0,0082 | Y | | |
| | N/A | N/A | N/A | | |
| Ctrl+ T790M 1/1000 | 35,44 | 0,000604 | Y | 0,00010 | 0,00025 |
| | N/A | N/A | N/A | | |
| | N/A | N/A | N/A | | |
| | N/A | N/A | N/A | | |
| | 36,84 | 0,000225 | N | | |
| | N/A | N/A | N/A | | |
| Ctrl+ T790M 1/10000 | N/A | N/A | N/A | | |
| | N/A | N/A | N/A | | |
| | N/A | N/A | N/A | | |
| | N/A | N/A | N/A | | |
| | N/A | N/A | N/A | | |
| | N/A | N/A | N/A | | |

The system could detect the T790M mutation down to a frequency of 0.1pg/µl. The sensitivity is thus very high.

The results here presented confirmed that the method comprised of the mutation-specific set T790M 16 + T790 P19 + T790 OB19 and of the wild-type set T790 WL L + T790 WT R exhibits high specificity efficiency and sensitivity.

### II7) Comparison with prior art method

We compared the efficiency of our method to the technique described in Zhao et al, Oncology Letters 11:2573:2579, 2016. This system uses a mismatch at the third base of 3' ends to decrease the non-specific combination of the allele-specific primer with the WT allele.

We employed the Zhao system and compared its sensitivity to our system on mutant genomic DNA and on circulating DNA from clinical samples. Zhao primer system targets a 106 bp mutant sequence whilst the invention targets a mutant sequence of 73 bp.

**Table 11: Comparison of the efficacy of the two systems for the qualitative and quantitative detection of the EGFR T790M mutation.**

| **Efficiency** | | Zhao system | | | | Invention system | |
|---|---|---|---|---|---|---|---|
| | | | | | | | |

| Target | Sample | Cq | [DNA] (ng/µL) extrait | Tm specificity | mean | Results obtained by DiaDX system | theorical conc. |
|---|---|---|---|---|---|---|---|
| B2/T790M Bk | CQ+T790M | 32,15 | 0,00326 | Y | 0,00358 | 0,34 | 0,5 |
| | | 31,87 | 0,00390 | Y | | | |
| | CQ+T790M1/10 | 37,25 | 0,00013 | Y | 0,00013 | 0,044 | 0,05 |
| | | 37,1 | 0,00014 | Y | | | |
| | CQ+ T790M1/100 | N/A | N/A | Y | 0,00029 | 0,009 | 0,005 |
| | | 35,94 | 0,00029 | | | | |
| | | N/A | N/A | | | | |
| | | N/A | N/A | | | | |
| | | N/A | N/A | | | | |
| | | N/A | N/A | | | | |
| | CQ+T7901/1000 | N/A | N/A | | N/A | 0,0006 | 0,0005 |
| | | N/A | N/A | | | | |
| | | N/A | N/A | | | | |
| | | N/A | N/A | | | | |
| | | N/A | N/A | | | | |
| | | N/A | N/A | | | | |

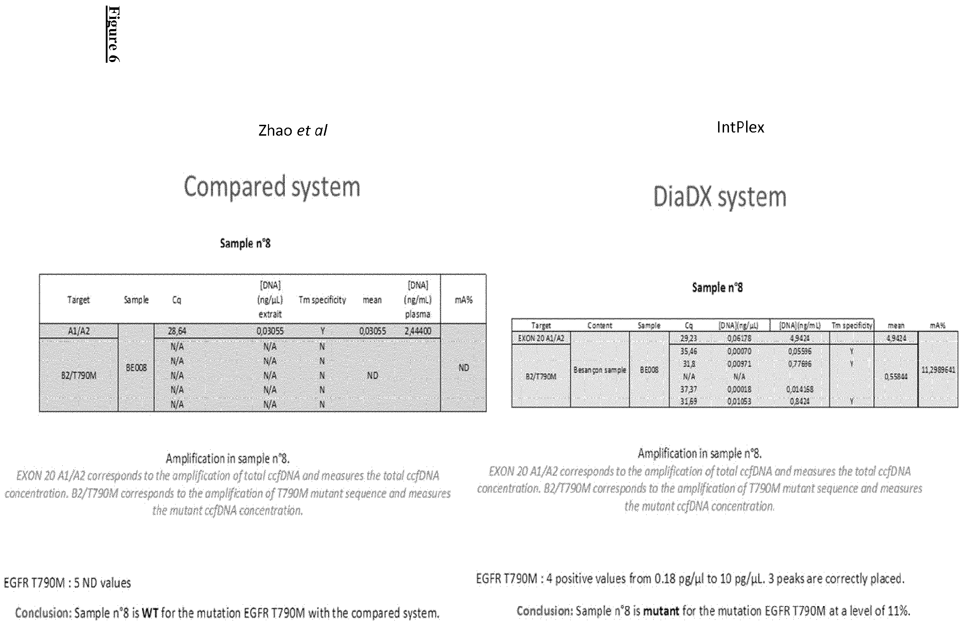

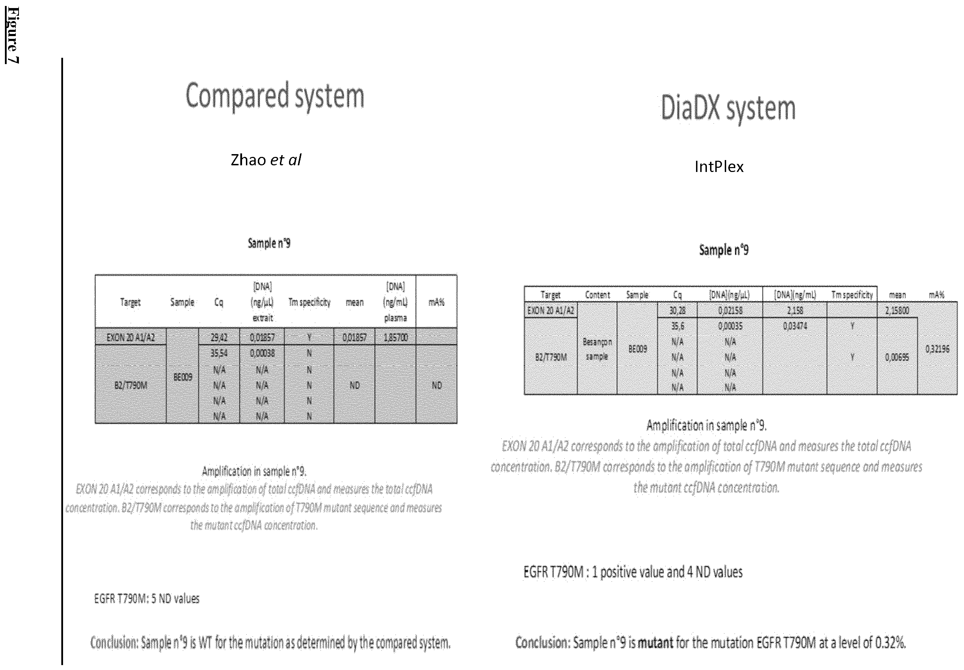

The result obtained show that Zhao system is less efficient than our method, as in same cases it does not detect mutations present at 11% in frequency when analysing clinical samples from lung cancer patients (See **Fig. 6**). Zhao system underestimates the real concentrations of mutant DNA and does not allow to determinate concentrations below 0.05 ng/µL (See **Fig. 7**).

Such results highlight the strong efficacy and sensitivity of our method compared with other mutation detection systems

### II8) Conclusions

Our method is specific, sensitive and more efficient than prior art techniques for detecting T790M. Among tested primer combinations, the best system to detect the *EGFR* T790M mutation is comprised by the mutation specific oligonucleotides: T790M 16 + T790 P19 + T790 OB19, in combination with the wild-type set T790 WT L + T790 WT R.

Other oligonucleotide combinations that allow efficient detection include:
- T790M 17 + T790 P19/72bp + T790 OB19
- T790M 17 + T790 P20/74bp + T790 OB19.

### III) L858R mutation

*EGFR* L858R is a substitution mutation. The L858R mutation results in an amino acid substitution at position 858, from a Leucine (L) to an Arginine (R). This mutation occurs within exon 21, which encodes part of the kinase domain. Nomenclature of *EGFR* L858R point mutation is c: 2573 T>G. It means that the 2573^{rd} nucleotide on the cDNA reference sequence is a Thymine that is substituted by a Guanine. After positioning the variant position on the cDNA sequence, it is positioned on the genomic DNA sequence.

The inventors have designed specific sets of primers allowing improved detection of the presence (or absence, or frequency) of such a mutation from a sample containing cfNA.

### III1) Design for the allele-specific L858R primer

Various allele-specific primers were considered in the design of this invention and their thermodynamic characteristics were analysed, as shown in Table 12:

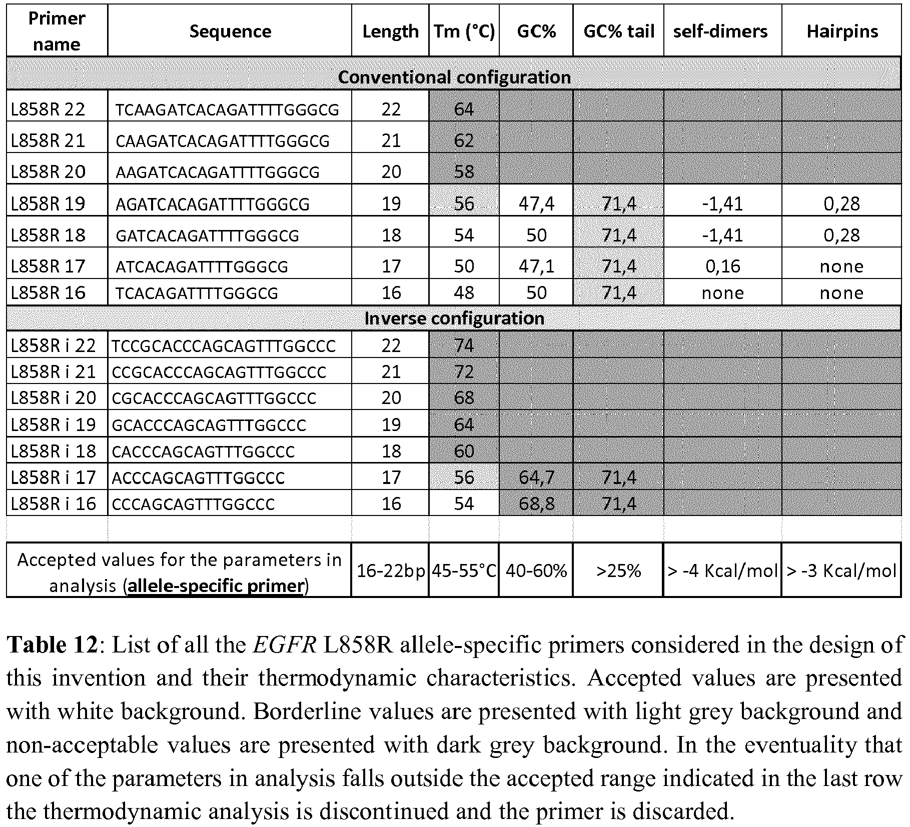

This preliminary analysis suggested that primer design in inverse sense is thermodynamically less favourable.

### III2) Paired Wild-Type primer

### Results obtained with Primer 3

All allele-specific primers that met our criteria (see Table 12) were used on Primer3 to find a compatible paired primer. Results are summarised in Table 13.

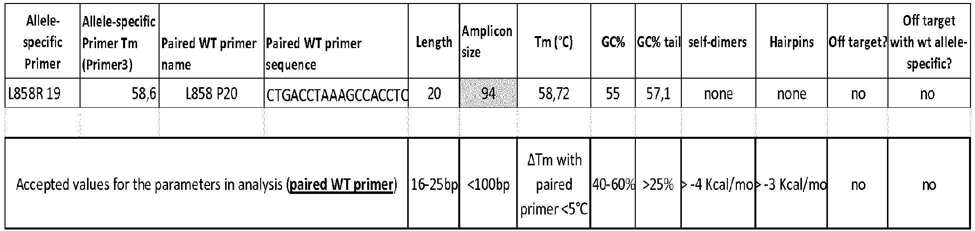

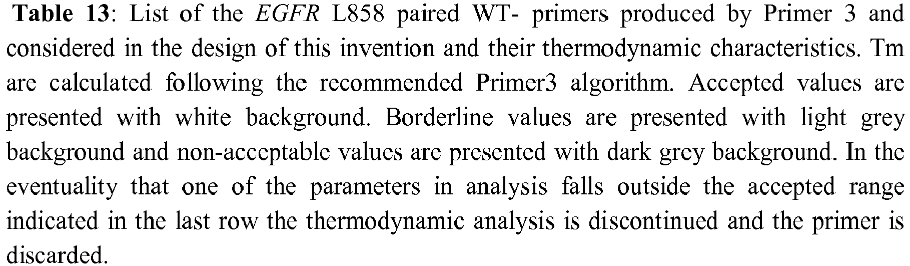

The only candidate identified via Primer3 has acceptable thermodynamic characteristics but it produces a PCR amplicon considered borderline for low frequency detection of mutations from tumour-derived cfDNA fragments. It was thus not possible to find appropriate primer using such approach.

### Manually generated primer pair candidates in conventional configuration

In view of the unsuccessful generation of a paired primer using conventional tools, we designed such primers manually. All designed primers are listed in the following table. Their characteristics were analysed.

**Table 14: List of a potential L858R paired WT- primers in conventional configuration manually generated and considered in the design of this invention and itsthermodynamic characteristics. Tm are calculated using the Oligoanalyzer® software. Accepted values are presented with white background and non-acceptable values are presented with dark grey background. In the eventuality that one of the parameters in analysis falls outside the accepted range indicated in the last row the thermodynamic analysis is discontinued and the primer is discarded.**

| **Allele- specific Primer** | **Allele- specific Primer™** | **Paired WT primer name** | **Paired WT primer sequence** | **Length** | **Amplico n size** | **Tm (°C)** | **GC%** | **3C% tai** | **self-dimers** | **Hairpins** | **Off target?** | **Off target with wt allele- specific?** |
|---|---|---|---|---|---|---|---|---|---|---|---|---|
| L858R 19 | 58,6 | L858 P20/2 | CTCCTTCTGCATGGTATTCT | 20 | 66 | 58 | 45 | 28,6 | -3,84 | none | no | no |
| | | | | | | | | | | | | |
| Accepted values for the parameters in analysis **(pairedWT primer)** | | | | 16-25bp | <100bp | ΔTm with paired primer <5°C | 40-60% | >25% | > -4 Kcal/mol | > -3 Kcal/mol | no | no |

The following combinations were found, that meet the thermodynamic criteria required:
- L858R 16 + L858 P20/2
- L858R 17 + L858 P20/2
- L858R 18 + L858 P20/2
- L858R 19 + L858 P20/2

### Blat alignment of the validated primer pairs in conventional sense

Blat alignments (Human GRCh38/hg38) for all the PCR amplicons generated by the combinations of primers meeting the thermodynamic criteria were performed. All primer pairs produced just one single amplicon at the expected chromosomal position. No other homologies are reported.

### III3) Oligoblocker desi₂n for the EGFR L858R position

The presence of a region of self-annealing prevents the design of the L858 oligoblocker with the WT base towards the middle of the oligonucleotide sequence. The oligoblocker was designed with the variant position towards its 3' end. The oligoblockers and their characteristics are described in the following table.

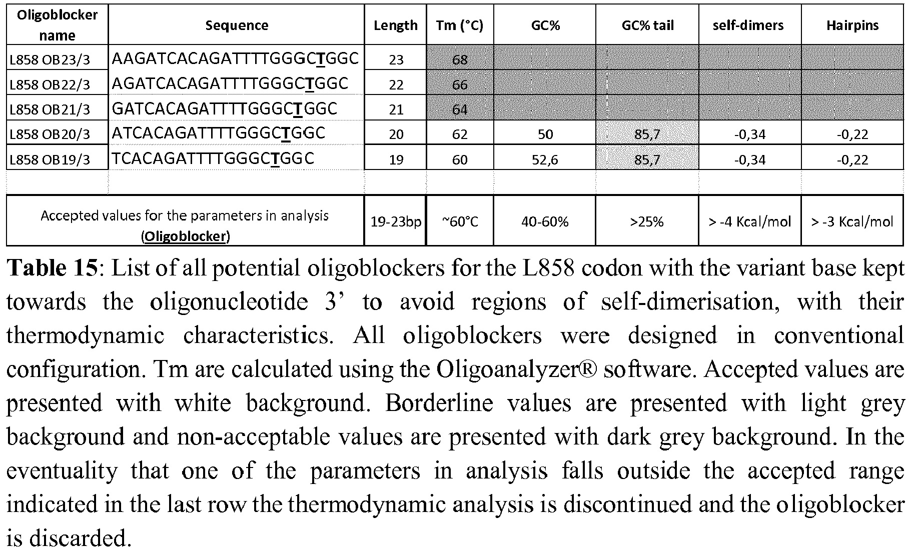

Oligoblockers L858 OB20/3 and L858 OB19/3 present the best thermodynamic characteristics.

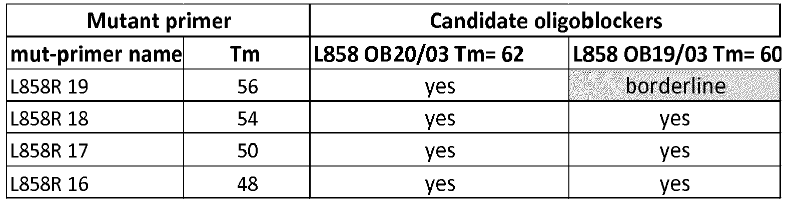

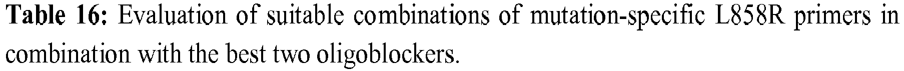

From the Tm analysis of Table 16, all the combinations of allele-specific primers and oligoblocker seem to fit the optimal criteria.

### III4) Thermodynamic analysis on all candidate primers and oligoblockers

The following heterodimers combinations were considered for all the primers/oligoblockers that met the thermodynamic criteria:
∘ Allele-specific primer + paired WT primer
∘ Allele-specific primer + oligoblocker
∘ Paired WT primer + oligoblocker

The results are presented in the following table:

**Table 17: Evaluation of heterodimers presence for all oligonucleotide candidates. The results show all oligonucleotide candidates meet the thermodynamic criteria required to ensure absence of non-specific amplification and to improve the amplification efficiency.**

| **Oligonucleotide combination** | **ΔG (Kcal/mol)** | **Validated? Y/N** |
|---|---|---|
| L858R 19 +L858 P20/2 | -1,92 | Y |
| L858R 19 + L858 OB19/3 | -0,34 | Y |
| L858 P20/2 + L858 OB19/3 | -1,92 | Y |
| L858R 18+L858 P20/2 | -1,92 | Y |
| L858R 18 + L858 OB19/3 | -0,34 | Y |
| L858R 17+L858 P20/2 | -1,92 | Y |
| L858R 17 + L858 OB19/3 | -0,34 | Y |
| | | |
| Accepted values for the parameters in analysis | >-4 Kcal/mol | Y |

### III5) Design of the L858 wild-type primer set

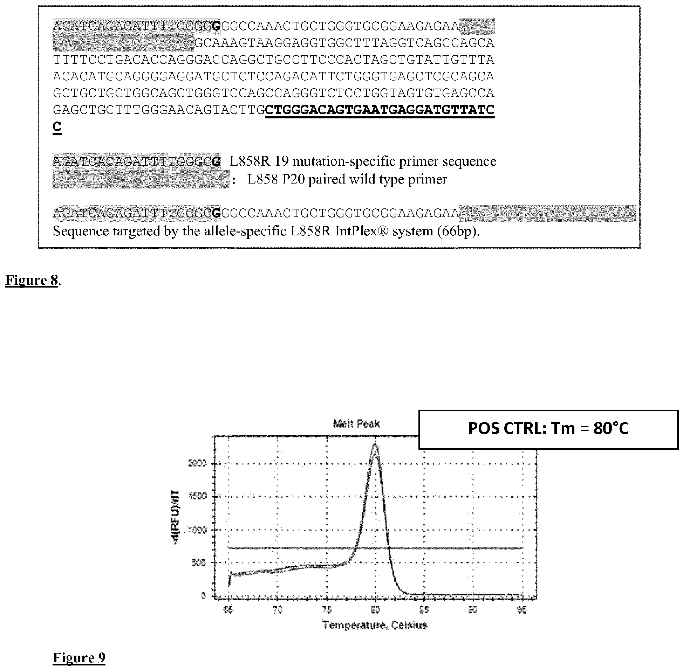

To produce optimal L858R wild-type primer sets, we chose to target the DNA region presented in **Fig 8****.**

### L858 wild-type set candidates:

The tested primer candidates are disclosed in the following table:

**Table 18: Evaluation of all thermodynamic parameters considered to select appropriate oligonucleotides for the L858 wild-type set. Accepted values are presented with white background.**

| **Length** | **Tm (°C)** | **GC%** | **GC% tail** | **self-dimers** | **Hairpins** | **Heterodimers** | **Validated (Y/N)** |
|---|---|---|---|---|---|---|---|
| 17 | 54 | 58,8 | 42,9 | none | none | -1,81 | Y |
| 18 | 54 | 50 | 57,1 | none | none | | Y |
| | | | | | | | |
| 16-22bp | 54-60°C ΔTm with paired primer <5°C | 40-60% | >25% | > -4 Kcal/mol | > -3 Kcal/mol | > -4 Kcal/mol | Y |

Our results confirm the primers satisfy the required optimal characteristics.

### Blat alignment of the validated primer pairs in conventional sense

Blat alignments (Human GRCh38/hg38) for all the PCR amplicons generated by the combinations of primers were performed.

The oligonucleotide candidates of the L858 WT set meet the thermodynamic criteria required to ensure absence of non-specific amplification and to improve the amplification efficiency.

**III6) *In vitro* validation of the L858R IntPlex® system**

**L858R 19+ L858 P20** + **L858 OB19/3**

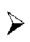 **Specificity**

The specificity of the combination was tested. The results are presented in Fig 9. They show one peak only, which indicates this oligonucleotide combination meets the specificity criteria.

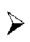 **Non-specificity**

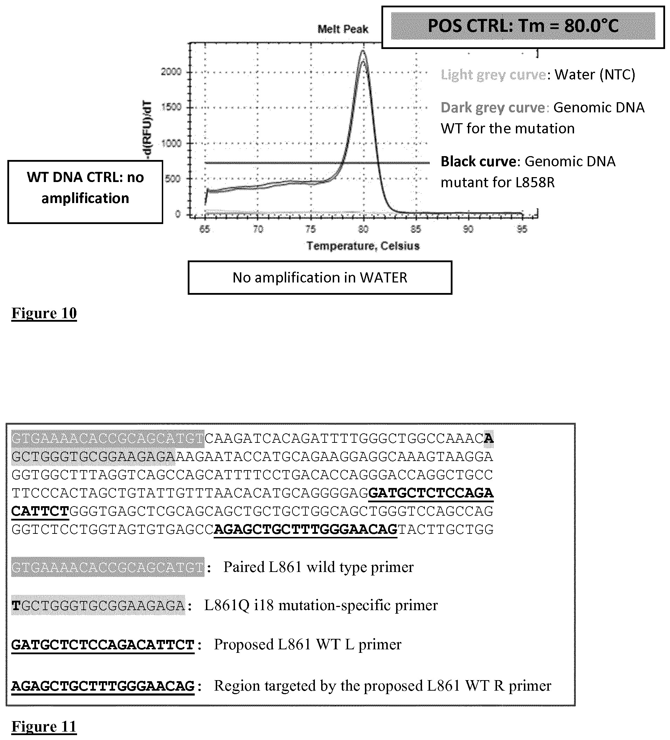

Melting curves of genomic DNA WT for the L858R mutation were compared to melting curves of commercial DNA mutant for L858R using this oligonucleotide combination (See **Fig.10**). There was not any non-specificity observed. This oligonucleotide combination was thus evaluated for efficiency.

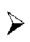 **Efficiency**

The efficiency analysis for the mutation-specific set comprised of L858R 19+ L858 P20 + L858 OB19 was performed in conjunction with the proposed combination for the L858WT primer set. The results are presented in the following table.

**Table 19: Demonstration of the efficiency of the oligonucleotide system in analysis for the detection of EGFR L858R. The mA% is calculated as ratio between the quantification performed on a commercial positive control for L858R, with expected frequency of 50%.**

| Target | Content | Sample | Cq | [DNA] (ng/µl) | Mean | mA% |
|---|---|---|---|---|---|---|
| L858R 19+ L858 P20 + L858 OB19/3 | Neg control | Di-Fi DNA | N/A | N/A | | |
| | | | N/A | N/A | | |
| | | | N/A | N/A | | |
| | NTC | Water | N/A | N/A | | |
| | | | N/A | N/A | | |
| | | | N/A | N/A | | |
| | Positive control | Horizon std L858R 50% | 27,73 | 0,238 | 0,24 | 76,58% |
| | | | 27,65 | 0,248 | | |
| L858 WT Assay (L858 WT L + L858 WT R) | | | 27,21 | 0,329 | 0,32 | |
| | | | 27,32 | 0,306 | | |

The observed mutant allele frequency of the L858R mutation-specific system on the L858WT system is 76%, close to expected 50% reported by the supplier of the positive control used for the experiment.

The efficiency is thus appropriate and this oligonucleotide combination was further evaluated for sensitivity.

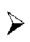 **Sensitivity**

The results are presented in the following table.

| Sample | Cq | mutant [DNA] (ng/µl) | Specific Tm? | mean |
|---|---|---|---|---|
| Ctrl+ L858R | 28,7 | 0,0722 | Y | 0,07745 |
| | 28,51 | 0,0827 | Y | |
| Ctrl+ L858R 1/10 | 31,05 | 0,0136 | Y | 0,00715 |
| | 35,33 | 0,0007 | Y | |
| Ctrl+ L858R 1/100 | N/A | N/A | Y | 0,0001 |
| | N/A | N/A | Y | |
| | N/A | N/A | Y | |
| | N/A | N/A | N/A | |
| | 35,62 | 0,0005 | Y | |
| | N/A | N/A | N/A | |
| Ctrl+ L858R 1/1000 | N/A | N/A | N/A | |
| | N/A | N/A | N/A | |
| | N/A | N/A | N/A | |

**Table 20: Demonstration of the high sensitivity of the method for the detection of the EGFR L858R point mutation. Serial dilutions of a commercial DNA control positive for the L858R mutation were performed, up to 1/10000.**

| | | | | |
|---|---|---|---|---|
| | N/A | N/A | N/A | |
| | N/A | N/A | N/A | |
| | N/A | N/A | N/A | |
| Ctrl+ L858R 1/10000 | N/A | N/A | N/A | |
| | N/A | N/A | N/A | |
| | N/A | N/A | N/A | |
| | N/A | N/A | N/A | |
| | N/A | N/A | N/A | |
| | N/A | N/A | N/A | |

The system can detect the L858R mutation down to a frequency of 0.1pg/µl. The sensitivity is thus very high.

The results here presented confirm that the method comprised of the mutation-specific set L858R 19+ L858 P20 + L858 OB19/3 and of the wild-type set L858 WT L + L858 WT R exhibits high specificity, efficiency and sensitivity.

### III7) Conclusion

Our method allows detection of L858R with high specificity, sensitivity and efficiency. Among tested primers, the optimal combination is L858R 19 + L858 P20/64bp + L858 OB 19/3. Other suitable primer combinations include:
- L858R 19 + L858 P20 + L858 OB20/3
- L858R 18 + L858 P20 + L858 OB19/3
- L858R 18 + L858 P20 + L858 OB20/3
- L858R 17 + L858 P20 + L858 OB19/3
- L858R 17 + L858 P20 + L858 OB20/3
- L858R 16 + L858 P20 + L858 OB19/3
- L858R 16 + L858 P20 + L858 OB20/3

### IV)L861Q mutation

*EGFR* L861Q is a substitution mutation. The L861Q mutation results in an amino acid substitution at position 861, from a Leucine (L) to a Glutamine (Q). This mutation occurs within exon 21, which encodes part of the kinase domain. Nomenclature of *EGFR* L861Q point mutation is c: 2582 T>A. It means that the 2582^{th} nucleotide on the cDNA reference sequence is a Thymine that is substituted by an Adenine.

The inventors have designed specific sets of primers allowing improved detection of the presence (or absence, or frequency) of such a mutation from a sample containing cfNA.

### IV1) Design of the allele-specific EGFR L861Q primer

Various allele-specific primers were considered in the design of this invention and their thermodynamic characteristics were analysed, as shownin Table 23:

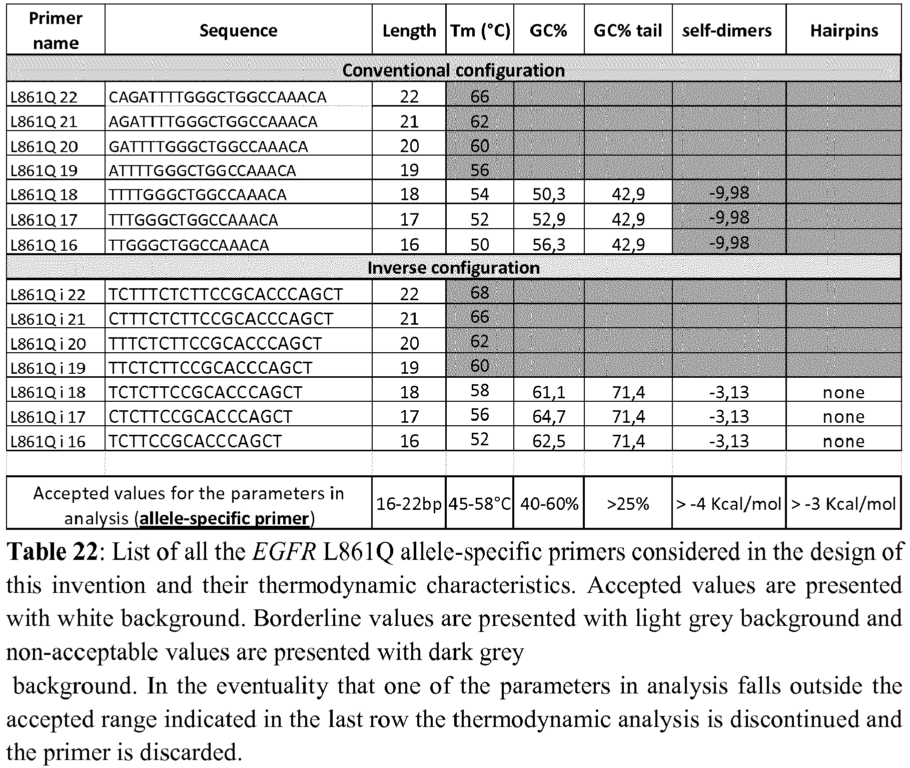

### IV2) Paired Wild-Type primer

### Results obtained with Primer 3

All allele-specific primers that met our IntPlex® criteria (see Table 22) were used on Primer3 to find a compatible paired primer. Unfortunately, no suitable primers were identifiable through this approach.
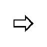 New potential paired-wild type primer candidates will be manually designed to overcome this issue.

### Manually generated primer pair candidates in inverse configuration

In view of the unsuccessful generation of a paired primer using conventional tools, we designed such primers manually. All primers are listed in the following table. There characteristics were analysed.

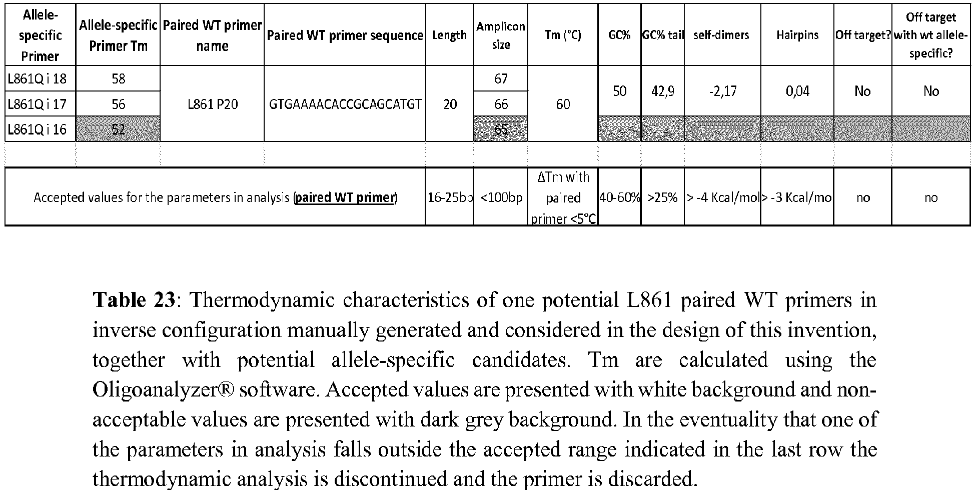

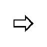 The following combinations were thus found, that meet the thermodynamic criteria required:
   - L861Q i18+ L861 iP20
   - L861Q i17+ L861 iP20

### Blat alignment of the validated primer pairs in conventional sense

Blat alignments (Human GRCh38/hg38) for all the PCR amplicons generated by the combinations of primers meeting the thermodynamic criteria were performed.
The oligonucleotide candidates of the L861Q set met the thermodynamic criteria required to ensure absence of non-specific amplification and to improve the amplification efficiency.

### IV3) Oligoblocker for the allele-specific EGFR L861Q assay

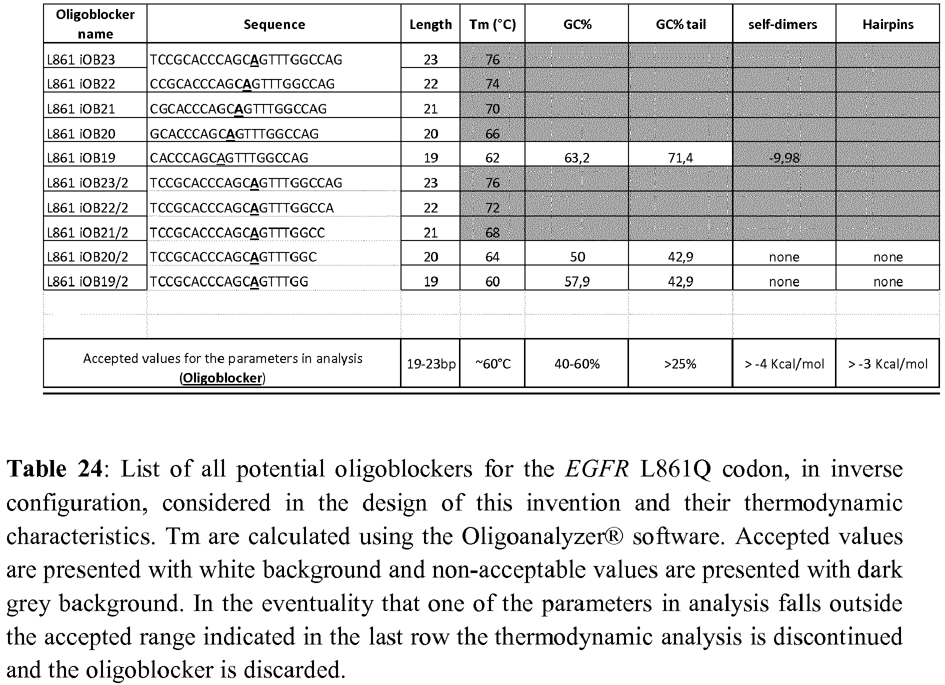

### IV4) Thermodynamic analysis on all candidate primers and oligoblockers

The following heterodimers combinations to consider for all the primers/oligoblocker candidates that met the thermodynamic criteria described in the previous sections:
∘ Allele-specific primer + paired WT primer
∘ Allele-specific primer + oligoblocker
∘ Paired WT primer + oligoblocker

The results are presented in the following table:

**Table 25: Evaluation of heterodimers presence for all oligonucleotide candidates evaluated for the allele-specific system.**

| **Oligonucleotide combination** | **ΔG (Kcal/mol)** | **Validated? Y/N** |
|---|---|---|
| L861Q i17+ L861 iP20 | -1,53 | Y |
| L861Q i18+ L861 iP20 | -1,53 | Y |
| L861Q i17+L861 iOB19/2 | -1,81 | Y |
| L861Q i17+L861 iOB20/2 | -1,81 | Y |
| L861Q i18+L861 iOB19/2 | -1,81 | Y |
| L861Q i18+L861 iOB20/2 | -1,81 | Y |
| L861 iP20+iOB19/2 | -2,02 | Y |
| L861 iP20+iOB20/2 | -2,02 | Y |
| | | |
| Accepted values for the parameters in analysis | >-4 Kcal/mol | Y |

The results show all oligonucleotide candidates meet the thermodynamic criteria required to ensure absence of non-specific amplification and to improve the amplification efficiency.

### IV5) Design of the EGFR L861Q wild-type set

To produce optimal L861Q Wild-Type primer sets, we chose to target the DNA region shown in **Fig 11****.**

### L861 wild-type set candidates

The tested primer candidates are disclosed in the following table.

**Table 26: Evaluation of all thermodynamic parameters considered to select appropriate oligonucleotides for the L861 wild-type set. Accepted values are presented with white background.**

| **Primer name** | **Sequence** | **Length** | **Tm (°C)** | **GC%** | **GC% tail** | **self-dimers** | **Hairpins** | **Heterodimers** | **Validated (Y/N)** |
|---|---|---|---|---|---|---|---|---|---|
| L861 WT R | CTGTTCCCAAAGCAGCTCT | 19 | 58 | 52,6 | 57,1 | -3,13 | -0,22 | -3,48 | Y |
| L861 WT L | GATGCTCTCCAGACATTCT | 19 | 56 | 47,4 | 28,6 | -0,22 | -0,1 | | Y |
| | | | | | | | | | |
| Accepted values for the parameters in analysis **(wild-typeprimerset** | | 16-22bp | 55-60°C ΔTm with paired primer <5°C | 40-60% | >25% | > -4 Kcal/mol | > -3 Kcal/mol | > -4 Kcal/mol | Y |

Our results confirm the candidate primers exhibit the required characteristics. The pair was thus validated for further analysis.

### Blat alignment of the validated primer pairs in inverse configuration

Blat alignments (Human GRCh38/hg38) for the PCR amplicons generated by the combinations of primers meeting the thermodynamic criteria were performed.

The Blat local alignment highlighted the presence of secondary homology regions. Nevertheless, none of the secondary homology region overlapped with the primer sequences. Hence no secondary product was produced. This primer combination could hence be validated. The oligonucleotide candidates of the L861 WT set met the thermodynamic criteria required to ensure absence of non-specific amplification and to improve the amplification efficiency.

***IV6)* *In vitro* validation of the L861Q primer pairs**

**L861Q i18** + **L861 iP20** + **L861 iOB20/2**

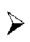 **Specificity**

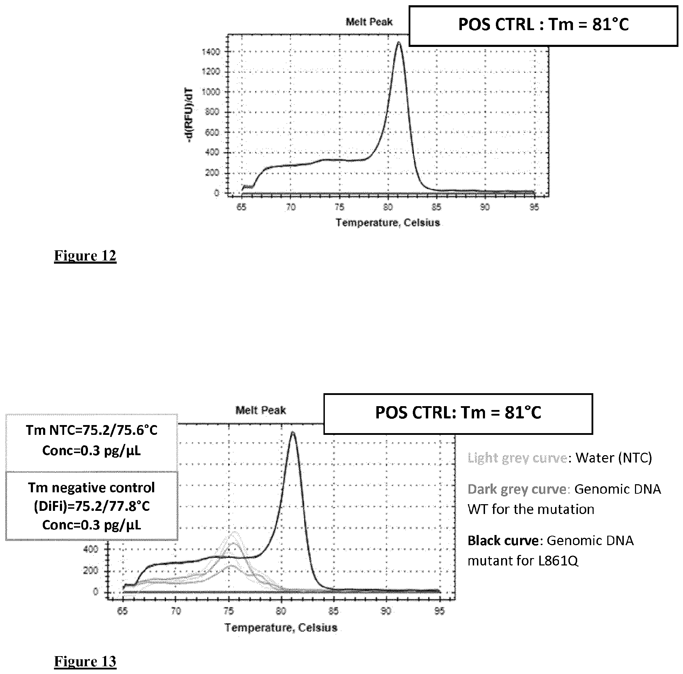

The specificity of the combination was tested. The results are presented in Fig 12. They showed one peak-only, indicating the specificity QC criteria are met.

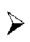 **Non-specificity**

Melting curves of genomic DNA WT for the L861 Q mutation were compared to melting curves of commercial DNA mutant for L861Q using this oligonucleotide combination (See **Fig.13**). There was not any non-specificity observed. This oligonucleotide combination was thus evaluated for efficiency.

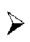 **Efficiency**

The efficiency analysis for the mutation-specific set comprised of L861Q i18 + L861 iP20 + L861 iOB20 was performed in conjunction with the proposed combination for the L861 WT primer set. The results are presented in the following table.

**Table 27: Demonstration of the efficiency of the oligonucleotide system for the detection of EGFR L861Q.**

| **Target** | **Content** | **Sample** | **Cq** | **[DNA] (ng/µl)** | **Tm specific? (Y/N)** | **Mean** | **mA%** |
|---|---|---|---|---|---|---|---|
| L861Q i18+L861 iP20+L861 iOB20/2 | Neg control | Di-Fi DNA | 36,88 | N/A | N | | |
| | | | 37,67 | N/A | N | | |
| | NTC | Water | 33,38 | N/A | N | | |
| | | | 36,24 | N/A | N | | |
| | Positive control | Horizon std L861Q 50% | 27,63 | 0,1825 | Y | 0,175 | 36,61% |
| | | | 27,75 | 0,1671 | Y | | |
| L861 WT Assay (L861 WT L + L861 WT R) | | | 25,66 | 0,4592 | Y | 0,477 | |
| | | | 25,55 | 0,4957 | Y | | |

The observed mutant allele frequency of the L861Q mutation-specific system on the L861Q WT system is 62%, close to expected 50% reported by the supplier of the positive control used for the experiment.

The efficiency is thus appropriate and this oligonucleotide combination was further evaluated for sensitivity.

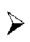 **Sensitivity**

The results are presented in the following table.

**Table 28: Demonstration of the high sensitivity of the method for the detection of the EGFR L861Q point mutation. Serial dilutions of a commercial DNA control positive for the L861Q mutation were performed, up to 1/10000.The system can detect the L861Q mutation down to a frequency of 0.2pg/µl. The sensitivity is thus very high.**

| Sample | Cq | [DNA] (ng/µL) extract | Tm specificity | mean |
|---|---|---|---|---|
| C+ L861Q | 27,63 | 0,182500 | Y | 0,174800 |
| | 27,75 | 0,167100 | Y | |
| C+861 1/10 | 30,96 | 0,016750 | Y | 0,017160 |
| | 30,89 | 0,017570 | Y | |
| C+ 8611/100 | 33,3 | 0,003115 | Y | 0,001519 |
| | 34,76 | 0,001090 | Y | |
| | 35,45 | 0,000666 | Y | |
| | 33,47 | 0,002750 | Y | |
| | 34,88 | 0,001001 | Y | |
| | 35,86 | 0,000492 | Y | |
| C+ 8611/1000 | 36,33 | 0,000353 | N | 0,000220 |
| | 37,36 | 0,000168 | N | |
| | 36,86 | 0,000240 | N | |
| | 35,3 | 0,000739 | N | |
| | 35,27 | 0,000757 | N | |
| | 36,98 | 0,000220 | Y | |
| C+ 8611/10000 | 35,85 | 0,000499 | N | ND |
| | 37,34 | 0,000171 | N | |
| | 35,62 | 0,000588 | N | |
| | 39 | 0,000052 | N | |
| | 37,76 | 0,000126 | N | |
| | 37,75 | 0,000127 | N | |

### IV7) Conclusions

Our method exhibits high specificity, efficiency and sensitivity. The results here presented confirm that among all tested primer combinations the method comprised of the mutation-specific set **L861Q i18** + **L861 iP20** + **L861 iOB20/2** and of the wild-type set **L861 WT L** + **L861 WT R** is the best system to detect the *EGFR* L861Q.

Other systems that allow efficient detection include:
- L861Q i17+ L861 iP20 + iOB19/2
- L861Q i17+ L861 iP20 + iOB20/2
- L861Q i18+ L861 iP20 + iOB19/2

### V) EGFR S768I

*EGFR* S768I is a substitution mutation. The S768I mutation results in an amino acid substitution at position 768, from a Serine (S) to an Isoleucine (I). This mutation occurs within exon 20, which encodes part of the kinase domain. Nomenclature *of EGFR* S768I point mutation is c: 2303 G>T. It means that the 2303^{th} nucleotide on the cDNA reference sequence is a Guanine that is substituted by a Thymine.

The inventors have designed specific sets of primers allowing improved detection of the presence (or absence, or frequency) of such a mutation from a sample containing cfNA.

### V1) Design for the allele-specific EGFR S768I primer

Various allele-specific primers considered in the design of this invention and their thermodynamic characteristics were analysed, as shown in **Table 29:**

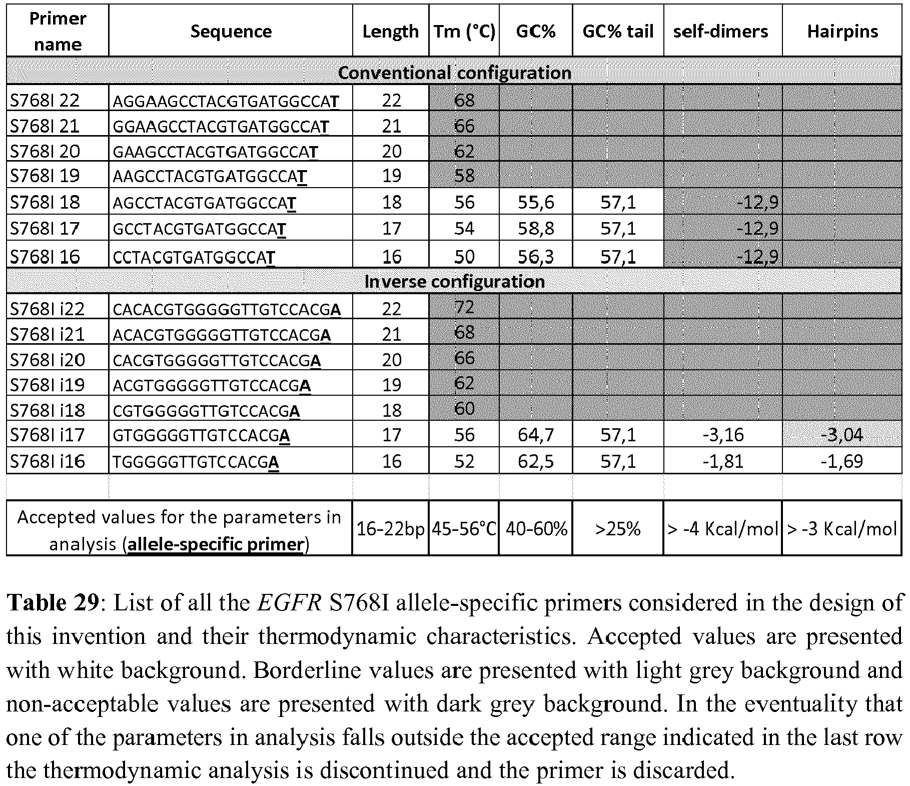

### V2) Paired Wild-Type primer

### Results obtained with Primer 3

All allele-specific primers that met our criteria (see Table 29) were used on Primer3 to find a compatible paired primer. Results are summarised in Table 30.

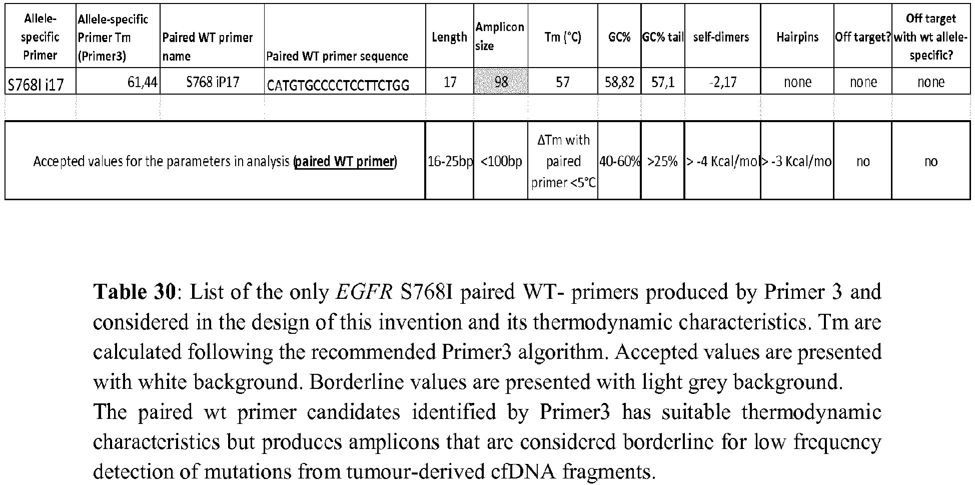

The paired-wild type primer was thus manually designed following the thermodynamic criteria described in the previous sections to try and reduce the PCR amplicon size.

Manually generated primer pair candidates in inverse configuration

Paired wild-type primers were manually generated to reduce the PCR amplicon size. All primers are listed in the following table. Their characteristics were analysed.

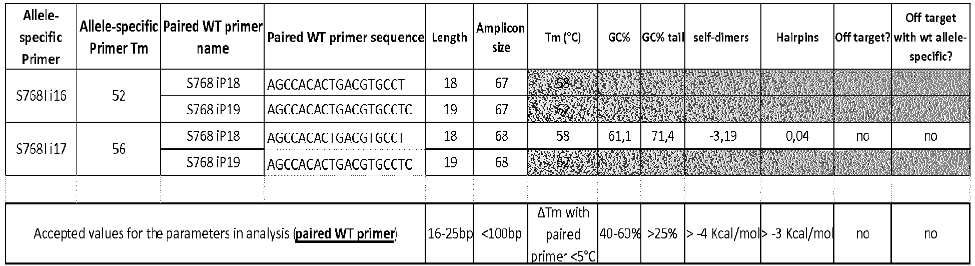

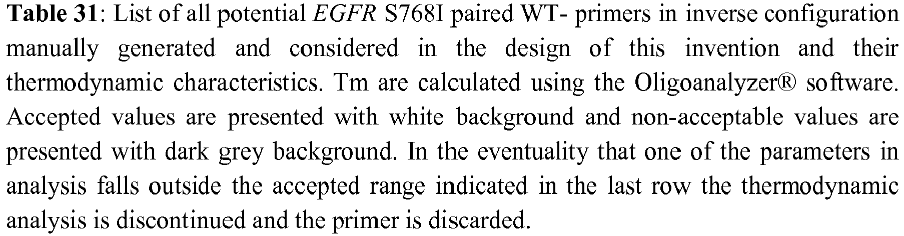

The following combinations were thus found, that met the thermodynamic criteria required:
- S768I i17 + S768 iP18

### Blat alignment of the validated primer pairs in inverse configuration

Blat alignments were produced (Human GRCh38/hg38) for the PCR amplicons generated by the combination of primers meeting the thermodynamic criteria.

The primer pair in analysis produced just one single amplicon at the expected chromosomal position. No other homologies are reported.

### V3) Oligoblocker for the EGFR S768 position

We generated several oligoblockers that target the wild-type sequence for S768 and tested their characteristics in combination with the primers.

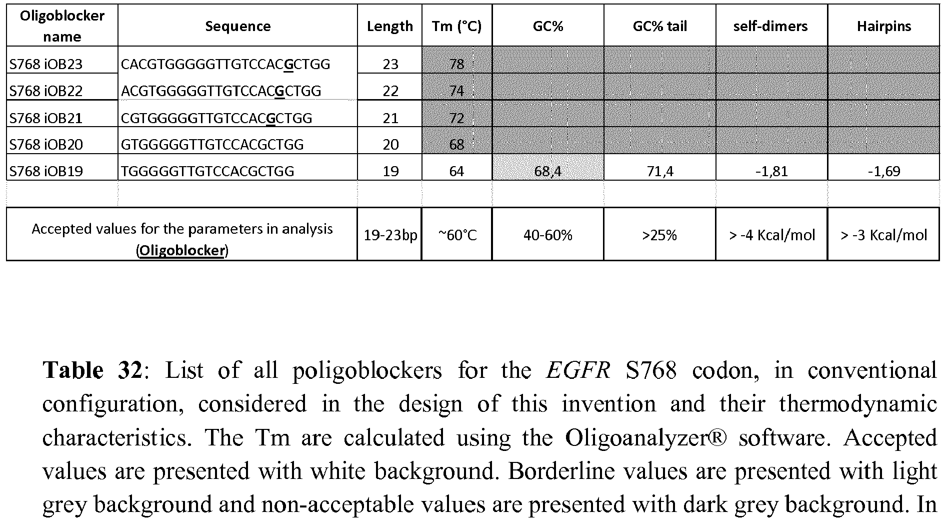

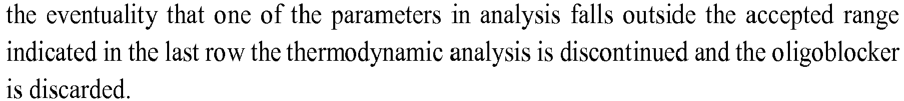

The best candidate is the S768 i OB19, despite slightly high GC% and Tm, as all other combination presented excessive too high Tm. All further thermodynamic analyses were thus conducted considering S768 i OB19 as best oligoblocker candidate.

### V4) Thermodynamic analysis on all candidate primers and oligoblockers

The following heterodimers combinations were considered for all the primers/oligoblocker candidates that met the thermodynamic criteria described in the previous sections:
∘ Allele-specific primer + paired WT primer
∘ Allele-specific primer + oligoblocker
∘ Paired WT primer + oligoblocker

The results are presented in the following table.

**Table 33: Evaluation of heterodimers presence for all oligonucleotide candidates evaluated for the allele-specific system.**

| **Oligonucleotide combination** | **ΔG (Kcal/mol)** | **Validated? Y/N** |
|---|---|---|
| 5768I i17+S768 iP18 | -3,7 | Y |
| 5768I i17+S768 iOB19 | -3,16 | Y |
| S768 iOB19+S768 iP18 | -3,7 | Y |
| | | |
| Accepted values for the parameters in analysis | >-4 Kcal/mol | Y |

The results show all oligonucleotide candidates meet the thermodynamic criteria required to ensure absence of non-specific amplification and to improve the amplification efficiency.

### V5) Design of the S768 wild-type set

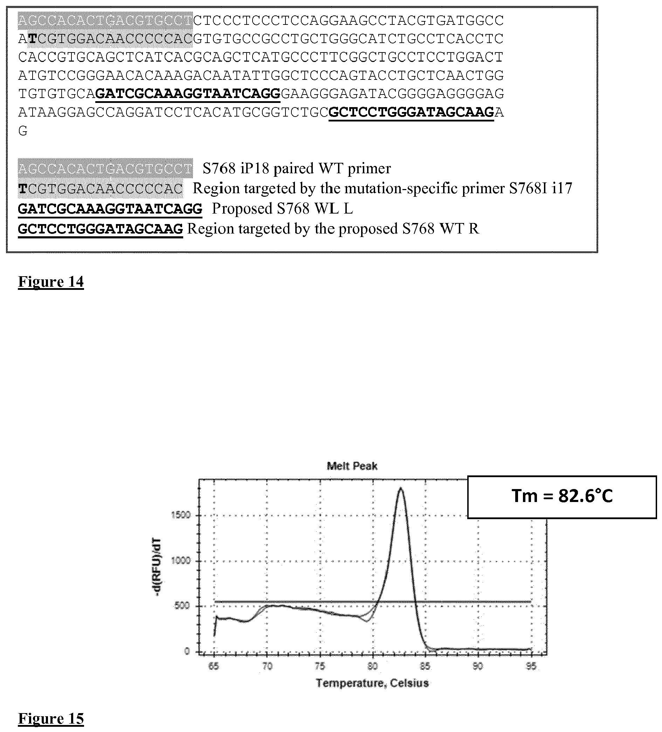

To produce optimak S768 wild-type sets, we chose to target the DNA region shown in Fig 14.
The tested primer candidates are disclosed in the following table

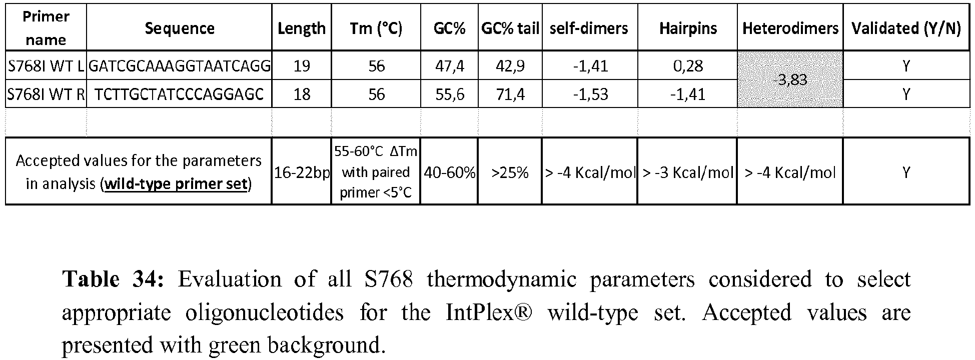

Our results confirm the candidate primers exhibit the required characteristics. The pair was thus validated for further analysis.

### Blat alignment of the validated primer pairs in inverse configuration

Blat alignments were produced (Human GRCh38/hg38) for the PCR amplicons generated by the combination of primers meeting the thermodynamic criteria.
The Blat local alignment highlighted the presence of some secondary homology region. Nevertheless, by analysing the structure and extension of the homology region reported on chrX we concluded that it would at most produce a partial annealing region for the S768 WT L primer but, as no homology is present in the target region of the S768 WT R primer, no secondary product would be produced. None of the other homology regions spans the primer target sequence. This primer combination could hence be validated.

The oligonucleotide candidates of the S768 WT set met the thermodynamic criteria required to ensure absence of non-specific amplification and to improve the amplification efficiency.

**V6) *In vitro* validation of the S768I primer pairs**

**S768I i16 + S768 iP18 +S768 iOB19**

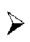 **Specificity**

The specificity of the combination was tested. The results showed one peak-only, indicating the specificity QC criteria are met.

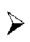 **Non-specificity**

A slight amplification was observed in the negative control and in the NTC, with a 4°C Tm difference from the expected amplification peak of the positive control. Moreover, the concentration of the non-specific amplification is <0.5pg/µl, in compliance with IntPlex® acceptance QC criteria.

As the Tm difference between the tested mutation-specific primer S768I i16 and the paired WT primer S768 iP18 is 6°C, the mutation-specific primer S768I i17 will be also tested.

**S768I i17 + S768 iP18 +S768 iOB19**

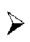 **Specificity**

The specificity of the combination was tested. The results are presented in **Fig. 15****.** They showed one peak-only, indicating that specificity QC criteria are met.

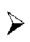 **Non-specificity**

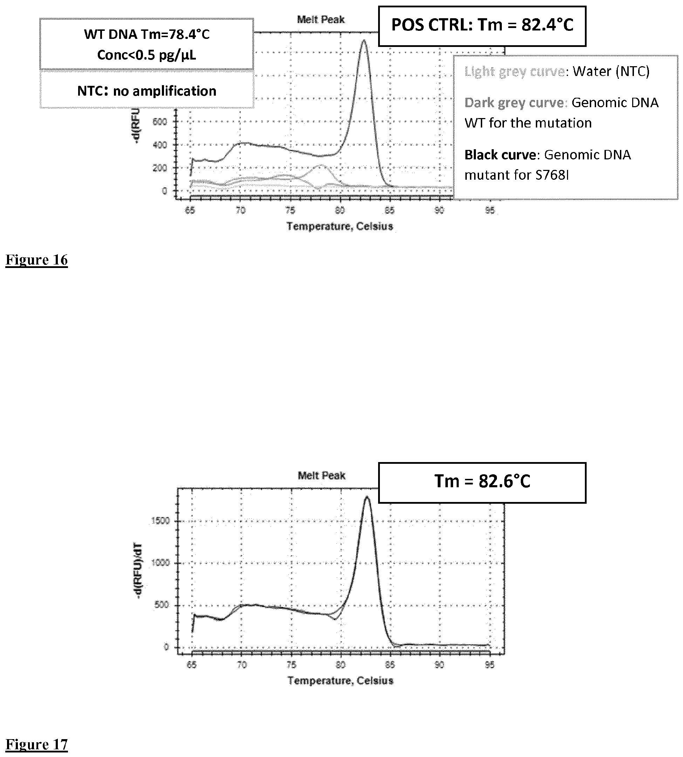

Melting curves of genomic DNA WT for the S768I mutation were compared to melting curves of commercial DNA mutant for S768I using this oligonucleotide combination (See
**Fig.16**). There was not any non-specificity observed, with no amplification observed in the NTC and in the WT DNA control. This oligonucleotide combination was thus evaluated for efficiency.

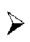 **Efficiency**

The efficiency analysis for the mutation-specific set comprised of S768 i17 + S768 iP18 +S768 iOB19 was performed in conjunction with the proposed combination for the S768 WT primer set. The results are presented in the following table.

**Table 35: Demonstration of the efficiency of the oligonucleotide system for the detection of EGFR S768I.**

| **Target** | **Content** | **Sample** | **Cq** | **[DNA] (ng/µl)** | **Tm (Y/N) specific?** | **Mean** | **mA%** |
|---|---|---|---|---|---|---|---|
| S768 i17 + S768 iP18 +S768 iOB19 | Neg control | Di-Fi DNA | N/A | N/A | | | |
| | | | 39.04 | N/A | N | | |
| | NTC | Water | N/A | N/A | | | |
| | | | N/A | N/A | | | |
| | Positive control | Horizon std L861Q 50% | 28.2 | 0.08 | Y | 0.085 | 28.33% |
| | | | 28.15 | 0.09 | Y | | |
| S768 WT L + L768 WT R) | | | 26.38 | 0.29 | Y | 0.300 | |
| | | | 36.31 | 0.31 | Y | | |

The observed mutant allele frequency of the S768I mutation-specific system on the S768 WT system is 28.33%, close to expected 50% reported by the supplier of the positive control used for the experiment.

The efficiency is thus appropriate and this oligonucleotide combination was further evaluated for sensitivity.

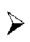 **Sensitivity**

The results are presented in the following table.

**Table 36: Demonstration of the high sensitivity of the method in analysis for the detection of the EGFR S768I point mutation. Serial dilutions of a commercial DNA control positive for the S768I mutation at expected mutational load of 0.08ng/µl were performed, up to 1/1000.**

| | Cq | [DNA] (ng/µL) extract | Tm specificity | mean | theorical |
|---|---|---|---|---|---|
| C+S768I | 28,2 | 0,08202 | Y | 0,0836 | 0,08 |
| | 28,15 | 0,08514 | Y | | |
| C+S768I1/10 | 31,75 | 0,00692 | Y | 0,0086 | 0,008 |
| | 31,19 | 0,01028 | Y | | |
| C+S768I1/100 | 34,8 | 0,00083 | Y | 0,0011 | 0,0008 |
| | 34,45 | 0,00106 | Y | | |
| | 33,56 | 0,00197 | Y | | |
| | 34,4 | 0,00110 | Y | | |
| | 34,24 | 0,00123 | Y | | |
| | 35,91 | 0,00038 | Y | | |
| C+S768I1/1000 | 37,65 | 0,00011 | N | 0,0002 | 0,00008 |
| | 39,12 | 0,00004 | N | | |
| | 35,61 | 0,00047 | N | | |
| | 36,86 | 0,00020 | Y | | |

The system can detect the S768I mutation down to a frequency of 0.04pg/µl. The sensitivity is thus very high.

### V7) Conclusions

Our method allows detection of the S768I mutation with high specificity, efficiency and sensitivity.

Among the tested primers, the optimal combination is comprised of the mutation-specific set **S768I i17 + S768 iP18 +S768 iOB19** and of the wild-type set **S768 WT L + S768 WT R.**
Other suitable primer combinations include:
- **S768I i16 + S768 iP18 +S768 iOB19**

### VI) G719S

The G719S mutation results in an amino acid substitution at position 719, from a glycine (G) to a serine (S). This mutation occurs within exon 18, which encodes part of the kinase domain. Nomenclature of *EGFR* G719S point mutation is c: 2155 G>A. It means that the 2155^{th} nucleotide on the cDNA reference sequence is a Guanine that is substituted by an Adenine.
The inventors have designed specific sets of primers allowing improved detection of the presence (or absence, or frequency) of such a mutation from a sample containing cfNA.

### VII) Design of the allele-specific EGFR G719S primer

Various allele-specific primers were considered in the design of this invention and their thermodynamic characteristics were analysed, as shown in Table 37:

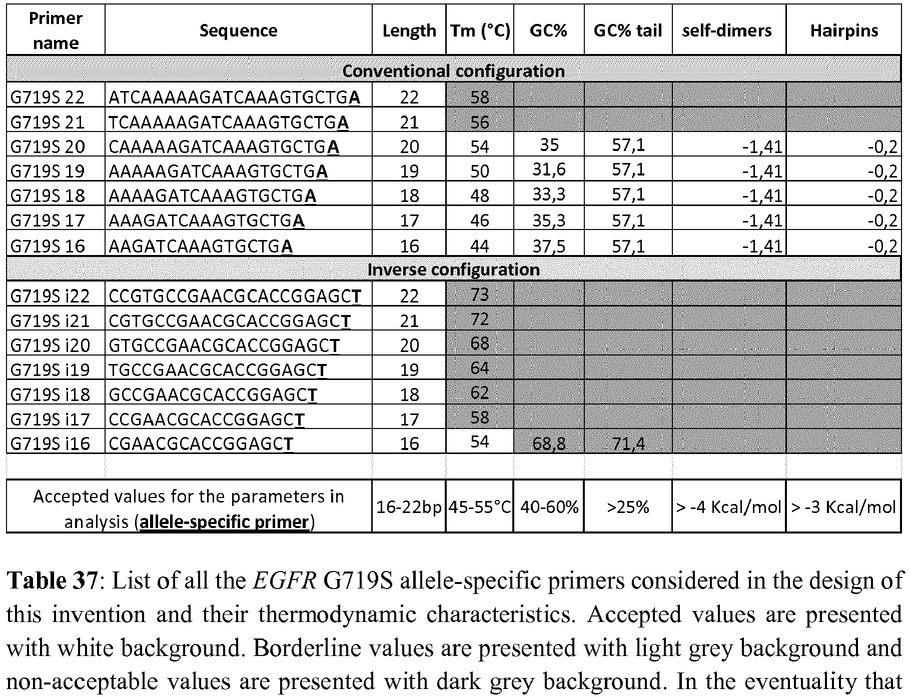

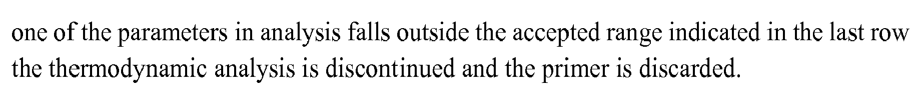

This preliminary analysis suggested that primer design in inverse configuration is thermodynamically less stable.

### VI2) Paired Wild-Type primer

### Results obtained with Primer 3

All allele-specific primers that met our criteria (see Table 37) were used on Primer3 to find a compatible paired primer. Results are summarised in Table 38.

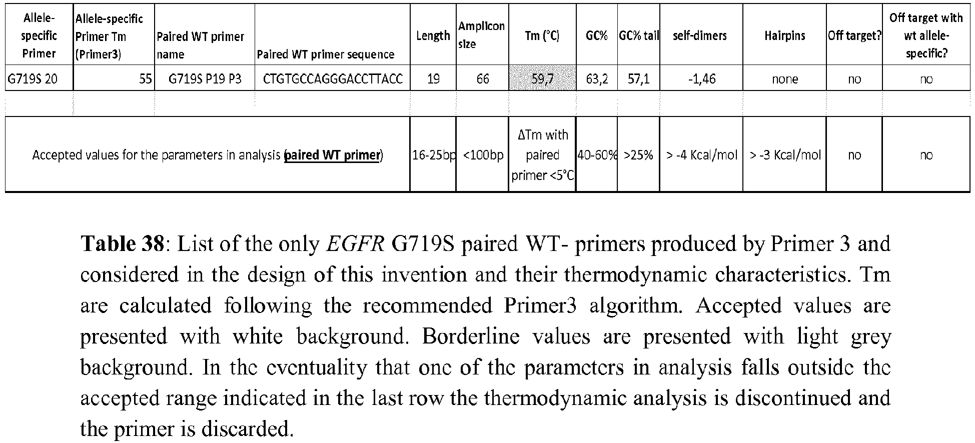

The only suitable paired primer obtained using the Primer3 software has a very borderline Tm difference with all mutation-specific primer candidates.A manual design of the paired WT primer was thus favoured to reduce the Tm difference and ensure optimal amplification efficiency.

### Manually generated primer pair candidates

In view of the unsuccessful generation of a paired primer using conventional tools, we designed such primers manually. All designed primers are listed in the following table. Their characteristics were analysed.

**Table 39: List of all potential EGFR G719S paired WT- primers in conventional configuration manually generated and considered in the design of this invention and their thermodynamic characteristics. Tm are calculated using the Oligoanalyzer® software. Accepted values are presented with white background**

| **Allele- specific Primer** | **Allele-specific Primer Tm** | **Paired WT primer name** | **Paired WT primer sequence** | **Length** | **Amplicon size** | **Tm (°C)** | **GC%** | **GC% tail** | **self-dimers** | **Hairpins** | **Off target?** | **Off target with wt allele- specific?** |
|---|---|---|---|---|---|---|---|---|---|---|---|---|
| G719S 20 | 54 | G719 P19 | CCAGGGACCTTACCTTATA | 19 | 61 | 56 | 47,4 | 28,6 | -1,46 | -1,34 | no | no |
| | | | | | | | | | | | | |
| Accepted values for the parameters in analysis (**pairedWT primer**) | | | | 16-25bp | <100bp | ΔTm with paired primer <5°C | 40-60% | >25% | > -4 Kcal/mol | > -3 Kcal/mol | no | no |

The following combinations were found, that met the thermodynamic criteria required:
- G719S 20 + G719X P19
- G719S 19 + G719X P19

### Blat alignment of the validated primer pairs in conventional sense

Blat alignments (Human GRCh38/hg38) for the PCR amplicon generated by the combinations of primers meeting the thermodynamic criteria were performed.
All primer pairs produced just one single amplicon at the expected chromosomal position. No other homologies are reported.

### VI3) Oligoblocker design for the EGFR G719 position

We generated several oligoblockers that target the wild-type sequence for G719 and tested their characteristics in combination with the primers.

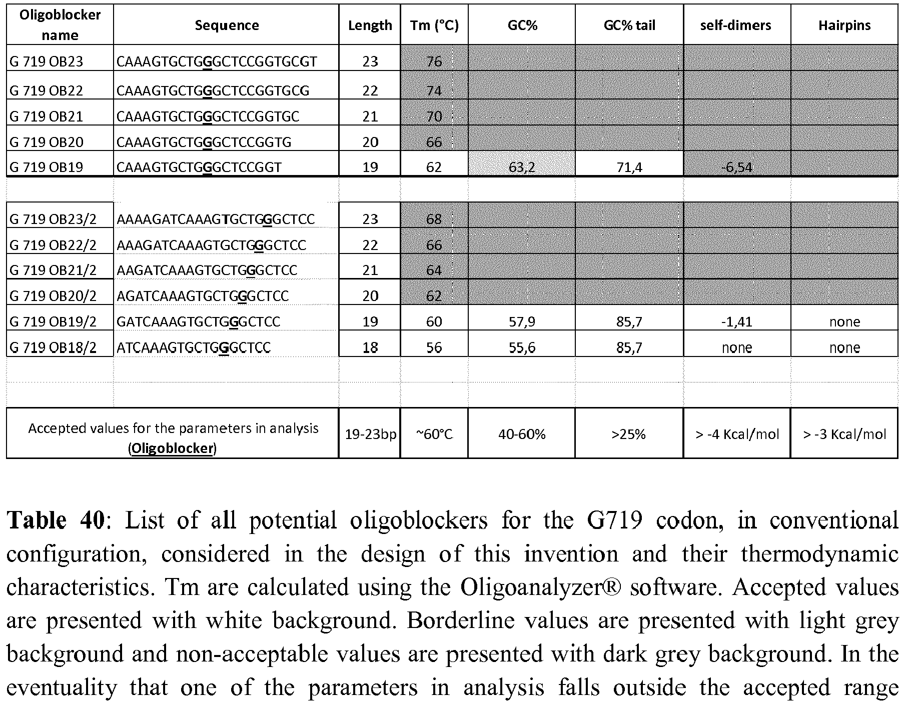

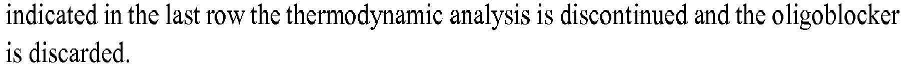

When the variant position is kept at the middle of the potential G719 oligoblocker, the self-dimerisation values are too high. By shifting the variant position towards the 3' end of the oligoblocker more favourable thermodynamic values were obtained.
The best oligoblockers were G719 BO 19/2 and G719 BO 18/2 that were thus considered for all further thermodynamic analyses.

### VI4) Thermodynamic analysis on all candidate primers and oligoblockers

The following heterodimers combinations were considered for all the primers/oligoblocker candidates that met the thermodynamic criteria described in the previous sections:
∘ Allele-specific primer + paired WT primer
∘ Allele-specific primer + oligoblocker
∘ Paired WT primer + oligoblocker

**Table 41: Evaluation of heterodimers presence for all oligonucleotide candidates evaluated for the allele-specific system.**

| **Oligonucleotide combination** | **ΔG (Kcal/mol)** | **Validated? Y/N** |
|---|---|---|
| G 719 OB19/2+G719X P19 | -3,41 | Y |
| G 719 OB19/2+G719S 20 | -1,41 | Y |
| G 719 OB19/2+G719S 19 | -1,41 | Y |
| G 719 OB18/2+G719X P19 | -3,41 | Y |
| G 719 OB18/2+G719S 20 | -0,31 | Y |
| G 719 OB18/2+G719S 19 | -0,31 | Y |
| G719S 19+G719X P19 | -0,34 | Y |
| G719S 20+G719X P19 | -0,34 | Y |
| | | |
| Accepted values for the parameters in analysis | >-4 Kcal/mol | Y |

The results show alloligonucleotide candidates meet the thermodynamic criteria required to ensure absence of non-specific amplification and to improve the amplification efficiency.

### VI5) Design of the EGFR G719S wild-type set

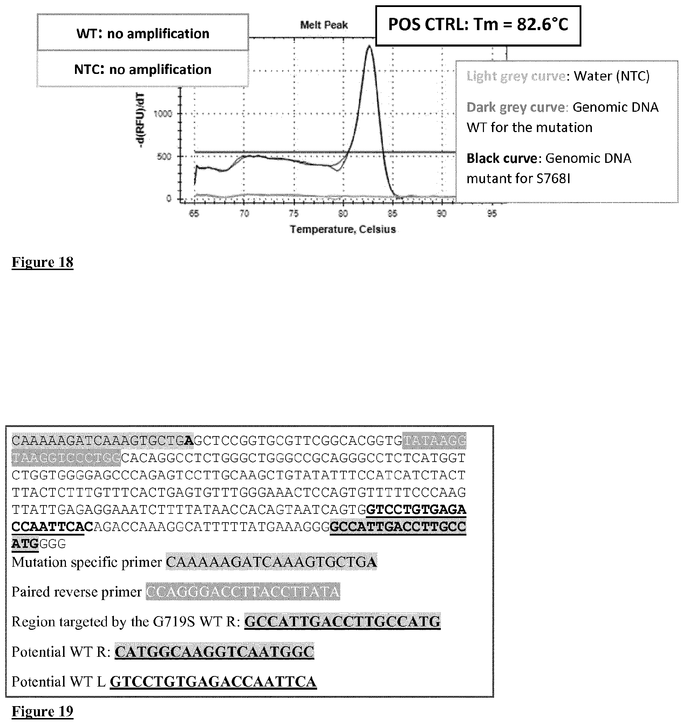

To produce optimal G719S Wild-Type primer sets we chose to target the DNA region shown in **Fig 19****.**
The tested primer candidates are disclosed in the following table.

**Table 42: Evaluation of all thermodynamic parameters considered to select appropriate oligonucleotides for the IntPlex® G719 wild-type set. Accepted values are presented with white background.**

| **Primer name** | **Sequence** | **Length** | **Tm (°C)** | **GC%** | **GC% tail** | **self-dimers** | **Hairpins** | **Heterodimers** | **Validated (Y/N)** |
|---|---|---|---|---|---|---|---|---|---|
| G719 WT L | GTCCTGTGAGACCAATTCA | 19 | 56 | 47,4 | 28,6 | -2,16 | -0,2 | -2,78 | Y |
| G719 WT R | CATGGCAAGGTCAATGGC | 18 | 56 | 55,6 | 57,1 | -2,17 | -0,1 | | Y |
| | | | | | | | | | |
| Accepted values for the parameters in analysis (**wild-type primer set**) | | 16-22bp | 55-60°C ΔTm with paired primer <5°C | 40-60% | >25% | > -4 Kcal/mol | > -3 Kcal/mol | > -4 Kcal/mol | Y |

Our results confirm that the candidate primers exhibit the required characteristics. The pair was thus validated for further analysis.

### Blat alignment of the validated primer pairs in conventional sense

Blat alignments (Human GRCh38/hg38) were performed for the PCR amplicons generated by the combinations of primers meeting the thermodynamic criteria.
The oligonucleotide candidates of the G719 WT set produced one single amplicon, thus meeting the thermodynamic criteria required to ensure absence of non-specific amplification and to improve the amplification efficiency.

**VI6) *In vitro* validation of the *EGFR* G719S primer pairs**

**G719S 20+G719 P19+ G719 OB19**

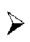 **Specificity**

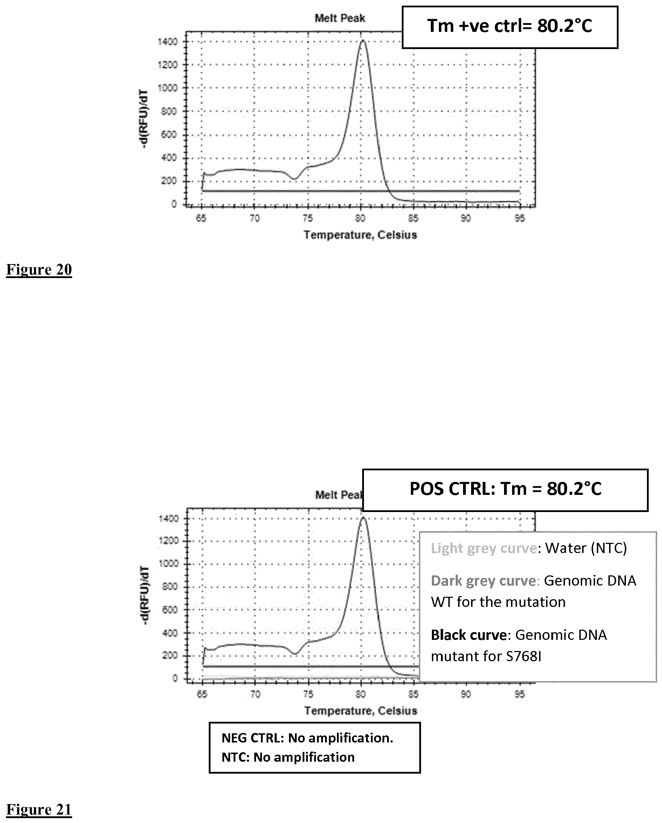

The specificity of the combination was tested. The results are presented in **Fig. 20****.** They showed one peak-only, indicating the specificity QC criteria are met.

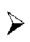 **Non-specificity**

Melting curves of genomic DNA WT for the G719S mutation were compared to melting curves of commercial DNA mutant for G719S using this oligonucleotide combination (See **Fig.21**). There is not any non-specificity observed. This oligonucleotide combination was thus evaluated for efficiency.

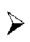 **Efficiency**

The efficiency analysis for the mutation-specific set comprised of G719S 20+G719 P19+ G719 OB 19 was performed in conjunction with the proposed combination for the G719 WT primer set. The results are presented in the following table.

**Table 43: Demonstration of the efficiency of the oligonucleotide system for the detection of EGFR S791S.**

| **Target** | **Content** | **Sample** | **Cq** | **[DNA] (ng/µl)** | **Tm specific? (Y/N)** | **Mean** | **mA%** |
|---|---|---|---|---|---|---|---|
| G719S 20+G719 P19 +G719 OB19 | Neg control | Di-Fi DNA | N/A | N/A | N | | |
| | | | N/A | N/A | N | | |
| | NTC | Water | N/A | N/A | N | | |
| | | | N/A | N/A | N | | |
| | Positive control | Horizon std L861Q 50% | 27.51 | 0.13305 | Y | 0.126 | 44.97% |
| | | | 27.68 | 0.11819 | Y | | |
| G719 WT Assay | | | 26.46 | 0.27645 | Y | 0.279 | |
| | | | 26.43 | 0.28229 | Y | | |

The target **G719 WT** corresponds to the combination G719 WL L + G719 WT R.

The observed mutant allele frequency of the G719S mutation-specific system on the G719 WT system is 44.97%, close to expected 50% reported by the supplier of the positive control used for the experiment. The efficiency was thus appropriate and this oligonucleotide combination was further evaluated for sensitivity.

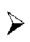 **Sensitivity**

The results are presented in the following table.

**Table 44: Demonstration of the high sensitivity of the method for the detection of the EGFR G719S point mutation. Serial dilutions of a commercial DNA control positive for the S719S mutation were performed, up to 1/10000.**

| Target | Sample | Cq | [DNA] (ng/µL) extract | Tm specificity | mean |
|---|---|---|---|---|---|
| | C+ | 27,51000 | 0,13305 | Y | 0,15960 |
| | | 27,68000 | 0,11819 | Y | |
| | C+ 1/10 | 30,38000 | 0,01803 | Y | 0,01618 |
| | | 30,71000 | 0,01432 | Y | |
| | C+ 1/100 | 34,76000 | 0,00085 | Y | 0,00057 |
| | | 36,11000 | 0,00033 | Y | |
| | | N/A | | | |
| | | 35,92000 | 0,00038 | Y | |
| | | N/A | | | |
| | | 34,98000 | 0,00073 | Y | |
| B2/G719S 20 | C+ 1/1000 | N/A | | | 0,00017 |
| | | N/A | | | |
| | | N/A | | | |
| | | N/A | | | |
| | | 37,06000 | 0,00017 | Y | |
| | | N/A | | | |
| | C+ 1/10000 | N/A | | | ND |
| | | N/A | | | |
| | | N/A | | | |
| | | N/A | | | |
| | | N/A | | | |
| | | N/A | | | |

The system can detect the G719S mutation down to a frequency of 0.1pg/µl. The sensitivity is thus very high.

### VI7) Conclusions

Our method has high specificity, efficiency and sensitivity.
The results here presented confirm that the best system to detect the *EGFR* G719S mutation is comprised of the mutation-specific set G719S 20 + G719 P19+ G719 OB19 and of the wild-type set G719 WT L + G719 WT R.
Other possible oligonucleotide systems include:
- G719S 19 + G719 P19 + G719 OB18
- G719S 19 + G719 P19 + G719 OB19
- G719S 20 + G719 P19 + G719 OB18

### VII) G719A

*EGFR* G719A is a substitution mutation. The G719A mutation results in an amino acid substitution at position 719, from a glycine (G) to an alanine (A). This mutation occurs within exon 18 which encodes part of the kinase domain. Nomenclature of the *EGFR* G719A point mutation is c: 2156 G>C. This means that the 2156^{th} nucleotide on the cDNA reference sequence is a Guanine that is substituted by a Cytosine.

The inventors have designed specific sets of primers allowing improved detection of the presence (or absence, or frequency) of such a mutation from a sample containing cfNA.

### VIII) Design of the allele-specific EGFR G719A primer

Various allele-specific primers were considered in the design of this invention and their thermodynamic characteristics were analysed, as shown in Table 45:

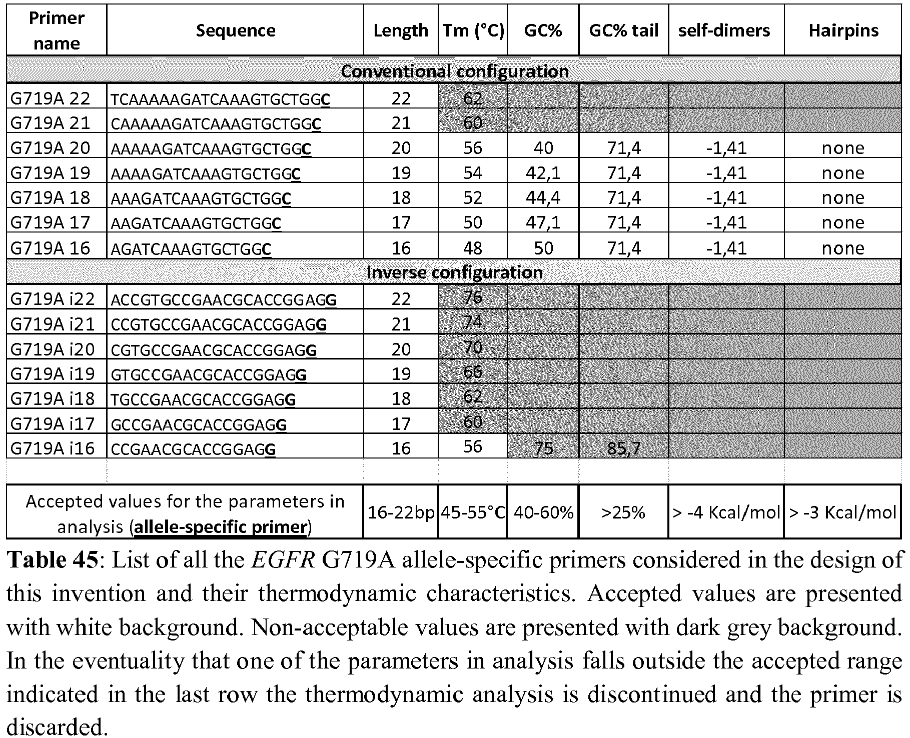

This preliminary analysis suggested that primer design in inverse configuration is thermodynamically less favourable.

### VII2) Paired Wild-Type primer

As the *EGFR* G719A assay is designed to target the same codon of the validated *EGFR* G719S assay (see chapter VI), it was extremely likely that the validated paired wild-type primer G719 P19 would work fine in combination with the proposed G719A mutation-specific primer candidates proposed in sectionVIII 1. A thermodynamic analysis was performed to exclude the presence of hetero-dimers with the mutation-specific primer candidates.

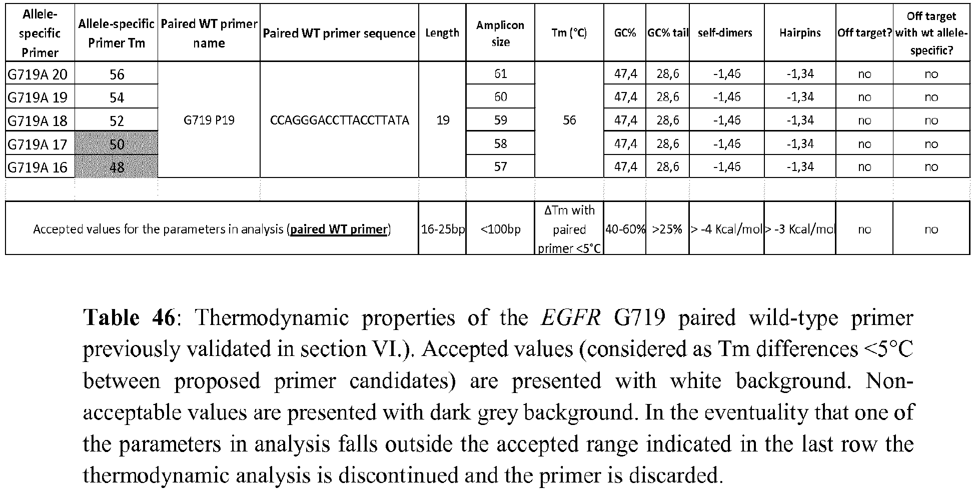

The following combinations were found, that met the thermodynamic criteria required:
- G719A 20 + G719 P19
- G719A 19 + G719 P19
- G719A 18 + G719 P19

### Blat alignment of the validated primer pairs in conventional sense

Blat alignments (Human GRCh38/hg38) for all the PCR amplicons generated by the combinations of primers meeting the thermodynamic criteria were performed.
All primer pairs produced just one single amplicon at the expected chromosomal position. No other homologies are reported.

### VII3) Oligoblocker design for the EGFR G719 position

As the *EGFR* G719A assay is designed to target the same codon of the validated *EGFR* G719A assay (see chapter VI), it was extremely likely that the validated oligoblocker G719 OB19/2 or the other thermodynamically validated oligoblocker candidate G719 OB 18/2 would work fine in combination with the primer candidates proposed in section VII.1/VII.2. A thermodynamic analysis was performed to exclude the presence of hetero-dimers with all primer candidates.

### VII4) Thermodynamic analysis on all candidate primers and oligoblockers

The following heterodimers combinations were considered for all the primers/oligoblocker candidates that met the thermodynamic criteria described in the previous sections:
∘ Allele-specific primer + paired WT primer
∘ Allele-specific primer + oligoblocker
∘ Paired WT primer + oligoblocker

The results are presented in the following table.

**Table 47: Evaluation of heterodimers presence for all oligonucleotide candidates evaluated for the allele-specific EGFR G719A system.**

| **Oligonucleotide combination** | **ΔG (Kcal/mol)** | **Validated? Y/N** |
|---|---|---|
| G719A 18+G719 P19 | -3,41 | Y |
| G719A 18+G719 OB18/2 | 0,16 | Y |
| G719A 18+G719 OB19/2 | -1,41 | Y |
| G719A 19+G719 P19 | -3,41 | Y |
| G719A 19+G719 OB19/2 | -1,41 | Y |
| G719A 19+G719 OB18/2 | 0,16 | Y |
| G719A 20+G719 P19 | -3,41 | Y |
| G719A 20+G719 OB19/2 | -1,41 | Y |
| G719A 20+G719 OB18/2 | 0,16 | Y |
| | | |
| Accepted values for the parameters in analysis | >-4 Kcal/mol | Y |

The results show all oligonucleotide candidates meet the thermodynamic criteria required to ensure absence of non-specific amplification and to improve the amplification efficiency.

### VII5) Design of the EGFR G719A wild-type set

As the *EGFR* G719A assay is designed to target the same codon of the validated *EGFR* G719A assay (see chapter VI), the validated wild-type *EGFR* G719 Wild-type assay had to respect all constraints in terms of distance from the mutation-specific primer set to be validated. The WT primer combination G719 WT L + G719 WT R was thus used for the *in-vitro* validation of the *EGFR* G719A IntPlex® assay.

**VII6) *In vitro* validation of the *EGFR* G719A primer pairs**

**G719A 18 + G719 P19 + G719 OB19/2**

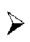 **Specificity**

The specificity of the combination was tested. The results showed one peak-only, indicating the specificity criteria are met.

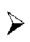 **Non-specificity**

A substantial non-specificity was observed when using this oligonucleotide combination. The QC criteria for non-specificity are not met. This combination is thus not optimal.

**G719A 18 + G719 P19 + G719 OB18/2**

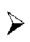 **Specificity**

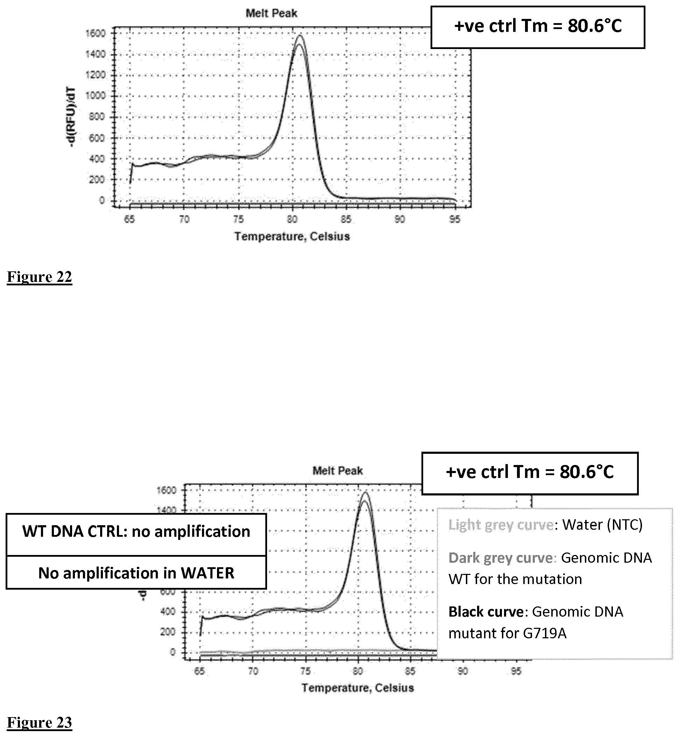

The specificity of this combination was tested. The results are presented in **Fig 22****.** They showed one peak-only, indicating the specificity criteria are met.

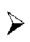 **Non-specificity**

Melting curves of genomic DNA WT for the G719A mutation were compared to melting curves of commercial DNA mutant for G719A using this oligonucleotide combination (See **Fig.23**). There was not any non-specificity observed. This oligonucleotide combination was thus evaluated for efficiency.

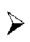 **Efficiency**

The efficiency analysis for the mutation-specific set comprised of G719A 18 + G719 P19 + G719 OB18/2 was performed in conjunction with the proposed combination for the G719 WT primer set. The results are presented in the following table.

**Table 48: Demonstration of the efficiency of the oligonucleotide system for the detection of EGFR G719A.**

| **Target** | **Content** | **Sample** | **Cq** | **[DNA] (ng/µl)** | **Tm specific? (Y/N)** | **Mean** | **mA%** |
|---|---|---|---|---|---|---|---|
| G719 18+G719 P19 +G719 OB18/2 | Neg control | G719S positive control | N/A | N/A | N | | |
| | | | N/A | N/A | N | | |
| | NTC | Water | N/A | N/A | N | | |
| | | | N/A | N/A | N | | |
| | Positive control | G719A 50% positive control | 27.62 | 0.18 | Y | 0.185 | 60.66% |
| | | | 27.72 | 0.19 | Y | | |
| G719 WT Assay | | | 27.28 | 0.26 | Y | 0.305 | |
| | | | 27.1 | 0.35 | Y | | |

The observed mutant allele frequency of the G719A mutation-specific system on the G719 WT system is 60.66%, close to expected 50% reported by the supplier of the positive control used for the experiment. The efficiency is thus appropriate and this oligonucleotide combination was further evaluated for sensitivity.

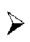 **Sensitivity**

The results are presented in the following table.

**Table 49: Demonstration of the high sensitivity of IntPlex® system in analysis for the detection of the EGFR G719A point mutation. Serial dilutions of a commercial DNA control positive for the G719A mutation at expected mutational load of 0.25ng/µl were performed, up to 1/1000.**

| Sample | Cq | [DNA] (ng/µl) extract | Tm specificity | MEAN | theorical level |
|---|---|---|---|---|---|
| C+G719A0.25 | 27,62 | 0,20450 | Y | 0,19750 | 0,25 |
| | 27,72 | 0,19050 | Y | | |
| C+G719A1/10 | 31,14 | 0,01629 | Y | 0,01587 | 0,025 |
| | 31,21 | 0,01544 | Y | | |
| C+G719A1/1000 | 35,05 | 0,00097 | Y | 0,00073 | 0,00025 |
| | 35,92 | 0,00052 | Y | | |
| | 35,53 | 0,00069 | Y | | |
| | N/A | N/A | | | |
| | N/A | N/A | | | |
| | N/A | N/A | | | |

The system can detect the G719A mutation down to a frequency of 0.5pg/µl. The sensitivity is thus very high.

### VII7) Conclusions

Our method presents high specificity, efficiency and sensitivity. Among the tested primer combinations, the best system to detect the EGFR G719A mutation is comprised of the mutation-specific set G719A 18 + G719 P19 + G719 OB18/2 and of the wild-type set G719 WT L + G719 WT R.

Other oligonucleotide combinations that allow efficient detection include:
- G719A 19 + G719 P19 + G719 OB18/2
- G719A 19 + G719 P19 + G719 OB19/2
- G719A 20 + G719 P19 + G719 OB18/2
- G719A 20 + G719 P19 + G719 OB19/2

### VIII) DEL19

EGFR exon 19 deletions are in-frame deletions occurring within exon 19, which encodes part of the kinase domain. The most frequent deletion in *EGFR* exon 19 is Δ(E746-A750) resulting from a 15 nucleotide deletion event. The nucleotidic nomenclature of *EGFR* E746-A750 deletion is c:2235-2249: The 15 nucleotides from 2235 to 2249 on the coding DNA reference sequence are deleted. The inventors have designed specific sets of primers allowing improved detection of the presence (or absence, or frequency) of such a mutation from a sample containing cfNA.

### VIII1) Strategy for detecting deletion mutations

Strategy for the qualitative detection of the deletions is described in **Fig. 24****.**

As previously described, the addition of an oligoblocker allows avoiding non-specific amplification of WT DNA sequences. The strategy for optimal positioning of the oligoblocker is described in **Fig 25****.**

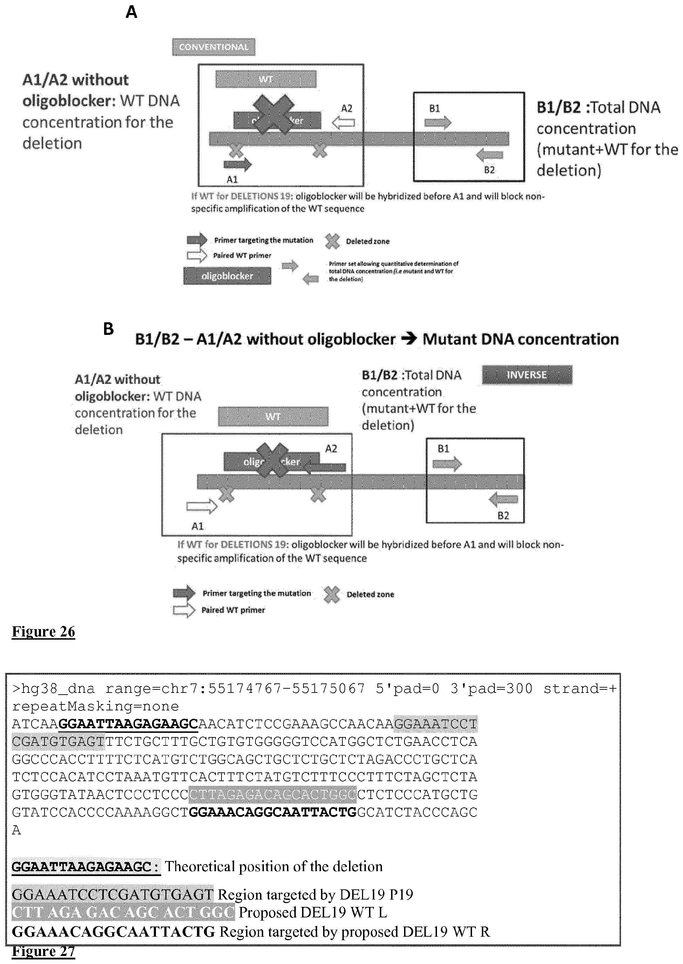

The system presented here allows highly specific and sensitive qualitative detection of the deletion. For quantitative detection, the total DNA concentration can be determined using a B1/B2 WT primer set and by measuring the difference between the total DNA concentration determined with B1/B2 primer set to the WT DNA concentration determined using A1/A2 primer set (**Fig 26**).

### VIII2) Design of the allele-specific EGFR DEL19 primer

The allele-specific primers generated and considered in the design of this invention and their thermodynamic characteristics are listed in Table 50:

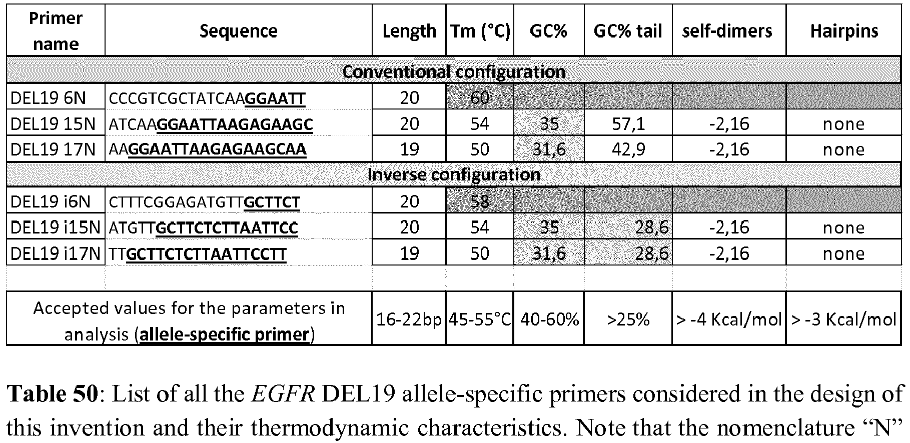

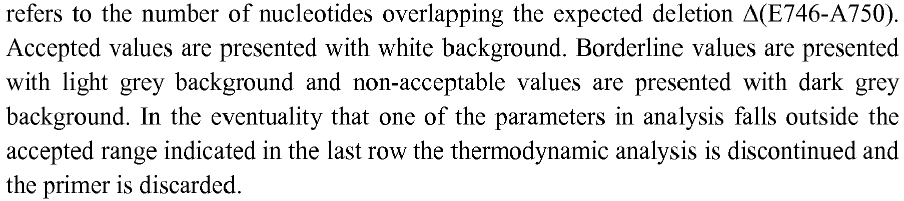

### VIII3) Paired Wild-Type primer

### Results obtained with Primer 3

All allele-specific primers that met our criteria (see Table 50) were used on Primer3 to try to find a compatible paired primer. No valid output results were generated applying the suggested parameters.

### Manually generated primer pair candidates

In view of the unsuccessful generation of a paired primer using conventional tools, we designed such primers manually. All designed primers are listed below, with their characteristics.

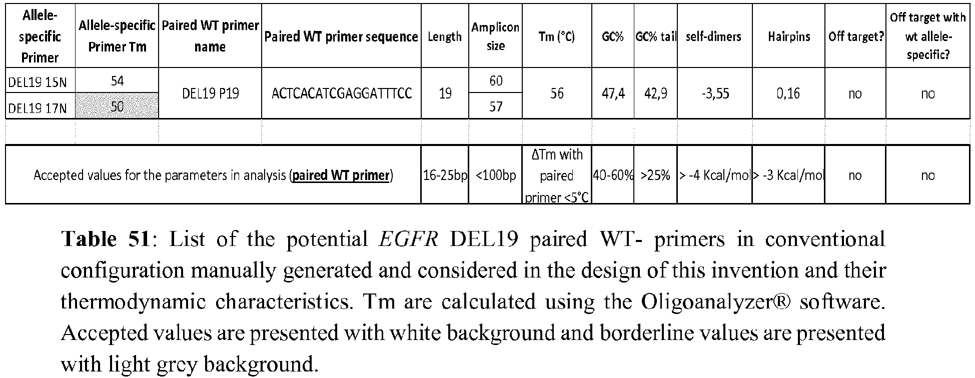

The results show DEL19 P19 represents an optimal candidate for the assay in conventional configuration.

### Blat alignment of the validated primer pairs in conventional sense

Blat alignments (Human GRCh38/hg38) were performed. They showed the selected optimal primer pairs produced just one single amplicon at the expected chromosomal position.

### VIII4) Oligoblocker design for the EGFR DEL19 position

We generated several oligoblockers and verified their characteristics, as shown in the following table.

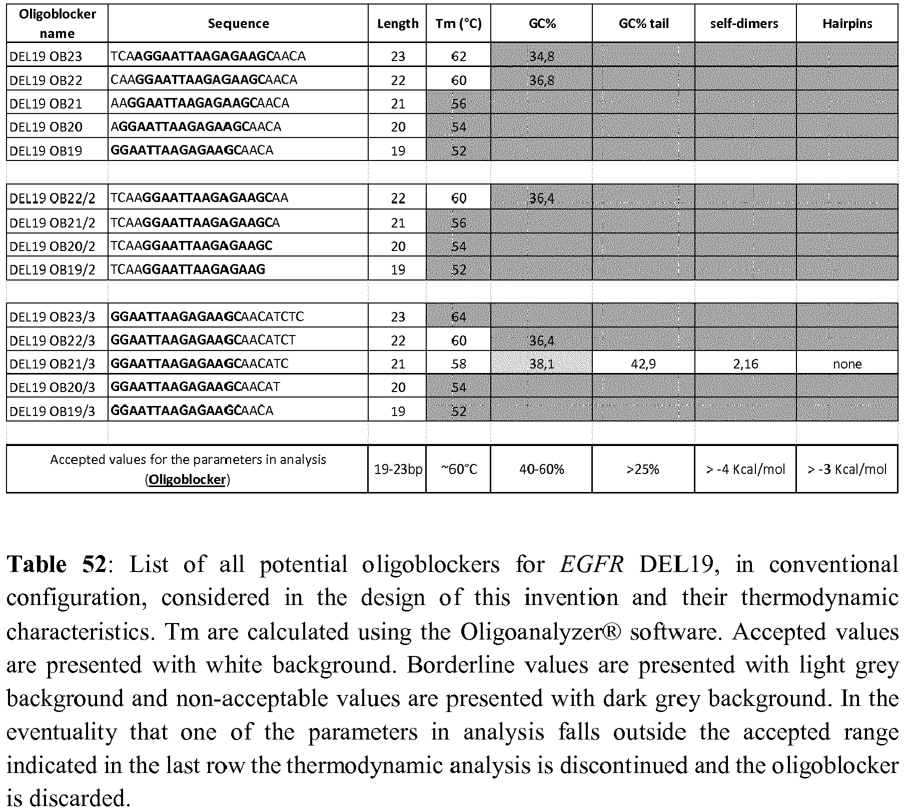

The best oligoblocker appeared to be DEL 19 OB21/3 and it was thus selected for all further thermodynamic analyses.

### VIII5) Thermodynamic analysis on all candidate primers and oligoblockers

The following heterodimers combinations were considered for all the primers/oligoblocker candidates that met the thermodynamic criteria:
∘ Allele-specific primer + paired WT primer
∘ Allele-specific primer + oligoblocker
∘ Paired WT primer + oligoblocker

The results are presented in the following table:

**Table 53: Evaluation of heterodimers presence for all oligonucleotide candidates evaluated for the allele-specific system.**

| **Oligonucleotide combination** | **ΔG (Kcal/mol)** | **Validated? Y/N** |
|---|---|---|
| DEL19 15N+DEL19 P19 | -3,38 | Y |
| DEL19 15N+DEL19 OB21/3 | -2,16 | Y |
| DEL19 17N+DEL19 P19 | -3,38 | Y |
| DEL19 17N+DEL19 OB21/3 | -2,16 | Y |
| DEL19 P19+DEL19 OB21/3 | -3,38 | Y |
| | | |
| Accepted values for the parameters in analysis | >-4 Kcal/mol | Y |

The results show all oligonucleotide candidates meet the thermodynamic criteria required to ensure absence of non-specific amplification and to improve the amplification efficiency.

### VIII6) Design of the DEL19 wild-type primer set

The optimal positioning of the DEL19 wild-type primer candidates on the genomic nucleic acid sequence is shown in **Fig 27****.**

The tested primers are disclosed in the following table.

**Table 54: Evaluation of all thermodynamic parameters considered to select appropriate oligonucleotides for the EGFR DEL19 wild-type set. Accepted values are presented with white background.**

| **Primer name** | **Sequence** | **Length** | **Tm (°C)** | **GC%** | **GC% tail** | **self-dimers** | **Hairpins** | **Heterodimers** | **Validated (Y/N)** |
|---|---|---|---|---|---|---|---|---|---|
| DEL19 WT L | CTTAGAGACAGCACTGGC | 18 | 56 | 55,6 | 71,4 | -0,34 | none | -3 | Y |
| DEL19 WT R | CAGTAATTGCCTGTTTCC | 18 | 52 | 44,4 | 42,9 | -2,16 | -0,22 | | Y |
| | | | | | | | | | |
| Accepted values for the parameters in analysis **(wild-type primer set)** | | 16-22bp | 55-60°C ΔTm with paired primer <5°C | 40-60% | >25% | >-4 Kcal/mol | >-3 Kcal/mol | >-4 Kcal/mol | Y |

The primers exhibited the required characteristics.

### Blat alignment of the validated primer pairs in conventional sense

Blat alignments (Human GRCh38/hg38) were performed for the PCR amplicons generated by the combinations of primers.

The Blat local alignment highlighted the presence of a secondary homology region. Nevertheless, by analysing the structure and extension of the homology region reported on chr3, we concluded that it would produce just a partial annealing region for the DEL19 WT L primer but, as no homology is present in the target region of the DEL19 WT R primer, no secondary product would be produced.

The oligonucleotide candidates of the DEL19 WT set thus met the thermodynamic criteria required to ensure absence of non-specific amplification and to improve the amplification efficiency.

**VIII7) In vitro validation of the EGFR DEL19 primers**

**DEL19 15N + DEL19 P19 + DEL19 OB21/3**

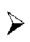 **Specificity and non-specificity**

A substantial non-specificity was observed when using this oligonucleotide combination.

**DEL19 17N** + **DEL19 P19** + **DEL19 OB21/3**

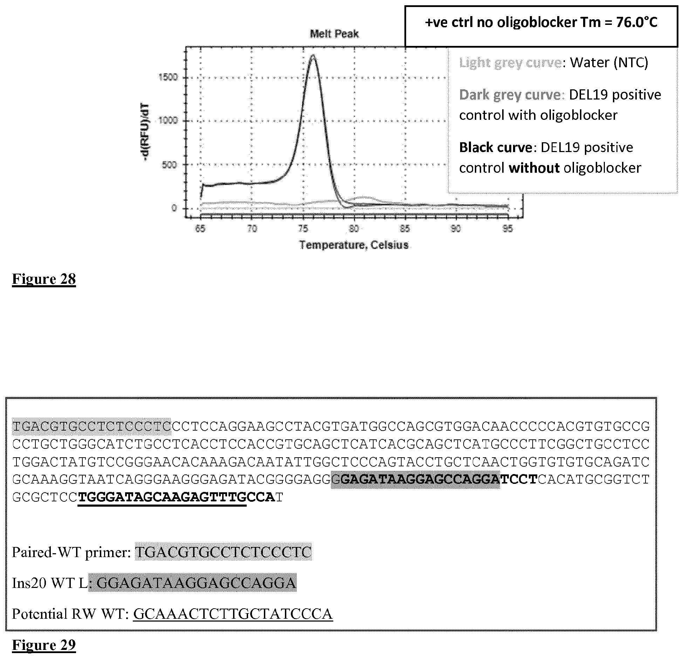

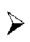 **Specificity and non-specificity** (See **Fig 28**)

This oligonucleotide combination is highly specific.

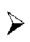 **Efficiency**

The efficiency analysis for the mutation-specific set comprised of DEL19 17N + DEL19 P19 + DEL19 OB21/3 was performed in conjunction with the proposed system for the DEL19 WT primer set.

**Table 55: Demonstration of the efficiency of the oligonucleotide system for the detection of EGFR DEL19 Δ(E746-A750).**

| **Target** | **Content** | **Sample** | **Cq** | **[DNA] (ng/µl)** | **Mean** | **mA%** |
|---|---|---|---|---|---|---|
| DEL19 17N+DEL19 P19+DEL19 OB21/3 | Neg control | T790M 50% +ve ctrl | N/A | N/A | | |
| | | | | N/A | | |
| | NTC | Water | N/A | N/A | | |
| | | | 3765 | 0,00008 | | |
| | Positive control | DEL19 50% +ve ctrl | 37,17 | 0,00012 | 0,00012 | |
| | | | 37,15 | 0,00012 | | |
| WT Assay | | | 25,16 | 1,152 | 1,142 | |
| | | | 25,18 | 1,131 | | |
| DEL19 17N+DEL19 P19+DEL19 | Positive control | DEL19 50% +ve ctrl | 25,82 | 0,6894 | 0,67385 | 47% |
| | | | 25,88 | 0,6583 | | |

The observed mutant allele frequency of the *EGFR* DEL19-specific system on the DEL 19 WT system is 47%, close to expected 50% reported by the supplier of the positive control used for the experiment. The method thus provides high efficiency.

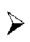 **Sensitivity**

**Table 56: Demonstration of the high sensitivity of the method for the detection of the EGFR Δ(E746-A750). Serial dilutions of a commercial DNA control positive for the EGFR Δ(E746-A750) were performed, up to 1/10000.**

| Target | Sample | Cq | [DNA] (ng/µl) extrait | mean |
|---|---|---|---|---|
| DEL 19 A1/A2-Blocker | C+ DEL19 | 27,63 | 0,1583 | 0,1594 |
| | | 27,61 | 0,1604 | |
| | C+ DEL19 1/10 | 30,86 | 0,0142 | 0,0145 |
| | | 30,8 | 0,0149 | |
| | C+ DEL19 1/100 | 33,72 | 0,0017 | 0,0017 |
| | C+ DEL19 1/1000 | 37,66 | 0,0001 | 0,0001 |
| | | N/A | N/A | |
| | C+ DEL19 1/10000 | N/A | N/A | N/A |
| | | N/A | N/A | |

The method can detect the *EGFR* deletion Δ(E746-A750)down to a frequency of 0.1pg/µl. The sensitivity is thus very high.

### VIII8) Conclusions

The results here presented confirm that the method allows detection of DEL 19 with high specificity, efficiency and sensitivity. Optimal primers include the mutation-specific set DEL19 17N + DEL19 P19 with and without oligoblocker DEL19 OB21/3 and of the wild-type set DEL19 WT L + DEL19 WT R.

### IX) EGFR Exon 20 insertions (Ins20) V769-D770 insASV

*EGFR* exon 20 insertions are in-frame insertions occurring within exon 20. Most *EGFR* exon 20 insertions occur in between amino acids 767 to 774 of exon 20, within the loop that follows the C-helix of the kinase domain of *EGFR* (Yasuda, Kobayashi, and Costa 2012). In terms of coding DNA reference sequence refers to c: 2302-2320. Insertions can vary from 3 to 12 nucleotides, with the most common being *EGFR* V769-D770 insASV (c.2308 -2309insGCCAGCGTG).

The inventors have designed specific sets of primers allowing improved detection of the presence (or absence, or frequency) of such a mutation from a sample containing cfNA.

### IX1) Design of the allele-specific EGFR Ins20 primer

The allele-specific primers generated and considered in the design of this invention and their thermodynamic characteristics are listed in Table 57.

The design in conventional configuration was strongly unfavoured: the presence of a region of self-dimerisation impaired any potential primer design in conventional sense spanning the insertion junction from the gene 5'. Only allele-specific primer candidates in inverse configuration were thus considered for all thermodynamic analyses.

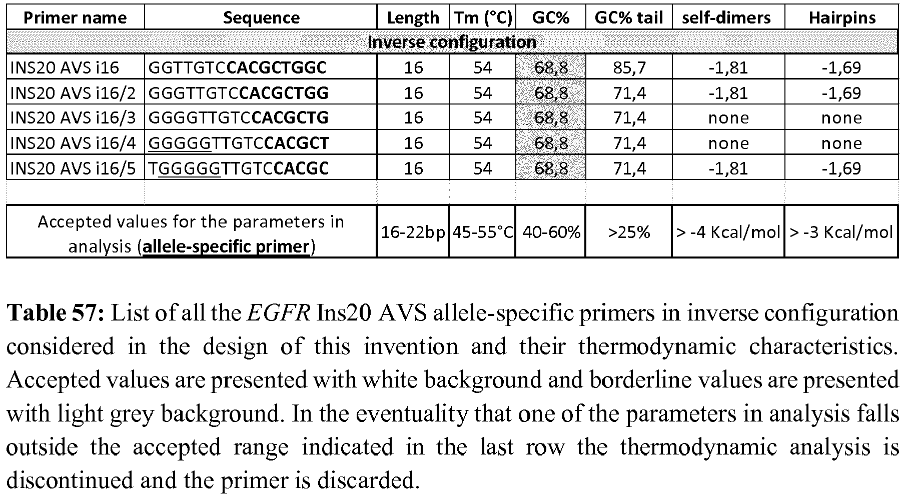

Because of the very high GC% of the area, longer allele-specific primers were not considered as the Tm would have been too close to 60°C (Optimal annealing temperature for the oligoblocker).
All primer pairs do have the same high GC% but there were no alternative options. According to Oligoanalyzer all primers possessed similar thermodynamic characteristics. Nevertheless, as general precaution when designing primers, it is recommended to avoid G quadruplexes. This rule was applied when analysing allele-specific primer with similar thermodynamic characteristics.

### IX2) Paired Wild-Type primer

### Results obtained with Primer 3

All allele-specific primers that met our Icriteria (see Table 57) were used on Primer3 to try to find a compatible paired primer. Results are summarised in Table 58.

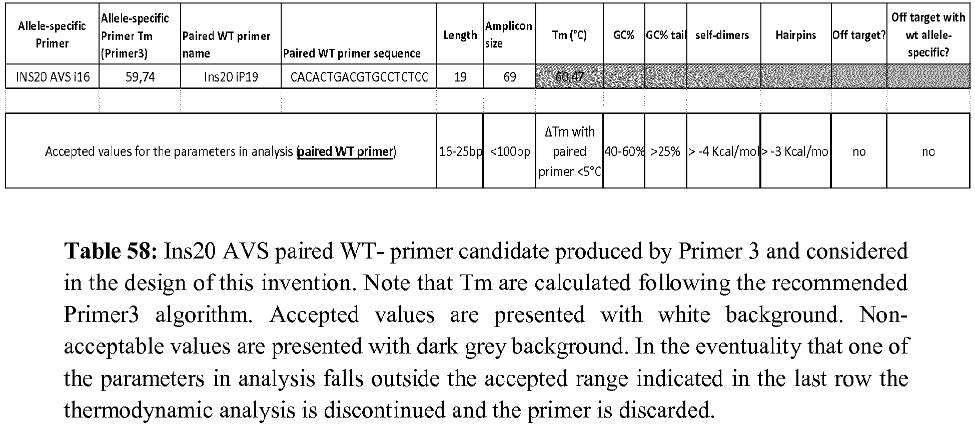

From the results presented in Table 58 it was not possible to select a suitable candidate as the only output presented too high melting temperature.
By manually reducing the size of the primer candidate proposed by Primer3 a better fit could be reached. The paired-wild type primer was manually designed.

### Manually generated primer pair candidates

In view of the unsuccessful generation of a paired primer using conventional tools, we designed such primers manually. All designed primers are listed below, with their characteristics.

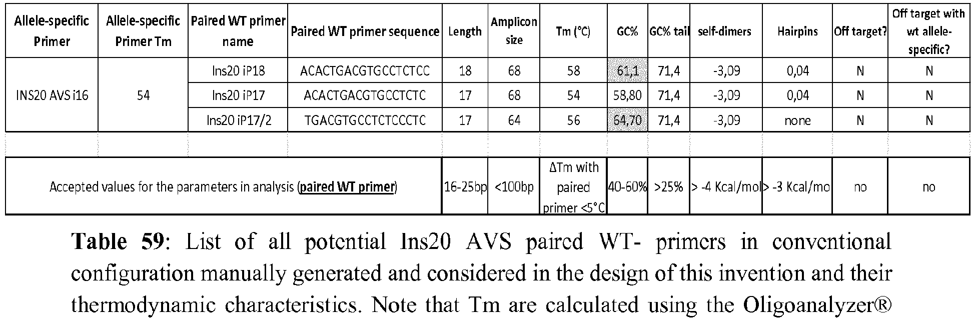

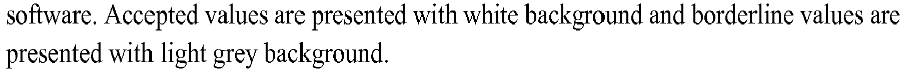

All WT primer candidates represented suitable choices for the assay in inverse configuration. The GC% was quite high but as it is region-dependent it could not be lowered further.

### Blat alignment of the validated primer pairs in inverse configuration

Blat alignments (Human GRCh38/hg38) for all the PCR amplicons generated all combinations of primers were performed. The primer combinations provided one principal hit on the EGFR gene. The percentage of identity is <100% as the presence of the insertion would add 9 nucleotides not present in the reference sequence. All secondary hits did not span any primer landing site.

All primer pair combinations produced just one single amplicon at the expected chromosomal position.

### IX3) Oligoblocker design for the allele-specific EGFR Ins20 AVS position

We generated several oligoblockers and verified their characteristics, as shown in the following table.

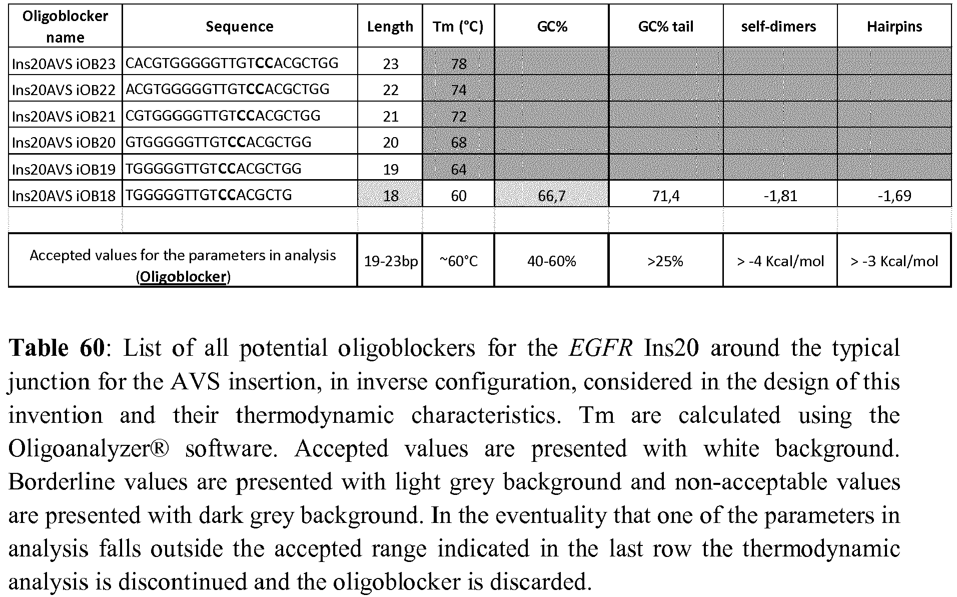

The best oligoblocker appeared to be Ins20AVS iOB 18 that was thus selected for all further thermodynamic analyses.

### IX4) Thermodynamic analysis on all candidate primers and oligoblockers

The following heterodimers combinations were considered for all the primers/oligoblocker candidates that met the thermodynamic criteria:
∘ Allele-specific primer + paired WT primer
∘ Allele-specific primer + oligoblocker
∘ Paired WT primer + oligoblocker

The results are presented in the following table:

**Table 61: Evaluation of heterodimers presence for all oligonucleotide candidates evaluated for the allele-specific system.**

| **Oligonucleotide combination** | **ΔG (Kcal/mol)** | **Validated? Y/N** |
|---|---|---|
| INS20 AVS i16+lns20 iP17 | -3,7 | Y |
| INS20 AVS i16+lns20 iP17/2 | -3,7 | Y |
| INS20 AVS i16+lns20 iP18 | -3,7 | Y |
| INS20 AVS i16+lns20AVS iOB18 | -1,81 | Y |
| Ins20 iP17+lns20AVS iOB18 | -3,7 | Y |
| Ins20 iP17/2+lns20AVS iOB18 | -3,7 | Y |
| Ins20 iP18+lns20AVS iOB18 | -3,7 | Y |
| | | |
| Accepted values for the parameters in analysis | >-4 Kcal/mol | Y |

The results show all oligonucleotide candidates meet the thermodynamic criteria required to ensure absence of non-specific amplification and to improve the amplification efficiency.

### IX5) Design of the Ins20 wild-type primer set

The optimal positioning of Ins20 wild-type primer candidates on the genomic nucleic acid sequence is presented in **Fig 29****.** The tested primers are disclosed in the following table.

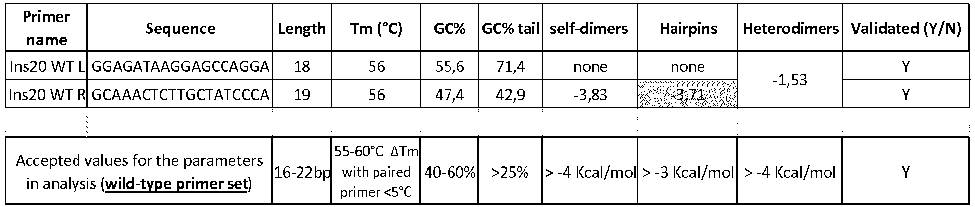

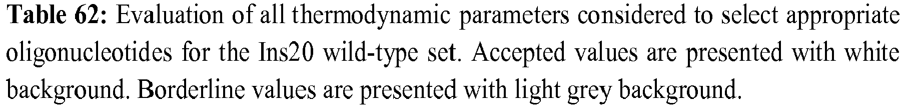

The primers exhibit the required characteristics.

### Blat alignment of the validated primer pairs in conventional sense

Blat alignments (Human GRCh38/hg38) were performed for the PCR amplicons generated by all combinations of primers. All primer pairs produced one single amplicon only at the expected chromosomal position. No other homologies were reported.
The oligonucleotide candidates of the *EGFR* Ins20 WT set met the thermodynamic criteria required to ensure absence of non-specific amplification and to improve the amplification efficiency.

### IX6) Conclusions

The results here presented confirmed that the method allows detection of *EGFR* Ins20 AVS insertion with high specificity, efficiency and sensitivity. Optimal primers include the following combinations:
INS20 AVS i16 + Ins20 iP17 + +Ins20AVS iOB18
INS20 AVS i16 + Ins20 iP 17/2 + +Ins20AVS iOB18
INS20 AVS i16 + Ins20 iP18 + +Ins20AVS iOB18

Other suitable combinations include:
INS20 AVS i16/2 + Ins20 iP17 + +Ins20AVS iOB18
INS20 AVS i16/2 + Ins20 iP17/2 + +Ins20AVS iOB18
INS20 AVS i16/2 + Ins20 iP18 + +Ins20AVS iOB18
INS20 AVS i16/3 + Ins20 iP17 + +Ins20AVS iOB18
INS20 AVS i16/3 + Ins20 iP 17/2 + +Ins20AVS iOB 18
INS20 AVS i16/3 + Ins20 iP18 + +Ins20AVS iOB18
INS20 AVS i16/4 + Ins20 iP17 + +Ins20AVS iOB18
INS20 AVS i16/4 + Ins20 iP17/2 + +Ins20AVS iOB 18
INS20 AVS i16/4 + Ins20 iP18 + +Ins20AVS iOB18
INS20 AVS i16/5 + Ins20 iP17 + +Ins20AVS iOB18
INS20 AVS i16/5 + Ins20 iP17/2 + +Ins20AVS iOB18
INS20 AVS i16/5 + Ins20 iP18 + +Ins20AVS iOB18

The following combination of primers can be used as paired wild-type assay for the *EGFR* Ins20 AVS insertion: Ins20 WT L + Ins20 WT R.

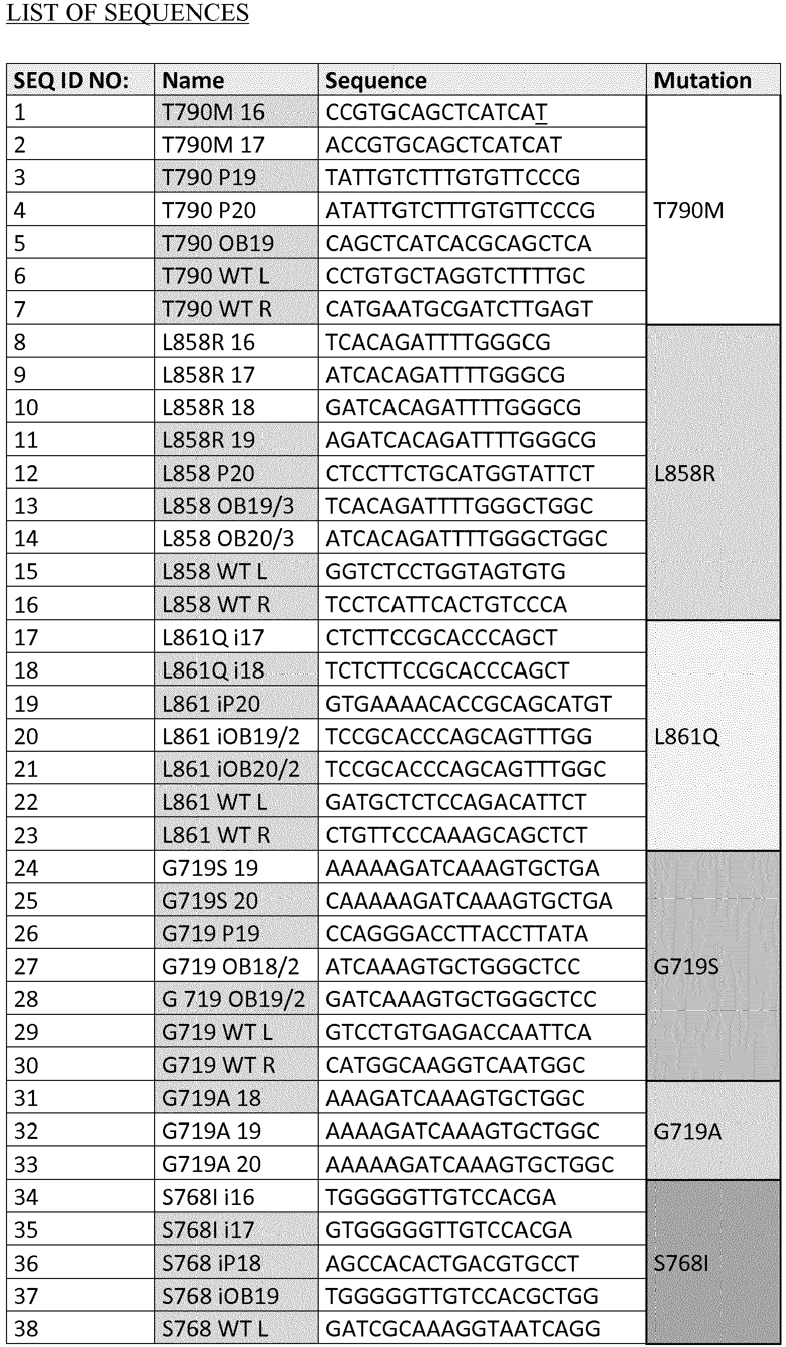

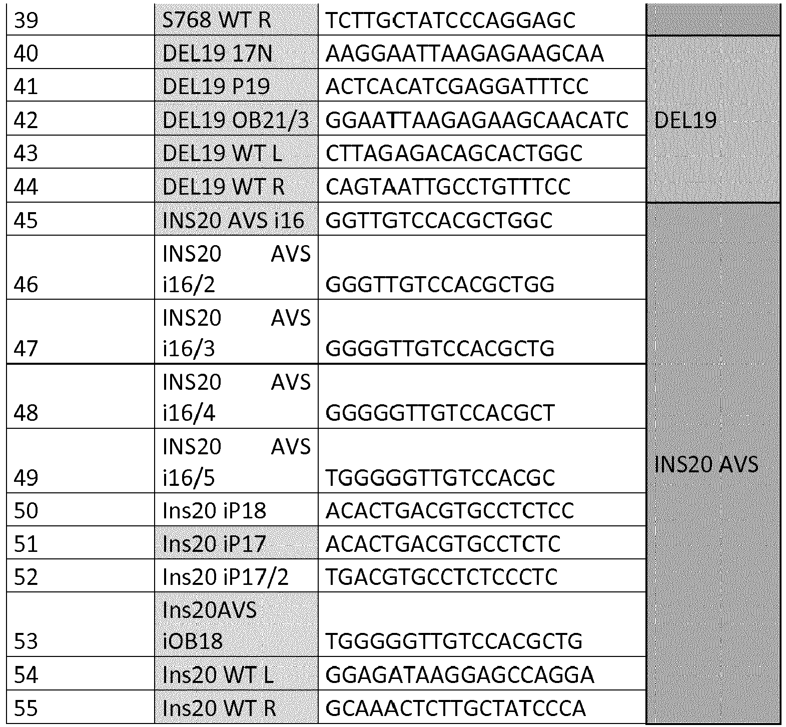

## Claims

1. A method for determining the presence of a mutation in the EGFR gene in a sample from a subject, the method comprising amplifying, in a sample of a biological fluid from the subject containing cell free nucleic acids, at least a first target sequence from the EGFR gene, the first target sequence containing a site of a mutation on said gene, wherein the first amplified target sequence is 50-95nt in length and amplification of the first target sequence is conducted with a first set of reagents comprising (i) a mutation-specific primer of 15-25nt in length, (ii) a paired primer of 15-25nt in length and (iii) an oligoblocker, wherein the oligoblocker hybridizes specifically to the first target sequence in non-mutated form and blocks polymerase elongation.

2. The method of claim 1, wherein the mutation is a nucleotide substitution and wherein the mutation-specific primer contains the mutated position at one terminal nucleotide.

3. The method of claim 1, wherein the mutation is a deletion and wherein the mutation-specific primer overlaps with the junction region in the gene created by said deletion.

4. The method of claim 1, wherein the mutation is an insertion and wherein the mutation-specific primer overlaps with the junction region in the gene created by said insertion.

5. The method of anyone of claims 1 to 4, wherein the mutation is selected from G719A, G719C, G719S, S768I, T790M, L858R, L861Q, exon19DELΔ(E746-A750) and exon20INS.

6. The method of claim 5, wherein the mutation is EGFR T790M and wherein the mutation-specific primer is selected from SEQ ID NO: 1 or 2, the paired primer is selected from SEQ ID NO: 3 or 4, and the oligoblocker is selected from SEQ ID NO: 5, preferably the mutation-specific primer is SEQ ID NO: 1, the paired primer is SEQ ID NO: 3, and the oligoblocker is SEQ ID NO: 5.

7. The method of claim 5, wherein the mutation is EGFR L858R and wherein the mutation-specific primer is selected from anyone of SEQ ID NOs: 8, 9, 10 or 11, the paired primer is selected from SEQ ID NO: 12, and the oligoblocker is selected from SEQ ID NO: 13 or 14, preferably, the mutation-specific primer is SEQ ID NO: 11, the paired primer is SEQ ID NO: 12, and the oligoblocker is SEQ ID NO: 13.

8. The method of claim 5, wherein the mutation is *EGFR* L861Q and wherein the mutation-specific primer is selected from SEQ ID NO: 17 or 18, the paired primer is selected from SEQ ID NO: 19, and the oligoblocker is selected from SEQ ID NO:20 or 21, preferably, the mutation-specific primer is SEQ ID NO: 18, the paired primer is SEQ ID NO: 19, and the oligoblocker is SEQ ID NO: 21.

9. The method of claim 5, wherein the mutation is EGFR S768I and wherein the mutation-specific primer is selected from SEQ ID NOs: 34 or 35, the paired primer is selected from SEQ ID NO: 36, and the oligoblocker is selected from SEQ ID NO: 37, preferably the mutation-specific primer is SEQ ID NO: 35, the paired primer is SEQ ID NO: 36, and the oligoblocker is SEQ ID NO: 37.

10. The method of claim 5, wherein the mutation is EGFR G719S and wherein the mutation-specific primer is selected from SEQ ID NO: 24 or 25, the paired primer is SEQ ID NO: 26, and the oligoblocker is selected from SEQ ID Nos: 27 or 28, preferably the mutation-specific primer is SEQ ID NO: 25, the paired primer is SEQ ID NO: 26, and the oligoblocker is SEQ ID NO 28.

11. The method of claim 5, wherein the mutation is EGFR G719A and wherein the mutation-specific primer is selected from anyone of SEQ ID NOs: 31, 32 or 33, the paired primer is SEQ ID NO: 26 (G719 P19) and the oligoblocker is SEQ ID NO: 27 or 28, preferably the mutation-specific primer is SEQ ID NO: 31, the paired primer is SEQ ID NO: 26, and the oligoblocker is SEQ ID NO: 27.

12. The method of claim 5, wherein the mutation is EGFR exon19 Δ(E746-A750) and wherein the mutation-specific primer is SEQ ID NO: 40, the paired primer is SEQ ID NO: 41, and the oligoblocker is SEQ ID NO 42.

13. The method of anyone of the preceding claims, which comprises amplifying a second non-mutated target sequence on the EGFR gene, said second target sequence being located between 100 and 400bp from the first target sequence on said gene, the first and second amplified sequences each being 50-95 nt in length and of a similar length, and wherein the method comprises determining a ratio of the amount of sequence amplified from the first and second sequence.

14. The method of claim 14, wherein :
• the mutation is EGFR T790M, the first set of reagents is as defined in claim 6, and the second set of reagents comprises a first primer SEQ ID NO: 6 and a second primer SEQ ID NO: 7; and/or
the mutation is EGFR L858R, the first set of reagents is as defined in claim 7, and the second set of reagents comprises a first primer SEQ ID NO: 15 and a second primer SEQ ID NO: 16; and/or
• the mutation is EGFR L861Q, the first set of reagents is as defined in claim 8, and the second set of reagents comprises a first primer SEQ ID NO: 22 and a second primer SEQ ID NO: 23; and/or
• the mutation is EGFR S768I, the first set of reagents is as defined in claim 9, and the second set of reagents comprises a first primer SEQ ID NO: 38 and a second primer SEQ ID NO: 39; and/or
• the mutation is EGFR G719, the first set of reagents is as defined in claim 10 or 11, and the second set of reagents comprises a first primer SEQ ID NO: 29 and a second primer SEQ ID NO: 30; and/or
• the mutation is EGFR DEL 19, the first set of reagents is as defined in claim 12, and the second set of reagents comprises a first primer SEQ ID NO: 43 and a second primer SEQ ID NO: 44.

15. A kit for determining the presence of a mutation in the EGFR gene in a sample of a biological fluid containing cell free nucleic acids from a subject, the kit comprising at least a first set of reagents, wherein the first set of reagents amplifies a first target sequence from the EGFR gene, said first target sequence containing a site of a mutation in the EGFR gene, said first set comprising (i) a mutation-specific primer of 15-25nt in length, (ii) a paired primer of 15-25nt in length positioned such that the amplified first target sequence is 50-95nt in length, and (iii) an oligoblocker, wherein the oligoblocker hybridizes specifically with the first target sequence in non-mutated form and blocks polymerase elongation.

16. The kit of claim 15, which comprises a second set of reagents (b) which amplifies a second non-mutated target sequence from the EGFR gene, said second target sequence being located between 100 and 400bp from the first target sequence on said gene, and wherein said amplified second target sequence is 50-95nt in length and of a length similar to that of the first amplified target sequence.

17. The kit of claim 16, wherein both sets are packaged in separate containers.

18. The kit of anyone of claims 15 to 17, which further comprises instructions for performing an amplification reaction and/or a polymerase, a buffer and/or nucleotides.

19. The kit of anyone of claims 15 to 18, wherein the mutation and primers are as defined in anyone of claims 2 to 14.

20. The kit of anyone of claims 15 to 19, which comprises several sets of reagents for determining the presence of several different mutations in the EGFR gene, preferably at least T790M and L858R, even more preferably at least T790M and L858R and exon19 A(E746-A750).

21. A nucleic acid molecule consisting of a nucleotide sequence selected from anyone one SEQ ID NOs: 1-55.

22. The use of a nucleic acid molecule of claim 21 for in vitro amplification of a target sequence in an EGFR gene.
